# EUROPEAN PATENT SPECIFICATION

(11) **EP 3 431 601 B1**
(45) Date of publication and mention of the grant of the patent: **07.09.2022**
(21) Application number: 18166305.5
(22) Date of filing: 09.07.2014
(51) Int. Cl.: C12N 15/113, C12N 15/82, A01H 5/00, A01N 57/16

(54) **RNAI FOR THE CONTROL OF PHYTOPATHOGENIC FUNGI AND OOMYCETES BY INHIBITING THE EXPRESSION OF CYP51 GENES**
RNAI ZUR BEKÄMPFUNG VON PHYTOPATHOGENEN PILZEN UND EIPILZEN DURCH HEMMUNG DER EXPRESSION DES CYP51-GENS
ARNI POUR LE CONTRÔLE DE CHAMPIGNONS PHYTOPATHOGÈNES ET D'OOMYCÈTES PAR INHIBITION DE L'EXPRESSION DE GÈNES CYP51

(30) Priority: 10.07.2013 EP 13175924
(43) Date of publication of application: 23.01.2019
(62) Divisional of application: 14736841.9
(73) Proprietor: BASF SE, 67056 Ludwigshafen am Rhein (DE)
(72) Inventor: KOGEL, Karl-Heinz, 35457 Lollar (DE); JAFARGHOLIE, Imami, 35392 Giessen (DE); KOCH, Aline, 35329 Gemuenden Felda, Otterbach (DE)
(74) Representative: BASF IP Association

(56) References cited:
- WO-A1-2011/069953
- WO-A1-2012/155112
- WO-A1-2013/043777
- WO-A1-2013/050410
- US-A1- 2008 113 351
- DATABASE EMBL [Online] 24 April 2013 (2013-04-24), "Phakopsora pachyrhizi eburicol 14 alpha-demethylase gene, complete cds.", XP002786323, retrieved from EBI accession no. EMBL:KC741475 Database accession no. KC741475 -& HELENA K SCHMITZ ET AL: "Sensitivity of Phakopsora pachyrhizi towards quinone-outside-inhibitors and demethylation-inhibitors, and corresponding resistance mechanisms : Sensitivity and resistance mechanisms of P . pachyrhizi towards fungicides", PEST MANAGEMENT SCIENCE, vol. 70, no. 3, 9 July 2013 (2013-07-09), pages 378-388, XP055521613, BOGNOR REGIS; GB ISSN: 1526-498X, DOI: 10.1002/ps.3562
- RAYKO BECHER ET AL: "Fungal cytochrome P450 sterol 14alpha-demethylase (CYP51) and azole resistance in plant and human pathogens", APPLIED MICROBIOLOGY AND BIOTECHNOLOGY, SPRINGER, BERLIN, DE, vol. 95, no. 4, 12 June 2012 (2012-06-12), pages 825-840, XP035090913, ISSN: 1432-0614, DOI: 10.1007/S00253-012-4195-9
- VINAY PANWAR ET AL: "Endogenous silencing of Puccinia triticina pathogenicity genes through in planta -expressed sequences leads to the suppression of rust diseases on wheat", THE PLANT JOURNAL, vol. 73, no. 3, 12 December 2012 (2012-12-12), pages 521-532, XP055106655, ISSN: 0960-7412, DOI: 10.1111/tpj.12047
- C. BURGER: "Virus-induced silencing of sterol biosynthetic genes: identification of a Nicotiana tabacum L. obtusifoliol-14 -demethylase (CYP51) by genetic manipulation of the sterol biosynthetic pathway in Nicotiana benthamiana L.", JOURNAL OF EXPERIMENTAL BOTANY, vol. 54, no. 388, 13 May 2003 (2003-05-13) , pages 1675-1683, XP055106656, DOI: 10.1093/jxb/erg184
- Anonymous: "International Reinhardsbrunn Symposium on Modern Fungicides and Antifungal Compounds Scientific Program", , 21 April 2013 (2013-04-21), XP055106660, Friedrichroda, DE Retrieved from the Internet: URL:http://www.reinhardsbrunn-symposium.de /fileadmin/PDF/Scientific%20Program%2013_3 _2012.pdf [retrieved on 2014-03-11]
- CHOWDHARY ANURADHA ET AL: "Clonal Expansion and Emergence of Environmental Multiple-Triazole-Resistant Aspergillus fumigatus Strains Carrying the TR34/L98H Mutations in the cyp51A Gene in India", PLOS ONE, vol. 7, no. 12, December 2012 (2012-12), page Article No.: e52871, ISSN: 1932-6203(print)
- COOLS HANS J ET AL: "Impact of Recently Emerged Sterol 14 alpha-Demethylase (CYP51) Variants of Mycosphaerella graminicola on Azole Fungicide Sensitivity", APPLIED AND ENVIRONMENTAL MICROBIOLOGY, vol. 77, no. 11, June 2011 (2011-06), pages 3830-3837, ISSN: 0099-2240(print)
- SPIESS BIRGIT ET AL: "Incidence of Cyp51 A Key Mutations in Aspergillus fumigatus-A Study on Primary Clinical Samples of Immunocompromised Patients in the Period of 1995-2013", PLOS ONE, vol. 9, no. 7, 29 July 2014 (2014-07-29), page Article No.: e103113, ISSN: 1932-6203(print)

## Description

### FIELD OF THE INVENTION

The present invention relates to double-stranded RNA (dsRNA)-mediated gene silencing of at least one CYP51 gene from the phytopathogen *Phakopsora pachyrhizi.* Accordingly, the present invention relates to phytopathogen-tolerant transgenic plants comprising a DNA sequence providing a transcriptional template for such inhibitory dsRNA molecules or antisense RNA; and methods of controlling a *Phakopsora pachyrhizi* phytopathogen.

### BACKGROUND OF THE INVENTION

Plant diseases caused by phytopathogenic fungi and oomycetes are a serious risk in the culturing of plants. Diseases of cereal crops such as Fusarium head blight and root rot caused by phytopathogenic fungi of the genus *Fusarium* exert great economic and agronomic impact on global grain production and the grain industry. The damages caused by phytopathogenic fungi and oomycetes impair quality and yield of the agricultural products up to total crop failure, and thus result in significant economic losses. In addition, food safety can be compromised by contamination of agricultural products with mycotoxins, which are produced by phytopathogenic fungi during plant infestation and represent a serious threat to human and animal health. The risk of devastating losses due to phytopathogenic fungi and oomycetes is particularly high in monocultures which currently represent the predominant method of agricultural crop plant production.

Currently, the major strategies in management of phytopathogenic fungi and oomycetes include the widely used application of fungicides, resistance breeding strategies, biological control and genetic engineering. The latter strategy relies on the use of transgenes such as chitinase, defensins, polygalacturonase, and the use of mycotoxin detoxifying enzymes. However, the use of such antifungal traits has so far not provided convincing practical solutions in terms of efficiency and reliability under agronomical practice.

Fungicides, such as systemic DMIs (demethylation inhibitors), are currently essential for controlling plant diseases such as fusarioses to reach the attainable production level of modern high-yield cultivars. DMI fungicides, such as tebuconazole, triadimefon, and prochloraz, inhibit ergosterol biosynthesis by binding to cytochrome P450 lanosterol C-14 α-demethylase (CYP51) which results in disturbance of the fungal membrane integrity (Yoshida: Lanosterol 14a-demethylase. In: Schenkman, H., Grein, K. (Eds.), Cytochromes P450. Springer-Verlag, Berlin, 1993. pages 627-639).

However, the heavy reliance on DMI fungicides since their discovery in the mid-1970s has led to the emergence of many DMI-resistant phytopathogen strains over the last few years. For these reasons, control of plant diseases caused by phytopathogenic fungi and oomycetes continues to be a challenge.

RNA interference, also known as "RNAi", has emerged as a genetic tool that accelerated research in plant biotechnology. RNAi is a conserved integral part of the gene regulation processes present in all eukaryotes. For RNAi in plants, the alternative term "post-transcriptional gene silencing" (PTGS) has been used. PTGS starts with the processing or cleaving of a precursor double-stranded RNA into short, about 20-25 ribonucleotides long, single- or double-stranded interfering RNA (siRNA) or micro RNA (miRNA) (Hamilton & Baulcombe, Science 286:950-952. 1999) by an RNaseIII-like enzyme called Dicer (Baulcombe, Nature 431:356-363. 2004). The siRNAs or miRNAs are incorporated into an RNA-induced silencing complex (RISC) which recognizes complementary messenger RNA (mRNA) molecules and degrades them, thus resulting in substantially decreased levels of protein translation and effectively turning off the corresponding gene.

*In planta* expression of double-stranded RNAs was recently described to induce host plant-induce gene silencing (HIGS) in fungal cells. In tobacco, expression of a GUS (β-glucuronidase) gene-interfering cassette was reported to specifically reduce transcript levels in a GUS-expressing strain of *Fusarium verticillioides* during plant colonization (Tinoco et al., BMC Biol. 31(8):27. 2011). Nowara et al. (Plant Cell 22:3130-41. 2010) prepared barley expressing a double-stranded RNA targeting the fungal effector gene Avra10 and reported reduced numbers of functional haustoria inside leave epidermal cells. *Agrobacterium tumefaciens-mediated* transient silencing of the *Puccinia triticina* (leaf rust) pathogenicity genes *mitogen-activatedprotein kinase* 1 (*PtMAPK1*)*, cyc*/*o-phi*/*in* (*PtCYC1),* and *calcineurin* B (Pt*CNB*) was described to partly suppress the growth of *P. triticina, P. graminis* and *P. striiformis* in wheat (Panwar et al., Plant J 73:521-32. 2013). WO 2011/069953 describes the provision of transgenic plants with resistance to the phytopathogen *Phakopsora pachyrhizi* by dsRNA-mediated silencing of the SMT1 gene.

It was an object of the present invention to provide a further RNAi-based approach against fungal and oomycete phytopathogens, and in particular to provide a method and means for controlling such phytopathogens in the agronomical practice.

### SUMMARY OF THE INVENTION

The inventors have found that RNAi targeting at least one CYP51 gene of a phytopathogenic fungus or oomycete is effective in controlling such phytopathogens. Accordingly, plant protection products and transgenic plants based on said novel approach can be useful in crop plant production.

The present invention provides a transgenic plant, preferably a cereal crop, comprising a DNA sequence providing a promoter operably linked to a transcriptional template of at least the antisense sequence(s) of the dsRNA molecule, said dsRNA molecule being capable of inhibiting the expression of at least one CYP51 gene from the phytopathogen *Phakopsora pachyrhizi,* said CYP 51 gene having a sequence according to SEQ ID NO. 18, and the dsRNA comprising
- a sense sequence being at least 95% identical to at least 20 contiguous nucleotides of the coding sequence of said CYP51 gene, and
- an antisense sequence being complementary to said sense sequence,
wherein the arrangement of the sequences within the dsRNA molecule allows hybridization of said sense sequence and said corresponding antisense sequence; and wherein
the plant is capable of generating at least the antisense sequence(s) of the dsRNA molecule, wherein in particular the plant is capable of generating said dsRNA molecules.

The present invention further provides a method for controlling a *Phakopsora pachyrhizi* phytopathogen*,* wherein a transgenic plant as defined above is cultivated to allow the generation of RNA comprising the antisense sequence(s) of the dsRNA molecule, and said RNA inhibits the growth and/or propagation of said phytopathogen.

Moreover, the present invention provides a method for controlling a *Phakopsora pachyrhizi* phytopathogen, wherein a plant infested by or at risk of being infested by said phytopathogen and/or the vicinity of said plant is contacted with an effective amount of the composition for controlling a fungal or oomycete phytopathogen comprising the dsRNA molecule as defined above, and a plant-compatible carrier.

### BRIEF DESCRIPTION OF FIGURES

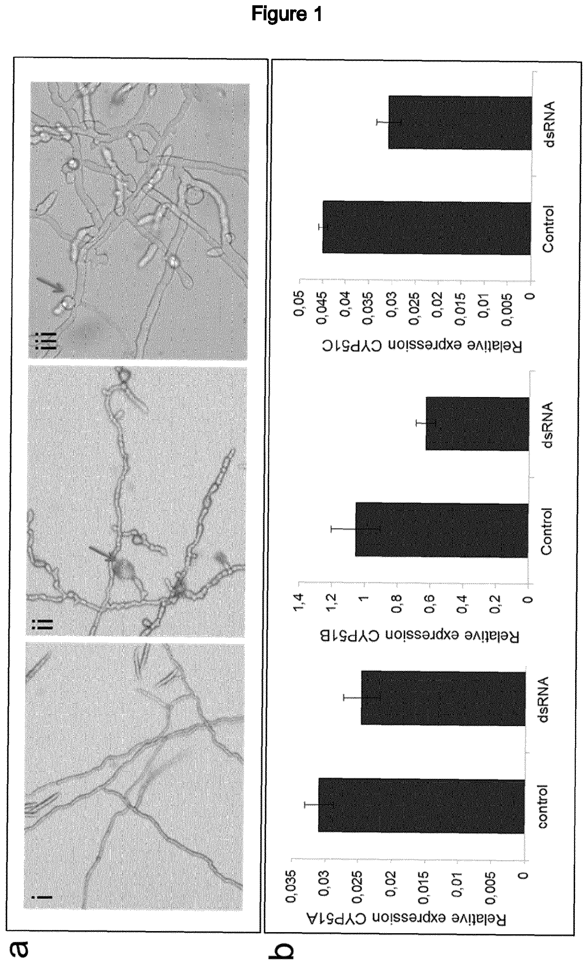

**Figure 1****:** Morphology of *Fusarium graminearum (Fg)* macroconidia grown in axenic culture following treatment with *CYP3RNA* or tebuconazole. **(a)** One hundred *Fg* macroconidia were suspended in 100 µl of liquid SNA medium and treated with **(i)** 1.5 µg *CYP3RNA* dsRNA, **(ii)** 5 mg/l tebuconazole and **(iii)** mock (50x annealing buffer) at room temperature. Pictures were taken 72 h post treatment. The arrows point to swelling and ballooning hyphal tips. **(b)** Quantification of fungal *CYP51A, CYP51B* and *CYP51C* transcripts by quantitative RT-PCR using the same mock- and *CYP3RNA-*treated samples as in **(a).** The data was normalized to β-tubulin.

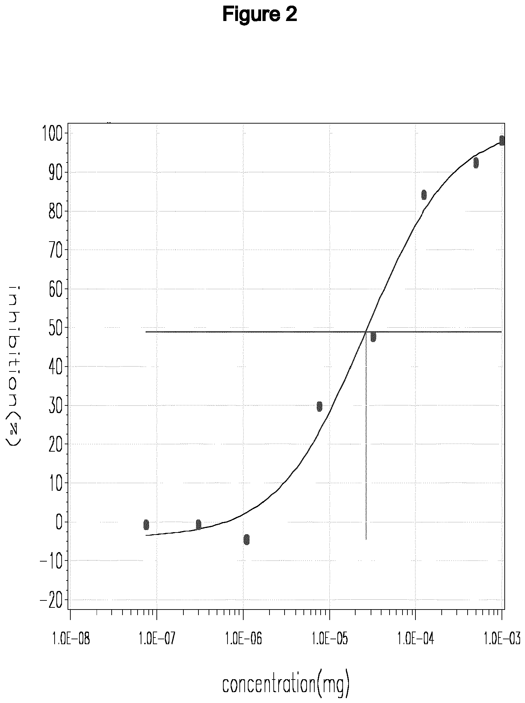

**Figure 2****:** Inhibition of the growth of *Fg* macroconidia by *CYP3RNA.* One hundred *Fg* macroconidia were suspended in 100 µl of liquid SNA medium and treated with different concentrations of *CYP3RNA* dsRNA in the range of from 75 pg to 1 µg dsRNA. Densities of Fg macroconidia cultures were measured at beginning of the treatment and 72 h post treatment.

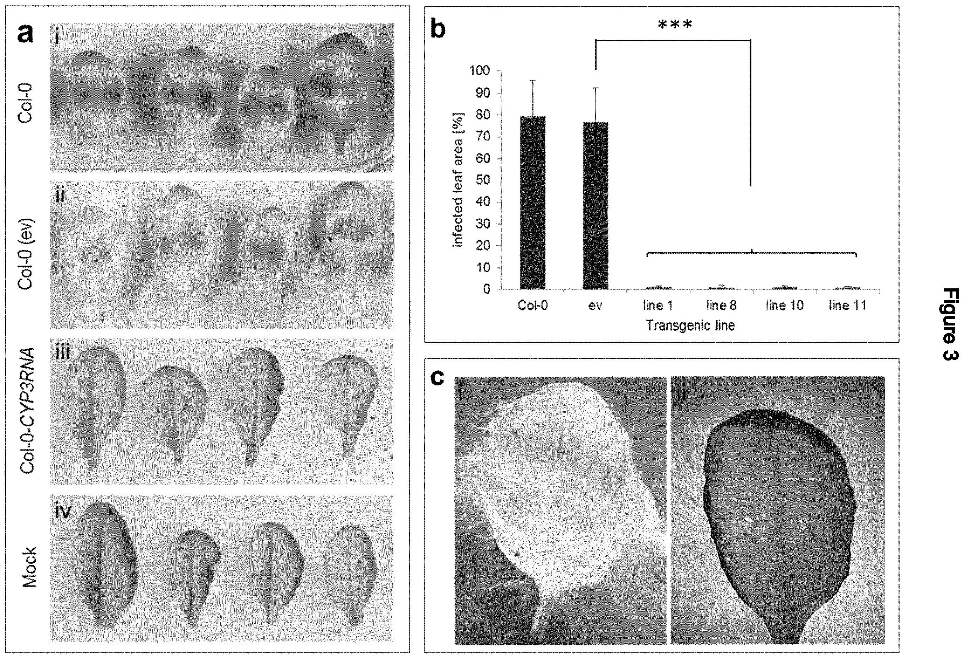

**Figure 3****:** Infestation symptoms on Arabidopsis leaves following inoculation with *Fg.* **(a)** Detached leaves of 5-week-old plants were treated with 5×10⁴ Fg macroconidia ml⁻¹ and evaluated for necrotic lesions at 3 days post inoculation (dpi). **(i)** Col-0 wt, **(ii)** Col-0 empty vector (ev) control, **(iii)** Col-0 expressing *CYP3RNA* (L8 Col-0-*CYP3RNA),* and **(iv)** Col-0 mock-inoculated (Tween water). **(b)** Quantification of *Fg*-infested leaf area at 3 dpi; typical infestation symptoms are expressed as a percent of the total leaf area. Bars represent mean values ± SDs of three independent experiments, each using 20 leaves collected from 15 different plants of each transgenic plant line, as well as Col-0 wt and Col-0 ev plants. The reduction in infestation symptoms on *CYP3RNA-*expressing leaves as compared to the Col-0 wt and Col-0 ev controls was statistically significant (*** p<0.0001; Student's *t*-test). **(c)** *Fg*-inoculated Arabidopsis leaves at 5 dpi. **(i)** The Col-0 ev leaf is heavily infested with *Fg,* **(ii)** Col-0 expressing *CYP3RNA* does not show infestation symptoms, although fungal mycelia are able to grow in the agar surrounding the intact, symptomless leaf.

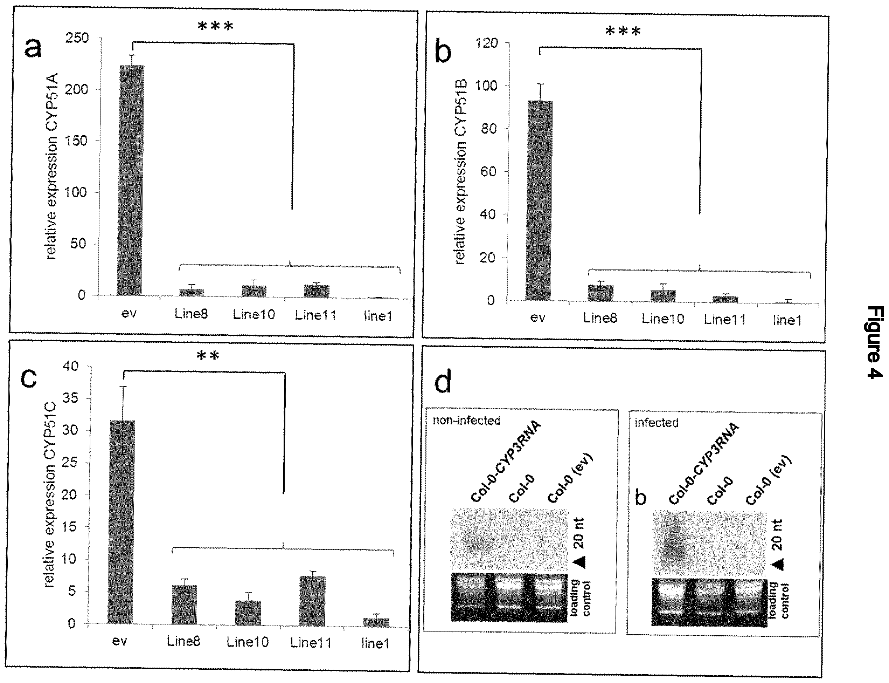

**Figure 4****:** Abundance of *CYP51* gene transcripts and siRNAs in Fg-inoculated Arabidopsis leaves. **(a-c)** Gene-specific analysis of *GYP51* transcripts at 3 dpi by qRT-PCR using fungal β-tubulin as the reference gene. **(a)** *CYP51A,* **(b)** *GYP518* and **(c)** *CYP51C.* cDNA was generated from total RNA isolated from Fg-inoculated leaves collected during the detached leaf assay. Bars represent mean values ± SDs of three independent sample collections. The reduction in *CYP51* gene expression in the *Fg-*inoculated Col-0-*GYP3RNA* leaves compared to the ev control was statistically significant (**p<0.001, *** p<0.0001; Student's t-test). **(d).** Detection of low molecular weight siRNA complementary to the *CYP51* genes in pooled leaf tissue from uninfested and Fg-infested Arabidopsis plants at 3 dpi. Northern blot analysis using ³²P-labelled *CYP3RNA* was followed by autoradiography. No signal was detected in the control samples from Col-0 and Col-0-ev plants. Ethidium bromide-stained rRNA in the bottom panel served as the loading control.

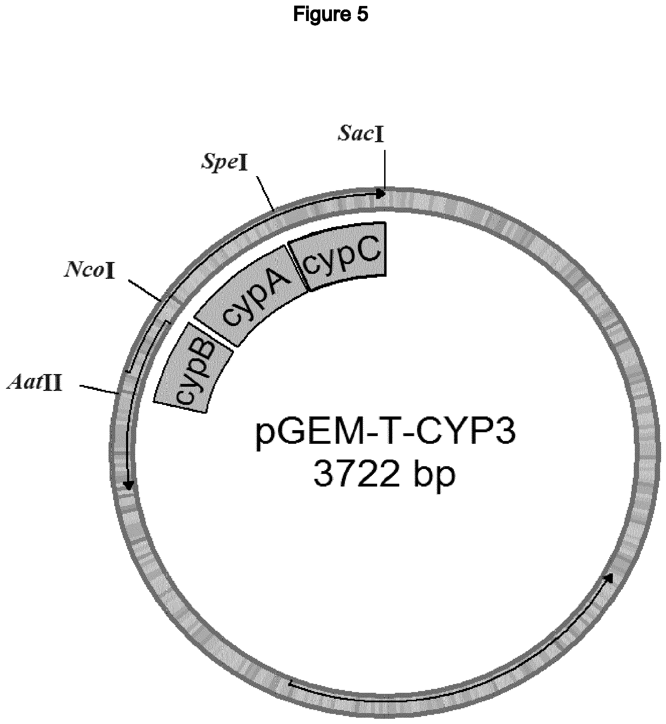

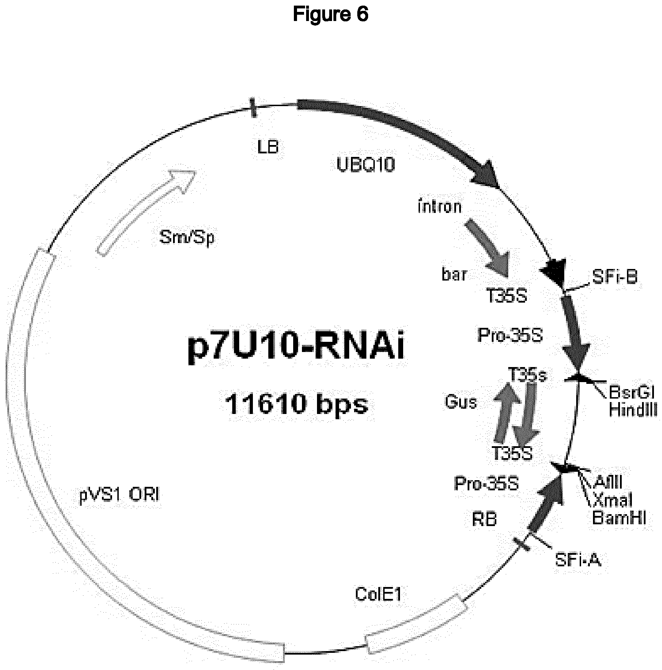

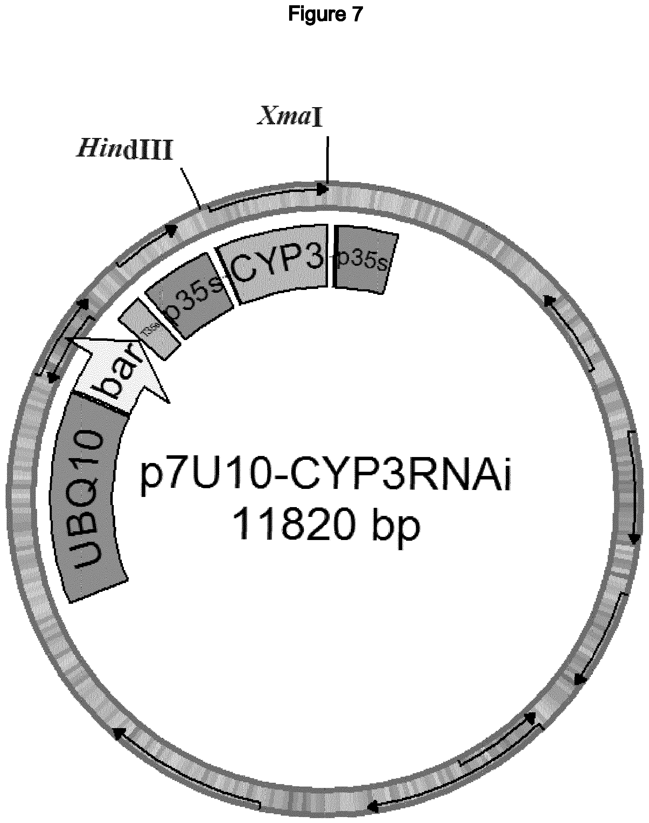

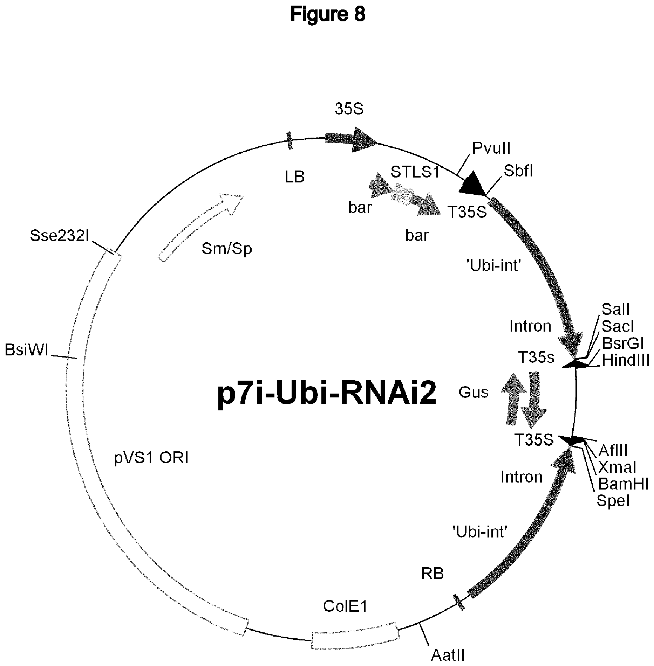

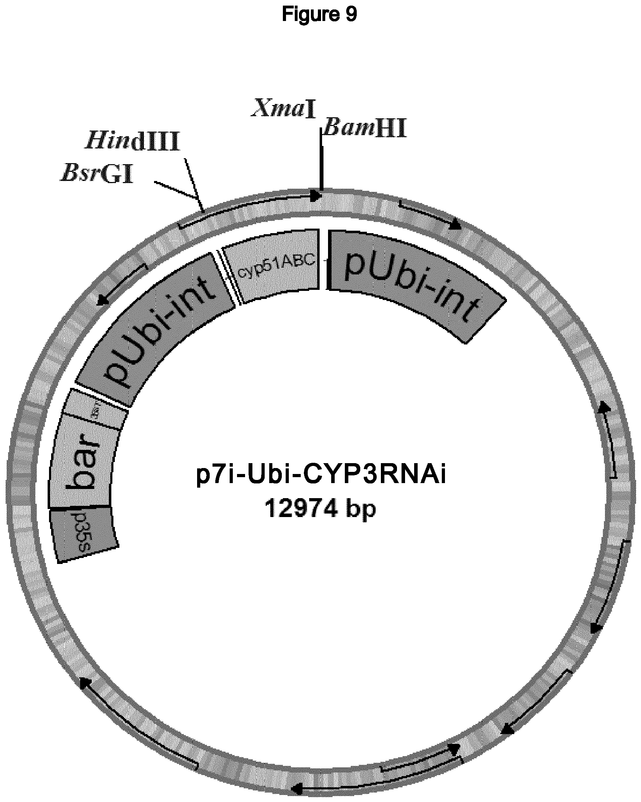

**Figures 5-9****:** Fragment combination strategy (stacking) in pGEM-T-CYP3 **(****Fig. 5****)** for generating *CYP3RNA,* a construct designed from partial sequences of CYP51A (SEQ ID NO:4), CYP51B (SEQ ID NO:5) and CYP51C (SEQ ID NO:6) to silence all three *CYP51* genes. **(****Fig. 6****)** *p7U10-RNAi,* a binary vector with inverted repeat CaMV35S promoters. **(****Fig. 7****)** *p7U10-GYP3RNAi,* generated by replacing the GUS-fragment in *p7U10-RNAi* by the *CYP3RNA* fragment (CYP51B-CYP51A-CYP51C) using Hindlll/Xmal restriction enzymes. **(****Fig. 8****)** *p7i-Ubi-RNAi2,* a binary vector with inverted repeat CaMV35S promoters. **(****Fig. 9****)** *p7i-UBi-CYP3RNAi,* generated by replacing the GUS-fragment in *p7i-Ubi-RNAi2* by a *CYP51ABC* fragment (CYP51A-CYP51B-CYP51C) using Hindlll/Xmal restriction enzymes.

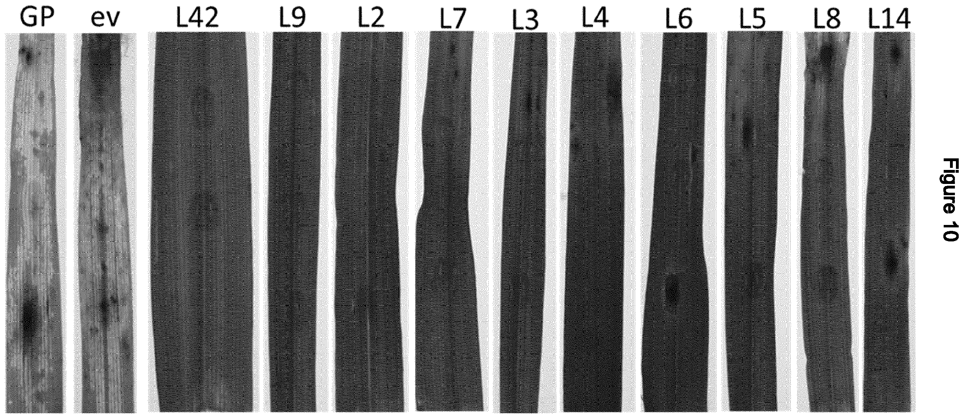

**Figure 10****:** Leaves of transgenic barley expressing *CYP51ABC* (L2-L9, L14, L42), of a wildtype control plant (cv. Golden Promise; GP) and of a transgenic control plant comprising only the transfection vector (empty vector; ev) 7 days after inoculation (7 dpi) with *Fg* as described in Example 4.

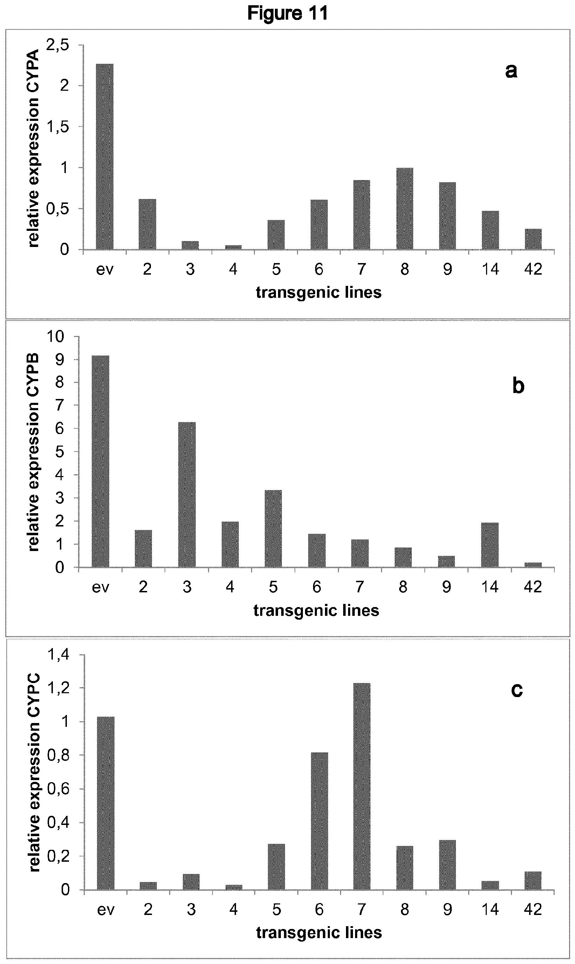

**Figure 11****:** Abundance of *GYP51* gene transcripts in Fg-inoculated barley leaves. **(a-c)** Gene-specific analysis of *CYP51* transcripts at 7 dpi by qRT-PCR using fungal β-tubulin as the reference gene. **(a)** *CYP51A,* **(b)** *CYP51B* and **(c)** *CYP51C.* cDNA was generated from total RNA isolated from Fg-inoculated leaves collected during the detached leaf assay.

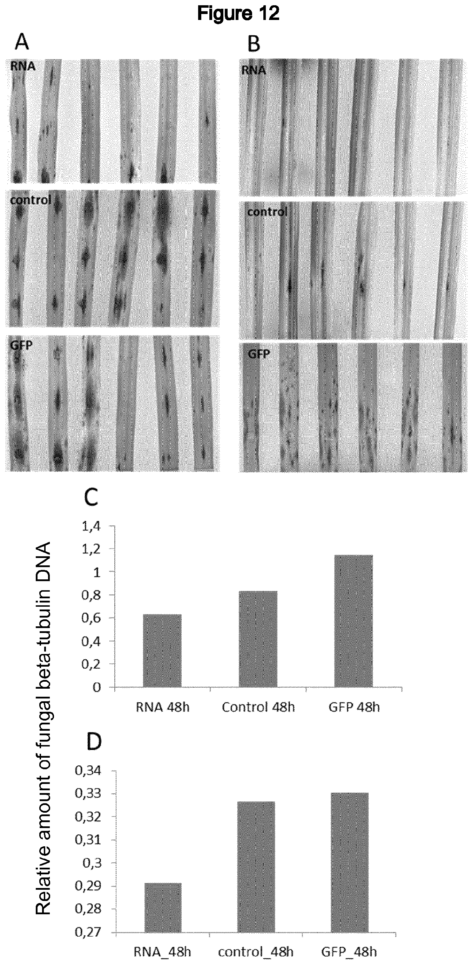

**Figure 12****:** Inhibition of fungal growth by spraying barley leaves with *CYP3RNA* dsRNA. Detached barley leaves were sprayed area-covering **(A)** or systemically **(B)** with *CYP3RNA* dsRNA ("RNA"), TE-buffer only ("control") or GFP-dsRNA ("GFP"), respectively. 48 h after spraying the leaves were drop-inoculated with *Fg.* 6 days after infection, the infection symptoms were recorded **(A, B)** and the relative amount of fungal genomic β-tubulin DNA in area-covering-sprayed **(C)** or systemically-sprayed **(D)** leaves, respectively, was measured using quantitative PCR (qPCR). Graphs **C** and **D** show the relative amount of fungal genomic β-tubulin DNA normalized to the amount of plant genomic ubiquitin DNA.

### DETAILED DESCRIPTION

### 1) Definitions

The term "fungal or oomycete phytopathogen", as used herein, refers to a fungus or oomycete that is parasitic on a plant host.

Phytopathogenic fungi include fungi of the phyla Ascomycota and Basidiomycota. The asexual reproduction stage (anamorph) of such fungus may be known under a different name than its sexual reproduction stage (teleomorph). For example, *Fusarium graminearum* refers to the anamorph and *Gibberella zeae* to the teleomorph of the same fungal species. For the purpose of the present invention, the names of the Ascomycota and Basidiomycota used herein are meant to refer the fungal species *per se* rather than to a particular reproduction stage thereof.

CYP51 genes are genes encoding a cytochrome P450 sterol 14α-demethylase (CYP51) and homologues thereof. A fungal or oomycete phytopathogen can have more than one CYP51 gene. The different CYP51 genes can be grouped into clades by molecular phylogenic analysis as described, e.g., by Becher et al. (BMS Genomics 12: 52. 2011). Three clades of CYP51 have been identified for fungi of the subphylum Pezizomycotina: CYP51A, CYP51B and CYP51C (*supra*)*.* CYP51 genes, CYP51 proteins and corresponding coding sequences of phytopathogenic fungi and oomycetes are known in the art and can be identified by homology searches, e.g. based on FGSG_04092.3, FGSG_01000.3 and FGSG_11024.3, by sequence analyses as described, e.g., by Becher et al. (BMS Genomics 12: 52. 2011; cf. section on Bioinformat-ics), and from sequence databases such as the Fungal Cytochrome P450 Database (http://p450.riceblast.snu.ac.kr).

The term "dsRNA molecule", as used herein for designating a subject matter of the invention, refers to a molecule comprising one, two or more polyribonucleotide strands capable of forming at least one region of double stranded RNA. Thus, the term "dsRNA molecule of the disclosure" includes molecules, wherein only part of the RNA, e.g. at least 70%, at least 80% or at least 90%, or all of the RNA is present as double stranded RNA. For example, the term "dsRNA molecule" includes molecules consisting of one, two or more polyribonucleotide strands. Also included by the term are molecules additionally comprising further chemical groups, for example groups stabilizing the regions of double stranded RNA as described herein.

The effect of the dsRNA molecules described herein is assumed to be due to RNA interference. The terms "RNAi" and "RNA interference", used herein, refer to a process of sequence-specific post-transcriptional gene silencing mediated by double-stranded RNA. In the RNAi process, a double-stranded RNA is processed into relatively small fragments, typically 20-25 nucleotides in length, which become part of an RNA-induced silencing complex (RISC) which binds to and cleaves complementary mRNA and thus prevents the mRNA from being used as a template for translation.

The term "antisense interference" also refers to a process of sequence-specific post-transcriptional gene silencing. In antisense interference, a single-stranded antisense RNA (antisense-ssRNA) that is substantially complementary to at least a part of a target gene causes inhibition of the target gene expression. It is assumed that in this process double-stranded RNA is formed by hybridization of the antisense ssRNA hybridizes with complementary mRNA and inhibits the target gene expression using the RNAi mechanism.

The expression "at least part of", when used with reference to a sequence, e.g. with reference to the coding sequence of a CYP51 gene, refers to at least 20 contiguous nucleotides of said sequence. For example, "at least part of" refers to at least 25, at least 50, at least 100, at least 150 and preferably at least 200 contiguous nucleotides of the sequence.

A "high degree of identity", as used herein to define the degree of sequence identity of a sense sequence to at least part of the coding sequence of a CYP51 gene or any one of SEQ ID NOs: 1-6 and 8-13, refers to an identity of at least 70%, at least 75%, at least 80%, at least 85%, at least 90%, and preferably at least 95% or at least 98%, and most preferably at least 99% or 100%.

With regard to sequence identity between RNA and DNA sequences, a uracil within a RNA sequence is considered "identical" to a thymine within a DNA sequence. Methods and tools such as computer programs for calculating sequence identity are well known in the art. For example, the degree of identity between nucleotide sequences can be determined using a Basic Local Alignment Search Tool (BLAST) such as the BLAST Similarity Search (gapped alignment) available on the website of the Broad Institute (http://www.broadinstitute.org/annotation/genome/fusarium_graminearum/ Blast.html?sp=Sblastn).

An "antisense sequence" as comprised by the dsRNA molecules of the present disclosure is an RNA sequence that is substantially complementary to the corresponding sense sequence.

"Complementary" polynucleotides are those capable of base pairing according to the Watson-Crick complementarity rules. Specifically, base pairs will form between purines and pyrimidines including guanine paired with cytosine (G:C) and adenine paired with either thymine (A:T) in case of DNA, or adenine paired with uracil (A:U) in the case of RNA. It is understood that two polynucleotides may hybridize to each other even if they are not completely complementary to each other, provided that each has at least one region that is substantially complementary to the other.

The term "substantially complementary", as used herein e.g. for characterizing the antisense sequences of the dsRNA molecule of the disclosure , means that two nucleotide sequences are complementary over at least at 80% of their nucleotides. Preferably, the two nucleotide sequences are complementary over at least at 85%, at least 90%, more preferably at least 95%, at least 96%, at least 97%, at least 98%, and most preferably at least 99% of their nucleotides up to their full length. Alternatively, "substantially complementary" can refer to two nucleic acid sequences which can hybridize under stringent conditions.

The term "hybridization", as used herein, includes any process by which a polynucleotide strand joins with a complementary strand through base pairing. (Coombs: Dictionary of Biotechnology, Stockton Press, New York, 1994). Hybridization and the strength of hybridization (i.e., the strength of the association between the two complementary strands) is impacted by factors such as the degree of complementarity between the strands, the stringency of the conditions involved, the melting temperature of the formed double strand, and the G:C ratio within the strands. The melting temperature (Tm) is the temperature at which a population of double-stranded polynucleotides becomes half dissociated into single strands. Tm can be calculated using equations well known in the art. An estimate for the Tm value of a polynucleotide in an aqueous 1 M NaCl solution is given by the equation: Tm=81.5+0.41(% G+C) (cf., e.g., Anderson and Young: Quantitative Filter Hybridization, in Nucleic Acid Hybridization, 1985). Stringent conditions are known to those skilled in the art (cf., e.g., Current Protocols in Molecular Biology, John Wiley & Sons, N.Y., 6.3.1 -6.3.6, 1989). In particular, the term "stringent conditions", as used herein, refers to hybridization to filter-bound nucleic acid in 6x sodium chloride/sodium citrate (SSC) at about 45°C followed by one or more washes in 0.2x SSC/0.1% SDS (sodium dodecyl sulfate) at about 50-65°C.

The term "expression", as used herein with respect to a gene sequence, refers to the translation of the coding sequence to a polypeptide. Inhibition of gene expression can become detectable on the level of mRNA, on the level of polypeptide or both. Methods for assessing changes in the mRNA level or the protein level of a gene are well-known in the art. For example, the change in the mRNA level of a gene can be assessed by quantitative real-time PCR (qRT-PCR) or Northern Blot.

"Controlling" a phytopathogenic fungus or oomycete, as used herein, includes measures for preventing the infestation of a plant with said pathogen as well as measures for combatting or curing the pathogen infestation of a plant. The term "combatting" refers to the reduction of the pathogen infestation. The term "curing" refers to the eradication of the pathogen infestation.

A "plant-compatible" carrier, as used herein, is a compound or a mixture of compounds that, under the conditions of its use, does not exert unacceptable phytotoxic activity to the treated plant.

The term "plant" is used herein to designate a whole plant at any stage of development as well as a part or derivative thereof, and thus includes, for instance, a plant cell, a plant cell population, a plant tissue (e.g. a meristematic tissue, callus tissue), a plant organ (e.g. stem, leaf, root, ovule, stamen), a reproductive form or reproductive part of a plant (e.g. a seed, tuber, cutting, gametophyte, sporophyte, pollen, microspore, embryo). A plant cell or plant cell population can be isolated (e.g. in suspension culture) or comprised in a plant tissue, plant organ or whole plant of any developmental stage.

A "transgenic" plant is a whole plant or part thereof that has been altered using recombinant DNA technology to contain a nucleic acid sequence which would otherwise not be present in said plant or which would be expressed to a considerably lower extent. The term "transgenic plant" also includes the transgenic progeny of a transgenic plant. A transgenic plant of the present invention may result from crossing a transgenic plant of the invention with a non-transgenic plant or with another transgenic plant of the invention or with a transgenic plant having a different transgene. In particular, the term "transgenic plant" also comprises true breeding transgenic plants which are obtained by repeated inbreeding steps.

A "transcriptional template" of an RNA sequence is a DNA sequence that can serve as a template for the generation of the RNA by enzymatic transcription with an RNA polymerase.

The term "promoter", when used herein in the context of the nucleic acid sequence(s) of the invention, refers to the singular as well as to the plural, unless explicitly stated otherwise. A "promoter", as used herein, is a DNA sequence which, when ligated to a DNA sequence of interest, is capable of controlling the transcription of said DNA sequence into RNA. A promoter is typically located 5' (e.g., upstream) of the DNA sequence of interest whose transcription into mRNA it controls (e.g., proximal to the transcriptional start site), and provides a site for specific binding by RNA polymerase and other transcription factors for initiation of transcription. A promoter can be a constitutive promoter or a regulated promoter.

A "regulated promoter" is a promoter that drives transcription not constitutively but in a temporally and/or spatially restricted manner. A promoter is regulated if the amount of RNA produced under the control of the activated promoter is at least 30%, 40%, 50%, 60%, 70%, 80%, 90%, 100%, 200% or 300% higher than the amount of RNA produced in parts of the plant or at periods of time, where the promoter is not activated. Regulated promoters include inducible promoters, tissue-specific promoters and development-specific promoters. Tissue-specific or development-specific promoters facilitate transcription of a sequence of interest in specific tissues, organs or cell types, or at specific different developmental stages while leaving the rest of the organism unmodified. In the case of plants, such promoters might specifically influence expression of genes in the roots, inflorescence, cereal ears, fruits, or seeds, or during the vegetative, flowering, or seed-setting stage. Inducible promoters facilitate transcription of a sequence of interest in the presence or absence of particular chemical compounds (e.g. an alcohol, tetracycline, a steroid, or a metal) or depending on particular physical conditions (e.g. the presence or absence of light, low or high temperatures).

The term "phytopathogen-tolerant" is used herein to designate a transgenic plant of the invention capable of reducing or preventing the growth and/or propagation of the phytopathogen. The tolerance to the phytopathogen can be transient, i.e. present only for a limited period of time, for example due to a transient expression of the antisense-ssRNA or the dsRNA molecule as herein described. A plant that is "tolerant" to a phytopathogen is infested less severely and/or less frequently by the phytopathogen. Severity of the fungal or oomycetes infestation can be determined based on the disease symptoms observed after the plant was inoculated with or attacked by the phytopathogen. The disease symptoms depend on the nature (species, variant) of the phytopathogen and of the plant. Symptoms of fungal or oomycete infestations of plants include, but are not limited to, premature bleaching of cereal ears and/or leaves; necrotic lesions on the exterior surface of the florets and glume; kernel atrophy; awn deformation; discoloration of kernels and/or spikelets; visible pathogen growth on leaves, stem, and/or cereal ears.

### 2) Particular aspects of the disclosure

Herein described are dsRNA molecules and antisense-ssRNA capable of inhibiting the expression of at least one CYP51 gene of a fungal or oomycete phytopathogen.

Further, these dsRNA molecules and antisense-ssRNA are capable of reducing or preventing the growth and/or propagation of fungal or oomycete phytopathogens. Specifically, these dsRNA molecules and antisense-ssRNA are capable of reducing or eradicating the infestation of a plant by fungal or oomycete phytopathogens, wherein said plant contains and/or is treated with said dsRNA molecules or antisense-ssRNA.

These dsRNA molecules and antisense-ssRNA are useful for controlling phytopathogenic fungi and oomycetes including, but not limited to, *Albugo* spp. (white rust) on ornamentals, vegetables (e.g. *A. candida)* and sunflowers (e.g. *A. tragopogonis*); *Alternaria* spp. (Alternaria leaf spot) on vegetables, rape (*A. brassicola* or *brassicae),* sugar beets (*A. tenuis*)*,* fruits, rice, soybeans, potatoes (e.g. *A. solani* or *A. alternata),* tomatoes (e.g. *A. solani* or *A. alternata)* and wheat; *Aphanomyces* spp. on sugar beets and vegetables; *Ascochyta* spp. on cereals and vegetables, e.g. *A. tritici* (anthracnose) on wheat and *A. hordei* on barley; *Bipolaris* and *Drechslera* spp. (teleomorph: *Cochliobolus* spp.), e.g. Southern leaf blight (*D. maydis*) or Northern leaf blight (*B. zeicola*) on corn, e.g. spot blotch (*B. sorokiniana*) on cereals and e.g. *B. oryzae* on rice and turfs; *Blumeria* (formerly *Erysiphe*) *graminis* (powdery mildew) on cereals (e.g. on wheat or barley); *Botrytis cinerea* (teleomorph: *Botryotinia fuckeliana:* grey mold) on fruits and berries (e.g. strawberries), vegetables (e.g. lettuce, carrots, celery and cabbages), rape, flowers, vines, forestry plants and wheat; *Bremia lactucae* (downy mildew) on lettuce; *Ceratocystis* (syn. *Ophiostoma*) spp. (rot or wilt) on broad-leaved trees and evergreens, e.g. *C. ulmi*(*Dutch* elm disease) on elms; *Cercospora* spp. (Cercospora leaf spots) on corn (e.g. Gray leaf spot: *C. zeae-maydis*)*,* rice, sugar beets (e.g. C. *beticola*)*,* sugar cane, vegetables, coffee, soybeans (e.g. *C. sojina* or *C. kikuchii*) and rice; *Cladosporium* spp. on tomatoes (e.g. *C. fulvum:* leaf mold) and cereals, e.g. *C. herbarum* (black ear) on wheat; *Claviceps purpurea* (ergot) on cereals; *Cochliobolus* (anamorph: *Helminthosporium* of *Bipolaris*) spp. (leaf spots) on corn (C. *carbonum),* cereals (e.g. *C. sativus,* anamorph: *B. sorokiniana*) and rice (e.g. *C. miyabeanus,* anamorph: *H. oryzae); Colletotrichum* (teleomorph: *Glomerella*) spp. (anthracnose) on cotton (e.g. *C. gossypii*)*,* corn (e.g. *C. graminicola:* Anthracnose stalk rot), soft fruits, potatoes (e.g. *C. coccodes:* black dot), beans (e.g. *C. lindemuthianum*) and soybeans (e.g. *C. truncatum* or *C. gloeosporioides*)*; Corticium* spp., e.g. *C. sasakii* (sheath blight) on rice; *Gorynespora cassiicola* (leaf spots) on soybeans and ornamentals; *Cycloconium* spp., e.g. *C. oleaginum* on olive trees; *Cylindrocarpon* spp. (e.g. fruit tree canker or young vine decline, teleomorph: *Nectria* or *Neonectria* spp.) on fruit trees, vines (e.g. C. *liriodendri,* teleomorph: *Neonectria liriodendri:* Black Foot Disease) and ornamentals; *Dematophora* (teleomorph: *Rosellinia)* necatrix (root and stem rot) on soybeans; *Diaporthe* spp., e.g. *D. phaseolorum* (damping off) on soybeans; *Drechslera* (syn. *Helminthosporium,* teleomorph: *Pyrenophora)* spp. on corn, cereals, such as barley (e.g. *D. teres,* net blotch) and wheat (e.g. *D. tritici-repentis:* tan spot), rice and turf; Esca (dieback, apoplexy) on vines, caused by *Formitiporia* (syn. *Phellinus) punctata, F. mediterranea, Phaeomoniella chlamydospora* (earlier *Phaeoacremonium chlamydosporum), Phaeoacremonium aleophilum* and/or *Botryosphaeria obtusa, Elsinoe* spp. on pome fruits *(E. pyri),* soft fruits *(E. veneta:* anthracnose) and vines (*E. ampelina:* anthracnose); *Entyloma oryzae* (leaf smut) on rice; *Epicoccum* spp. (black mold) on wheat; *Erysiphe* spp. (powdery mildew) on sugar beets (*E. betae),* vegetables (e.g. *E. pisi),* such as cucurbits (e.g. *E. cichoracearum),* cabbages, rape (e.g. *E. cruciferarum); Eutypa lata* (Eutypa canker or dieback, anamorph: *Cytosporina lata,* syn. *Libertella blepharis)* on fruit trees, vines and ornamental woods; *Exserohilum* (syn. *Helminthosporium)* spp. on corn (e.g. *E. turcicum); Fusarium* (teleomorph: *Gibberella)* spp. (wilt, root or stem rot) on various plants, such as *F. graminearum* or *F. culmorum* (root rot, scab or head blight) on cereals (e.g. wheat or barley), *F. oxysporum* on tomatoes, *F. solani* (f. sp. *glycines* now syn. *F. virguliforme*) and *F. tucumaniae* and *F. brasi*/*iense* each causing sudden death syndrome on soybeans, and *F. verticillioides* on corn; *Gaeumannomyces graminis* (take-all) on cereals (e.g. wheat or barley) and corn; *Gibberella spp.* on cereals (e.g. *G. zeae)* and rice (e.g. *G. fujikuroi:* Bakanae disease); *Glomerella cingulata* on vines, pome fruits and other plants and *G. gossypii* on cotton; Grainstaining complex on rice; *Guignardia bidwellii* (black rot) on vines; *Gymnosporan-gium spp.* on rosaceous plants and junipers, e.g. *G. sabinae* (rust) on pears; *Helminthosporium* spp. (syn. *Drechslera,* teleomorph: *Cochliobolus)* on corn, cereals and rice; *Hemileia* spp., e.g. *H. vastatrix* (coffee leaf rust) on coffee; *Isariopsis davispora* (syn. *Cladosporium vitis)* on vines; *Macrophomina phaseolina* (syn. *phaseoli)* (root and stem rot) on soybeans and cotton; *Microdochium* (syn. *Fusarium) nivale* (pink snow mold) on cereals (e.g. wheat or barley); *Microsphaera diffusa* (powdery mildew) on soybeans; *Monilinia* spp., e.g. *M. laxa, M. fructicola* and *M. fructigena* (bloom and twig blight, brown rot) on stone fruits and other rosaceous plants; *Mycosphaerella* spp. on cereals, bananas, soft fruits and ground nuts, such as e.g. *M. graminicola* (anamorph: *Septoria tritici,* Septoria blotch) on wheat or *M. fijiensis* (black Sigatoka disease) on bananas; *Peronospora spp.* (downy mildew) on cabbage (e.g. *P. brassicae),* rape (e.g. *P. parasitica),* onions (e.g. *P. destructor),* tobacco *(P. tabacina)* and soybeans (e.g. *P. manshurica); Phakopsora pachyrhizi* and *P. meibomiae* (soybean rust) on soybeans; *Phialophora* spp. e.g. on vines (e.g. *P. tracheiphila* and *P. tetraspora)* and soybeans (e.g. *P. gregata:* stem rot); *Phoma lingam* (root and stem rot) on rape and cabbage and *P. betae* (root rot, leaf spot and damping-off) on sugar beets; *Phomopsis* spp. on sunflowers, vines (e.g. *P. viticola:* can and leaf spot) and soybeans (e.g. stem rot: *P. phaseoli,* teleomorph: *Diaporthe phaseolorum); Physoderma maydis* (brown spots) on corn; *Phytophthora* spp. (wilt, root, leaf, fruit and stem root) on various plants, such as paprika and cucurbits (e.g. *P. capsici*)*,* soybeans (e.g. *P. megasperma,* syn. *P. sojae),* potatoes and tomatoes (e.g. *P. infestans:* late blight) and broad-leaved trees (e.g. *P. ramorum:* sudden oak death); *Plasmodiophora brassicae* (club root) on cabbage, rape, radish and other plants; *Plasmopara* spp., e.g. *P. viticola* (grapevine downy mildew) on vines and *P. halstedii on* sunflowers; *Podosphaeraspp.* (powdery mildew) on rosaceous plants, hop, pome and soft fruits, e.g. *P. leucotricha* on apples; *Polymyxa* spp., e.g. on cereals, such as barley and wheat *(P. graminis)* and sugar beets (*P. betae)* and thereby transmitted viral diseases; *Pseudocercosporella herpotrichoides* (eyespot, teleomorph: *Tapesia yallundae)* on cereals, e.g. wheat or barley; *Pseudoperonospora* (downy mildew) on various plants, e.g. *P. cubensis* on cucurbits or *P. humili* on hop; *Pseudopezicula tracheiphila* (red fire disease or ,rotbrenner', anamorph: *Phialophora)* on vines; *Puccinia* spp. (rusts) on various plants, e.g. *P. triticina* (brown or leaf rust), *P. striiformis* (stripe or yellow rust), *P. hordei* (dwarf rust), *P. graminis* (stem or black rust) or *P. recondita* (brown or leaf rust) on cereals, such as e.g. wheat, barley or rye, *P. kuehnii* (orange rust) on sugar cane and *P. asparagi* on asparagus; *Pyrenophora* (anamorph: *Drechslera) tritici-repentis* (tan spot) on wheat or *P. teres* (net blotch) on barley; *Pyricularia* spp., e.g. *P. oryzae* (teleomorph: *Magnaporthe grisea,* rice blast) on rice and *P. grisea* on turf and cereals; *Pythium* spp. (damping-off) on turf, rice, corn, wheat, cotton, rape, sunflowers, soybeans, sugar beets, vegetables and various other plants (e.g. *P. ultimum* or *P. aphanidermatum); Ramularia* spp., e.g. *R. collo-cygni* (Ramularia leaf spots, Physiological leaf spots) on barley and *R. beticola* on sugar beets; *Rhizoctonia* spp. on cotton, rice, potatoes, turf, corn, rape, potatoes, sugar beets, vegetables and various other plants, e.g. *R. solani* (root and stem rot) on soybeans, *R. solani* (sheath blight) on rice or *R. cerealis* (Rhizoctonia spring blight) on wheat or barley; *Rhizopus stolonifer* (black mold, soft rot) on strawberries, carrots, cabbage, vines and tomatoes; *Rhynchosporium seca*/*is* (scald) on barley, rye and triticale; *Sarociadium oryzae* and *S. attenuatum* (sheath rot) on rice; *Sclerotinia* spp. (stem rot or white mold) on vegetables and field crops, such as rape, sunflowers (e.g. *S. sclerotiorum)* and soybeans (e.g. *S. rolfsii* or *S. sclerotiorum); Septoriaspp.* on various plants, e.g. *S. glycines* (brown spot) on soybeans, *S. tritici* (Septoria blotch) on wheat and *S.* (syn. *Stagonospora)* nodorum (Stagonospora blotch) on cereals; *Uncinula* (syn. *Erysiphe) necator(powdery* mildew, anamorph: *Oidium tuckeri)* on vines; *Setospaeria* spp. (leaf blight) on corn (e.g. *S. turcicum,* syn. *Helminthosporium turcicum)* and turf; *Sphacelotheca* spp. (smut) on corn, (e.g. *S. reiliana:* head smut), sorghum und sugar cane; *Sphaerotheca fuliginea* (powdery mildew) on cucurbits; *Spongospora subterranea* (powdery scab) on potatoes and thereby transmitted viral diseases; *Stagonospora* spp. on cereals, e.g. *S. nodorum* (Stagonospora blotch, teleomorph: *Leptosphaeria* [syn. *Phaeosphaeria*] *nodorum)* on wheat; *Synchytrium endobioticum* on potatoes (potato wart disease); *Taphrinaspp.,* e.g. *T. deformans* (leaf curl disease) on peaches and *T. pruni* (plum pocket) on plums; *Thielaviopsis* spp. (black root rot) on tobacco, pome fruits, vegetables, soybeans and cotton, e.g. *T. basicola* (syn. *Chalara elegans); Tilletia* spp. (common bunt or stinking smut) on cereals, such as e.g. *T. tritici*(syn. *T. caries,* wheat bunt) and *T. controversa* (dwarf bunt) on wheat; *Typhula incarnata* (grey snow mold) on barley or wheat; *Urocystis* spp., e.g. *U. occulta* (stem smut) on rye; *Uromyces* spp. (rust) on vegetables, such as beans (e.g. *U. appendiculatus,* syn. *U. phaseoli)* and sugar beets (e.g. *U. betae); Ustilago* spp. (loose smut) on cereals (e.g. *U. nuda* and *U. avaenae),* corn (e.g. *U. maydis:* corn smut) and sugar cane; *Venturia* spp. (scab) on apples (e.g. *V. inaequalis)* and pears; and *Verticillium* spp. (wilt) on various plants, such as fruits and ornamentals, vines, soft fruits, vegetables and field crops, e.g. V. *dahliae* on strawberries, rape, potatoes and tomatoes.

In particular, these dsRNA molecules and antisense-ssRNA are capable of inhibiting the expression of at least one CYP51 gene of a fungal or oomycete phytopathogen selected from the genera *Fusarium, Blumeria, Magnaporthe, Sclerotinia, Phakopsora, Botrytis, Puccinia, Pyrenophora, Phaeosphaeria, Septoria, Cochliobolus, Ustilago, Rhynchosporium* and *Venturia.* Particular phytopthogens of these genera and their CYP51 genes are listed in Table 1 below. Preferably, the phytopathogen is a fungus of the genus *Fusarium.*

**Table 1: Fungal and oomycete phytopathogens and CYP51 genes thereof**

| Phytopathogen | Diseases | CYP51 gene(s) [SEQ ID NO of coding sequence] |
|---|---|---|
| *Fusarium graminearum* (*Fg)* | head blight on wheat, barley, and other cereal crops | CYP51A [1] (FGSG_04092.3)¹ |
| | | CYP51B [2] (FGSG_01000.3)¹ |
| | | CYP51C [3] (FGSG_11024.3)¹ |
| *Fusarium oxysporum* | wilt of tomato and other crops | CYP51A [8] (FOXG_11545.2)¹ |
| | | CYP51B [9] (FOXG_00394.2)¹ |
| | | CYP51C [10] (FOXG_13138.2)¹ |
| *Fusarium verticillioides* | ear rot of corn | CYP51A [11] (FVEG_10277.3) 1 |
| | | CYP51B [12] (FVEG_01123.3)¹ |
| | | CYP51C [13] (FVEG_12391.3)¹ |
| *Blumeria graminis* | Powdery mildew on grasses | CYP51 [14] |
| *Magnaporthe grisea* | Rice seedling blight, blast of rice, oval leaf spot of graminea, pitting disease, ryegrass blast, and Johnson spot | CYP51A [15] |
| | | CYP51B [16] |
| *Sclerotinia sclerotinium* | White mold, cottony rot, watery soft rot, stem rot, drop, crown rot and blossom blight | CYP51B [17] |
| *Phakopsora pachyrhizi* | Asian soybean rust | CYP51 [18] |
| *Botrytis cinerea* | Blossom blights and fruit rots but also as leaf spots and bulb rots | CYP51B [19] |
| *Puccinia triticina* | Rust disease on cereals | CYP51 [20] |
| *Puccinia graminis* | Rust disease on cereals | CYP51 [21] |
| *Puccinia recondita* | Rust disease on cereals | CYP51 [22] |
| *Pyrenophora teres* | Blotch of barley, net blotch, and spot form net blotch | CYP51 [23] |
| *Pyrenophora tritici* | Tan spot or helminthosporiosis on cereals | CYP51A [24] |
| | | CYP51B [25] |
| *Phaeosphaeria nodorum* | Glume blotch and Septoria nodorum blotch | CYP51B [26] |
| *Septoria tritici* | Septoria leaf blotch | CYP51 [27] |
| *Cochliobolus sativus* | Spot blotch and common root rot | CYP51B [28] |
| *Ustilago maydis* | Smut disease on maize | CYP51 [29] |
| *Rhynchosporium secalis* | Barley and rye scald | CYP51 [30] |
| *Venturia inaequalis* | Apple scab disease | CYP51 [31] |

| | | |
|---|---|---|
| ¹ The gene designations refer to the loci in the Fusarium Comparative Database accessible *via,* e.g., the website of the Broad Institute (http://www.broadinstitute.org). | | |

The dsRNA molecule is capable of inhibiting the expression of one, two, three, or all CYP51 genes of a fungal or oomycete phytopathogen, and preferably is capable of inhibiting all CYP51 genes of the phytopathogen. A dsRNA molecule described herein is capable of inhibiting the expression of the CYP51A gene, the CYP51B gene and the CYP51C gene of a phytopathogenic fungus of the genus *Fusarium.*

For example, the CYP51 gene(s) to be inhibited by the dsRNA molecule described herein is/are selected from the CYP51 genes of a phytopathogen listed in Table 1.

The dsRNA molecule can be stabilized against unwanted chemical and enzymatic degradation by reducing the dissociation of double-stranded RNA into single-stranded, non-hybridized RNA. To this end, the two hybridizing ribonucleotide sequences or regions adjacent to them can be chemically linked to each other as described, e.g., in US 2008/0171861 A1. Chemical linkage groups suitable for stabilizing regions of double stranded RNA include, but are not limited to, purine analogs replacing purines and branched nucleotide analogs replacing nucleotides. The dsRNA molecule can also be stabilized against unwanted enzymatic degradation by ribonucleotide modifications. Suitable ribonucleotide modifications include the replacement of the 2'-hydroxyl group of one or more than one ribonucleotide by, preferably, a 2'-amino or 2'-methyl group; and the replacement of one or more than one ribonucleotide by the same number of corresponding locked nucleotides, wherein the sugar ring is chemically modified, preferably by a 2'-O 4'-C methylene bridge.

The dsRNA molecule described herein comprises a sense sequence having a high degree of identity to at least part of the coding sequence of at a CYP51 gene from a fungal or oomycete phytopathogen, and an antisense sequence being substantially complementary to said sense sequence. In this context, "a sense sequence" refers to one or more than one sense sequence, and "a CYP51 gene" refers to one or more than one CYP51 gene. Expediently, the antisense sequences encompass a substantially complementary antisense sequence for each sense sequence comprised by the same dsRNA molecule. Generally, terms such as "a sense sequence", "an antisense sequence", "a polynucleotide", "a CYP51 gene", when used herein, include the singular as well as the plural, unless stated otherwise.

Accordingly, the dsRNA molecule described herein comprises
a first sense sequence having a high degree of identity to at least part of the coding sequence of a first CYP51 gene, and
a second sense sequence having a high degree of identity to at least part of the coding sequence of a second CYP51 gene,
wherein the first CYP51 gene and the second CYP51 gene are different CYP51 genes of a fungal or oomycete phytopathogen.

More specifically, the dsRNA molecule described herein comprises
a first sense sequence having a high degree of identity to at least part of the coding sequence of a first CYP51 gene,
a second sense sequence having a high degree of identity to at least part of the coding sequence of a second CYP51 gene, and
a third sense sequence having a high degree of identity to at least part of the coding sequence of a second CYP51 gene,
wherein the first CYP51 gene, the second CYP51 gene and the third CYP51 gene are different CYP51 genes of a fungal or oomycete phytopathogen.

The coding sequence of the at least one CYP51 gene, e.g. the first, the second and the third CYP51 gene, can be selected from the coding sequences of the CYP51 genes listed in Table 1.

According to one aspect described herein, the dsRNA molecule is capable of inhibiting the expression of at least one CYP51 gene from *Fusarium graminearum,* and comprises a sense sequence having a high degree of identity to at least part of a sequence selected from SEQ ID NOs:1-6, and an antisense sequence being substantially complementary to said sense sequence; wherein the arrangement of the sequences within the dsRNA molecule allows hybridization of said sense sequence and said corresponding antisense sequence. According to particular embodiments of said aspect of the disclosure, the dsRNA molecule:
(a) is capable of inhibiting the expression of the CYP51A gene from *Fusarium graminearum* and comprises a sense sequence having a high degree of identity to at least part of SEQ ID NO:1 or SEQ ID NO:4;
(b) is capable of inhibiting the expression of the CYP51B gene from *Fusarium graminearum* and comprises a sense sequence having a high degree of identity to at least part of SEQ ID NO:2 or SEQ ID NO:5;
(c) is capable of inhibiting the expression of the CYP51C gene from *Fusarium graminearum* and comprises a sense sequence having a high degree of identity to at least part of SEQ ID NO:3 or SEQ ID NO:6;
(d) is characterized by (a) and (b);
(e) is characterized by (a) and (c);
(f) is characterized by (b) and (c); or
(g) is characterized by (a), (b) and (c).

According to another aspect described herein, the dsRNA molecule is capable of inhibiting the expression of at least one CYP51 gene from *Fusarium graminearum* and comprises a sense sequence that is at least 70%, at least 75%, at least 80%, at least 85%, at least 90%, preferably at least 95% or at least 98%, and most preferably at least 99% or 100% identical to SEQ ID NO:7, and an antisense sequence being substantially complementary to said sense sequence; wherein the arrangement of the sequences within the dsRNA molecule allows hybridization of said sense sequence and said corresponding antisense sequence. According to particular embodiments of said aspect of the disclosure, the dsRNA molecule consists of said sense and said antisense sequence. For example, said dsRNA molecule may consist of a first RNA strand having said sense sequence and a second RNA strand having said antisense sequence.

According to a further aspect described herein, the dsRNA molecule is capable of inhibiting the expression of at least one CYP51 gene from *Fusarium oxysporum,* and comprises a sense sequence having a high degree of identity to at least part of a sequence selected from SEQ ID NOs:8-10, and an antisense sequence being substantially complementary to said sense sequence; wherein the arrangement of the sequences within the dsRNA molecule allows hybridization of said sense sequence and said corresponding antisense sequence. According to particular embodiments of said aspect of the disclosure, the dsRNA molecule:
(a) is capable of inhibiting the expression of the CYP51A gene from *Fusarium oxysporum* and comprises a sense sequence having a high degree of identity to at least part of SEQ ID NO:8;
(b) is capable of inhibiting the expression of the CYP51B gene from *Fusarium oxysporum* and comprises a sense sequence having a high degree of identity to at least part of SEQ ID NO:9;
(c) is capable of inhibiting the expression of the CYP51C gene from *Fusarium oxysporum* and comprises a sense sequence having a high degree of identity to at least part of SEQ ID NO:10;
(d) is characterized by (a) and (b);
(e) is characterized by (a) and (c);
(f) is characterized by (b) and (c); or
(g) is characterized by (a), (b) and (c).

According to a further aspect described herein, the dsRNA molecule is capable of inhibiting the expression of at least one CYP51 gene from *Fusarium verticillioides,* and comprises a sense sequence having a high degree of identity to at least part of a sequence selected from SEQ ID NOs:11-13, and an antisense sequence being substantially complementary to said sense sequence; wherein the arrangement of the sequences within the dsRNA molecule allows hybridization of said sense sequence and said corresponding antisense sequence. According to particular embodiments of said aspect described herein, the dsRNA molecule:
(a) is capable of inhibiting the expression of the CYP51A gene from *Fusarium verticillioides* and comprises a sense sequence having a high degree of identity to at least part of SEQ ID NO:11;
(b) is capable of inhibiting the expression of the CYP51B gene from *Fusarium verticillioides* and comprises a sense sequence having a high degree of identity to at least part of SEQ ID NO:12;
(c) is capable of inhibiting the expression of the CYP51C gene from *Fusarium verticillioides* and comprises a sense sequence having a high degree of identity to at least part of SEQ ID NO:13;
(d) is characterized by (a) and (b);
(e) is characterized by (a) and (c);
(f) is characterized by (b) and (c); or
(g) is characterized by (a), (b) and (c).

According to a another aspect as described herein, the dsRNA molecule is capable of inhibiting the expression of the CYP51A gene, the CYP51B gene and the CYP51C gene from a phytopathogenic *Fusarium* species; and said dsRNA molecule comprises
(i) a sense sequence having a high degree of identity to at least part of the coding sequence of said CYP51A gene;
(ii) a sense sequence having a high degree of identity to at least part of the coding sequence of said CYP51B gene;
(iii) a sense sequence having a high degree of identity to at least part of the coding sequence of said CYP51C gene; and
(iv) antisense sequences being substantially complementary to the sense sequences (i), (ii), and (iii);
wherein the arrangement of the sequences within the dsRNA molecule allows hybridization of each sense sequence with its substantially complementary antisense sequence.

In aspects where the phytopathogenic *Fusarium* species is *Fusarium graminearum,* the coding sequence of the CYP51 genes may be the sequences set forth in SEQ ID NO:1 (CYP51A), SEQ ID NO:2 (CYP51B) and SEQ ID NO:3 (CYP51C).

In aspects where the phytopathogenic *Fusarium* species is *Fusarium oxysporum,* the coding sequence of the CYP51 genes may be the sequences set forth in SEQ ID NO:8 (CYP51A), SEQ ID NO:9 (CYP51B) and SEQ ID NO:10 (CYP51C).

In aspects where the phytopathogenic *Fusarium* species is *Fusarium verticillioides,* the coding sequence of the CYP51 genes may be the sequences set forth in SEQ ID NO:11 (CYP51A), SEQ ID NO:12 (CYP51B) and SEQ ID NO:13 (CYP51C).

According to a further aspect as described herein, the dsRNA molecule is capable of inhibiting the expression of the CYP51A gene, the CYP51B gene and the CYP51C gene from *Fusarium graminearum,* and said dsRNA molecule comprises a sense sequence (i) having a high degree of identity to at least part of SEQ ID NO:4, a sense sequence (ii) having a high degree of identity to at least part of SEQ ID NO:5, a sense sequence (iii) having a high degree of identity to at least part of SEQ ID NO:6, and
antisense sequences (iv) being substantially complementary to the sense sequences (i), (ii), and (iii);
wherein the arrangement of the sequences within the dsRNA molecule allows hybridization of each sense sequence with its substantially complementary antisense sequence.

The expression "at least part of" SEQ ID NO:4, SEQ ID NO:5 or SEQ ID NO:6 refers to at least 20 contiguous nucleotides, for example of at least 25, at least 50, at least 100, at least 150, and preferably at least 200 contiguous nucleotides of SEQ ID NO:4, SEQ ID NO:5 or SEQ ID NO:6. According to a particular aspect, the high degree of identity of the sense sequences (i), (ii) and (iii) pertains to the entire length of SEQ ID NO:4, SEQ ID NO:5 and SEQ ID NO:6, respectively.

Due to the sequence homology of the CYP51A, CYP51B and CYP51C genes from *Fusarium* species, dsRNA molecules can be designed, wherein the sense sequences have a high degree of identity to at least part of the coding sequences of the CYP51A genes, the CYP51B genes and the CYP51C genes, respectively, from more than one phytopathogenic *Fusarium* species. For example, the sense sequences of the dsRNA can have a high degree of identity to at least part of the coding sequence of the CYP51A genes, the CYP51B genes and the CYP51C genes, respectively, from two or more *Fusarium* species selected from *F. graminearum, F. oxysporum* and *F. verticil lioides.*

The sense and the antisense sequences are arranged within the dsRNA molecule in a manner that allows hybridization of each sense sequence with its substantially complementary antisense sequence, thus forming regions of double-stranded RNA.

Each sense sequence is located on a different RNA strand of the dsRNA molecule than its substantially complementary antisense sequence. Thus, the sense and antisense sequences can be located on two or more than two separate strands of the dsRNA molecule. Upon hybridization of the substantially complementary sequences the strands are joined through base pairing to form one or more than one region of double-stranded RNA. The sense sequences can be on separate strands of the dsRNA molecule. In particular, the substantially complementary sense and antisense sequences of the dsRNA molecule of the disclosure are located on two separate substantially complementary strands of the dsRNA molecule.

Alternatively, the substantially complementary sense and antisense sequences of the dsRNA molecule as described herein are located on a single strand of the dsRNA molecule. The substantially complementary sequences are arranged on the RNA single strand in a manner that upon their hybridization the strand is looped back on itself through base pairing to form one or more than one hairpin structure. A hairpin structure consists of a double-stranded stem formed by hybridization of a first polynucleotide segment with a second polynucleotide segment, and a singled-stranded loop linking the two segments.

The hybridized sense and antisense sequences of the dsRNA molecule as described herein can be comprised within one contiguous region of double-stranded RNA. According to a preferred embodiment, the dsRNA molecule as described herein comprises a region of double-stranded RNA consisting essentially (i.e. to at least 80%, at least 90%, or 100%) of the sense and antisense sequences, most preferably consisting of one sense sequence (i), one sense sequence (ii), and one sense sequence (iii), each hybridized to a substantially complementary antisense sequence.

The order of the regions of double-stranded RNA comprising the sense sequences (i), (ii) and (iii) is not particularly restricted. For example, the double-stranded RNA formed by sense sequence (i) and its antisense sequence is flanked on one side by the double-stranded RNA formed by sense sequence (ii) and its antisense sequence and on the other side by the double-stranded RNA formed by sense sequence (iii) and its antisense sequence.

The dsRNA molecules as described herein are capable of inhibiting the expression of CYP51 gene(s) of the fungal or oomycete phytopathogen. The inhibition of CYP51 gene expression by dsRNA molecules of the present disclosure becomes detectable on the level of mRNA, on the level of polypeptide or both. For example, the change in the mRNA level of a CYP51 gene can be assessed by quantitative real-time PCR (qRT-PCR) or Northern Blot.

The dsRNA molecules as described herein are effective in inhibiting the expression of CYP51 gene(s) of the fungal or oomycete phytopathogen. The generation of dsRNA molecules as described herein within a plant inoculated with the fungal or oomycete phytopathogen can result in mRNA levels of the CYP51 gene(s) which are at least 50%, at least 60%, at least 70% or, preferably, at least 75% lower compared to those in a control plant not generating RNA molecules as described herein. For example, such change in fungal or oomycete CYP51 mRNA levels (e.g. FgCYP51A, CYP51B and CYP51C mRNA levels) can be determined in *Arabidopsis thaliana* leaves inoculated three days earlier with the fungus or oomycete (e.g. *Fg* macroconidia) from which the CYP51 gene(s) defining the sense sequences of the dsRNA molecule originate(s). For example, such change in fungal or oomycete CYP51 mRNA levels (e.g. *Fg* CYP51A, CYP51B and CYP51C mRNA levels) can alternatively be determined in barley leaves inoculated 7 days earlier with the fungus or oomycete (e.g. *Fg* macroconidia) from which the CYP51 gene(s) defining the sense sequences of the dsRNA molecule originate(s).

The dsRNA molecules as described herein are useful for controlling fungal or oomycete phytopathogens, e.g. phytopathogens as listed in Table 1. In particular, dsRNA molecules as described herein are suitable for controlling fungi from the genus *Fusarium.* According to a particular aspect, the phytopathogenic fungus to be controlled is selected from *Fusarium graminearum, Fusarium oxysporum* and *Fusarium verticil lioides.*

A dsRNA molecule as described herein can be capable of inhibiting the expression of two or more fungal and/or oomycete phytopathogens, and accordingly can be useful for controlling said two or more different phytopathogen species.

Controlling a phytopathogenic fungus or oomycetes refers to measures for preventing the infestation of a plant with said pathogen as well as measures for combatting or curing the pathogen infestation of a plant. The infestation of a plant with a fungal or oomycete phytopathogen can become visible as a plant disease. Accordingly, the uses and methods of the present disclosure for controlling a phytopathogenic fungus or oomycete are uses and methods for preventing and/or treating plant diseases caused by the phytopathogenic fungus or oomycete, e.g. those listed in Table 1.

The present disclosure also provides antisense-ssRNA comprising the antisense sequences but not the sense sequences of the dsRNA molecule as described herein. Such antisense-ssRNA is capable of inhibiting the expression of CYP51 gene(s) of the fungal or oomycete phytopathogen and is useful for controlling said pathogen in a manner comparable to the dsRNA molecules of the disclosure.

For controlling a phytopathogenic fungus or oomycete, the dsRNA molecules as described herein can be applied to a plant infested by or at risk of being infested by the phytopathogen and/or the vicinity of said plant. The dsRNA molecule as described herein is expediently applied in the form of a composition. Accordingly, a composition for controlling a phytopathogenic fungus or oomycete comprising the dsRNA molecule of the disclosure and a plant-compatible carrier, is provided herein.

Plant-compatible carriers can be liquids, solids or mixtures thereof, for example selected from water, alcohols (e.g. butanol organic solvents), glycols, fatty acids, mineral and plant oils, derivatives of mineral and plant oils, natural minerals (e.g. kaolins, clays, talc, chalk) and synthetic minerals (e.g. finely divided silica, silicates), waxes, solid fertilizers, cellulose derivatives (e.g. methylcellulose), and mixtures thereof. The carrier is expediently chosen so as to be compatible with the application of the composition to the plant to be protected or cured, or the vicinity thereof. For example, in liquid compositions, the carrier may include at least one surfactant (i.e. a compound modifying surface tension) to positively modify the dispersion, suspension or precipitation of the ingredients, and/or to improve wetting and spreading of the composition.

Such composition comprises isolated or purified dsRNA molecules as described herein. According to alternative embodiments, the composition of the disclosure comprises host cells, e.g. bacterial or yeast cells, capable of expressing dsRNA molecules of the invention, or an extract of such cells.

Such composition can comprise further agents including
RNA protecting agents that reduce or prevent unwanted chemical and/or enzymatic degradation of the dsRNA molecules, e.g. RNase inhibitors;
compounds which enhance or promote uptake of the dsRNA by the phytopathogen, e.g. compounds which generally promote the uptake of RNA into cells such as lipofectamin; and
phytopharmaceutical and/or plant growth promoting compounds, e.g., selected from fungicides, herbicides, insecticides, nematicides, acaricides, molluscicides, and pheromone active substances.

Such compositions comprising such phytopharmaceutical and/or plant growth promoting agents can exert a broadened spectrum of biological activity. Particularly advantageous are compositions which comprise dsRNA molecules as described herein and a plant-compatible carrier together with one or more than one further fungicide.

Such compositions are also suitable for controlling harmful fungi in the protection of stored products or harvest and in the protection of materials. The term "protection of materials" is to be understood to denote the protection of technical and non-living materials, such as adhesives, glues, wood, paper and paperboard, textiles, leather, paint dispersions, plastics, cooling lubricants, fiber or fabrics, against the infestation and destruction by harmful microorganisms, such as fungi. As to the protection of wood and other materials, the particular attention is paid to the following harmful fungi: Ascomy-cetes such as *Ophiostoma* spp., *Ceratocystis* spp., *Aureobasidium pullulans, Sclerophoma* spp., *Chaetomium* spp., *Humicola* spp., *Petriella* spp., *Trichurus* spp.; Basidiomycetes such as *Coniophora* spp., *Coriolus* spp., *Gloeophyllum* spp., *Lentinus* spp., *Pleurotus* spp., *Poria* spp., *Serpula* spp. and *Tyromyces* spp., Deuteromycetes such as *Aspergillus* spp., *Cladosporium* spp., *Penicillium* spp., *Trichorma* spp., *Alternaria* spp., *Paecilomyces* spp. and Zygomycetes such as *Mucor* spp*.,* and in addition in the protection of stored products and harvest the following yeast fungi are worthy of note: *Candida* spp. and *Saccharomyces cerevisae.*

The form of such composition is expediently adapted to the desired mode of application. Suitable forms include ready-to-use compositions as well as concentrates. For example, the composition can have the form of a sprayable liquid, a dustable powder, an emulsifiable concentrate, granules (e.g. coated, encapsulated or impregnated granules), a paste, or a water-dispersable or water-soluble powder for seed treatment. Methods for preparing such compositions are known in the art.

Described herein is also a kit comprising dsRNA molecules as described herein, or a host cell capable of expressing dsRNA molecules as described herein, or the ingredients of a composition, or a composition as described herein. The kit may be supplied with suitable instructions for use.

The present disclosure provides the use of dsRNA molecules as described herein, or a host cell capable of expressing dsRNA molecules as described herein, or a composition as described herein for controlling the phytopathogenic fungus or oomycete.

The present invention further provides methods for controlling the phytopathogenic fungus or oomycete, wherein a plant infested by or at risk of being infested by the fungus and/or the vicinity of said plant is contacted with the composition.

Application methods for contacting a plant and/or the vicinity thereof with a composition are known in the art. Suitable application methods include, but are not limited to, spraying a liquid composition, dusting a powdery composition, incorporating a powdery or granular composition into the soil, daubing a pasty composition, and coating or film-coating plant seeds with the composition.

The dose of dsRNA molecules applied in the method of the present invention is typically from 0.0001 to 10,000 g/ha, preferably from 0.0001 to 1,000 g/ha and more preferably from 0.001 to 300 g/ha for foliar treatment; from 0.0001 to 200 g composition per 100 kg seed, preferably from 0.001 to 150 g per 100 kg of seed; and from 0.0001 to 10,000 g/ha and preferably from 0.0001 to 5,000 g/ha for soil treatment.

The dsRNA molecules can be prepared by methods known in the art such as by classical chemical synthesis, by *in vitro* transcription methods, or by heterologous expression in, e.g., microorganisms (bacteria, yeasts), cell cultures or plants. Accordingly, transcription can be mediated by an endogenous RNA polymerase of the host cell *in vivo,* or by a cloned RNA polymerase for transcription *in vivo* or *in vitro.* The dsRNA molecules as described herein can be purified or isolated dsRNA. For example, RNA can be purified from a mixture by extraction with a solvent or resin, precipitation, electrophoresis, chromatography, or a combination thereof. Alternatively, the dsRNA may be used with no or only a minimum of purification to avoid losses due to sample processing. The RNA may be dried for storage or dissolved in an aqueous solution. The solution may contain buffers or salts to promote annealing, and/or stabilization of double-stranded RNA regions.

In the method of the present invention for controlling a phytopathogenic fungus or oomycete by applying a composition of the disclosure, the plant infested by or at risk of being infested by the phytopathogen and/or the vicinity of said plant is expediently contacted with an effective amount of the composition. An "effective amount" of a composition of the disclosure, is an amount comprising an amount of dsRNA molecules as described herein that is sufficient to control the fungus. Such effective amount can vary depending on the phytopathogen to be controlled, the plant to be protected (species, variety, developmental stage), the climatic conditions and the further components of the composition. The effective amount of the composition as described herein can be determined by systematic field trials which are routine and well within the capabilities of a person skilled in the art.

The present invention also provides means and methods for controlling a phytopathogenic fungus or oomycete by generating the dsRNA molecules or the antisense-ssRNA as described herein within a plant to be protected from the fungus. Accordingly, the present invention provides a method for preparing a transgenic plant that is tolerant to a phytopathogenic fungus or oomycete by introducing into a plant a transcriptional template of at least the antisense sequences (iv) of the dsRNA molecule as described herein.

A plant to be protected from or to be treated against infestation with a phytopathogen as described herein, e.g. a transgenic plant according to the present invention, can be a monocotyledonous or a dicotyledonous plant.

Examples of monocotyledonous plants are plants belonging to the genera *Avena* (oat), *Triticum* (wheat), *Secale* (rye), *Hordeum* (barley), *Oryza* (rice), *Panicum, Pennisetum, Setaria, Sorghum* and *Zea* (maize).

Examples of dicotyledonous plants include cotton, leguminous plants, in particular alfalfa and soy bean, rape, tomato, sugar beet, potato, ornamental plants, and trees. Further useful plants include fruit (in particular apples, pears, cherries, grapes, citrus, pineapple, and bananas), pumpkin, cucumber, wine, oil palms, tea shrubs, cacao trees, and coffee shrubs, tobacco, sisal, as well as, with medicinal plants, rauwolfia and digitalis.

The transgenic or non-transgenic plant used in the phytopathogen-controlling methods of the present invention herein is preferably selected from barley, maize, wheat, soy, oat, rye, rice, millet, sugar beet, rape, tomato, potato, cotton and tobacco; more preferably selected from barley, maize, wheat, soy, oat, rye and rice plants; and most preferably selected from barley, maize and wheat plants.

The present disclosure provides a DNA sequence or a multitude of DNA sequences providing a transcriptional template of at least the antisense sequences (iv) of the RNA molecule of the disclosure. According to a preferred embodiment, the DNA sequence(s) provide a transcriptional template of the dsRNA molecule as described herein, i.e. including the antisense sequence(s) as well as the sense sequence(s) described herein. Alternatively, the transgenic plant of the invention comprises a transcriptional template of only the antisense sequence(s), i.e. a transcriptional template of the antisense-ssRNA of the disclosure.

The DNA sequence(s) can further comprise at least one promoter. The promoter comprised by the DNA sequence(s) of the disclosure is operably linked to the transcriptional template so as to allow the generation of the antisense-ssRNA or the dsRNA molecule of the present disclosure by transcription. Preferably, said promoter is a promoter that is functional in a plant cell to allow generation of the antisense-ssRNA or the dsRNA molecule as described herein by a plant. The promoter can be selected from constitutive promoters and regulated promoters.

Non-limiting examples of constitutive promoters include the 35S promoter and the 19S promoter from cauliflower mosaic virus (CaMV35S and CaMV19S; US 6255560), an ubiquitin promoter such as Arabidopsis ubiquitin-10 promoter (UBQ10) (EP 1210446, EP 342926) an opine promoter (EP 729514), and an active promoter such as the rice actin 1 promoter (Act-1).

Regulated promoters include inducible promoters, tissue-specific promoters and development-specific promoters. Non-limiting examples of chemically regulated inducible promoters include the alcohol dehydrogenase I gene promotor (alcA) (EP 637339); tetracycline-responsive promoter systems, e.g. promoter systems including a tetracycline repressor protein (TetR), a tetracycline operator sequence and a tetracycline transactivator fusion protein, which is the fusion of TetR and a herpes simplex virus protein 16 (VP16) activation sequence (US 6242667 and US 6252136); steroid-responsive promoters, e.g. promoters based on ecdysone receptors, human estrogen receptor or rat glucocorticoid receptor (EP 1232273, EP 1242604, US 6379945, EP 1112360); metal-regulated promoters, e.g., derived from metallothionein; and promoters derived from pathogenesis-releated proteins, e.g. from Arabidopsis or maize (US 5689044, EP 1056862). Non-limiting examples of physically regulated inducible promoters include cold- and heat-shock-induced promoters (EP 159884, EP 787194) as wells as light-inducible promoters (EP 310619) and light-repressible promoters (US 5639952, EP 1077257). Non-limiting examples of tissue-specific promoters include root-specific promoters (EP 1248850), fruit promoters (EP 316441, EP 968292, EP 973922), and seed promoters (EP 255378, EP 781849, US 6642437, EP 1019517). Particularly suitable regulated promoters for the present invention are promoters induced by the presence of a phytopathogenic fungus or oomycete but not by useful organisms such as the mycorrhiza; and promoters which are active on the site of the entry of the phytopathogenic fungus or oomycete, such as epidermis-specific promoters. Inducible promoters can be used to transiently generate the antisense ssRNA or the dsRNA molecule of the disclosure, e.g. at times when there is an acute or increased risk of infestation by fungal or oomycete phytopathogens.

Described herein is also an isolated polynucleotide having or comprising the DNA sequence(s) of the present disclosure, or a multitude of isolated polynucleotides having or comprising the DNA sequences of the present disclosure. Said isolated polynucleotides can be transformation vectors useful for introducing DNA sequences into a plant. Such plant transformation vectors include plasmids (e.g., Ti plasmids, Ri plasmids, plasmids of the pUC series or the M13mp series, pBR322), viral vectors (e.g. derived from potato virus X, tobacco rattle virus, Gemini virus) and other vectors known in the art of genetic engineering. The plant transformation vectors can comprise DNA sequences which facilitate plant transformation and/or selection, such as sequences encoding plant selection markers (e.g. the bar gene) and T-DNA sequences.

Described herein are also methods for preparing a transgenic plant comprising the introduction of the DNA sequence(s) of the disclosure into the plant. The DNA sequence(s) can be introduced in the form of the isolated polynucleotide(s) of the invention (e.g. in the form of plant transformation vectors). A number of well-known methods are available for introducing polynucleotides into a plant cell. Suitable plant transformation methods include, but are not limited to, plant transformation by means of T-DNA and *Agrobacterium tumefaciens* or *Agrobacterium rhizogenes* (EP 0116718), plant transformation using viral vectors and viral infection (EP 0067553, US 4407956, WO 9534668, WO 9303161), plant transformation by means of pollen (EP 0270356), fusion of protoplasts, polyethylene glycol-induced DNA uptake, liposome-mediated transformation (US 4536475), biolistic DNA introduction (Fromm et al., Bio/Technology 8(9):833-9, 1990), electroporation, microinjection, and incubation of dry plant embryos in DNA-comprising solution.

A transgenic plant of the present invention is a plant, wherein at least one cell of the plant contains a DNA sequence or multitude of DNA sequences providing a transcriptional template of at least the antisense sequence(s) of the dsRNA molecule of the disclosure (i.e. a transcriptional template for the antisense-ssRNA or the dsRNA molecule of the invention). Preferably, said DNA sequence(s) is/are stability integrated into a chromosome or a stable extra-chromosomal element of the transgenic plant, so that the DNA sequence(s) can be passed on to the progeny of the transgenic plant. The tolerance to phytopathogenic fungi and/or oomycetes conferred by the DNA sequence(s) of the disclosure can thus be inherited by the progeny of the transgenic plant. The transgenic plant of the present invention is preferably a cereal crop, e.g. a barley plant, capable of generating antisense-ssRNA or dsRNA molecules for controlling one or more than one phytopathogen, in particular selected from phytopathogenic fungi of the genus *Fusarium,* e.g. *F. graminearum.*

In addition to direct transformation of a plant with recombinant DNA sequence(s) of the disclosure, transgenic plants can be prepared by selfing plants having said recombinant DNA sequence(s), or by crossing a first plant having said recombinant DNA sequence(s) with a second plant lacking such recombinant DNA sequence. The resulting seed can be used to grow progeny plants including plant lines comprising the DNA sequence(s) of the present disclosure.

For example, DNA sequence(s) of the disclosure can be introduced into a first plant line that is amenable to transformation to produce a transgenic plant that can be crossed with a second plant line to introduce said DNA sequence(s) into the second plant line. It may be advantageous to express DNA sequence(s) of the disclosure in a male-sterile plant, for example, as a means for reducing concern about transgene flow to neighboring plants. The present invention can be, in practice, combined with other anti-pathogen traits in a plant.

According to a preferred embodiment, the transgenic plant of the invention is capable of generating the antisense-ssRNA or the dsRNA molecule of the disclosure. Said antisense-ssRNA and the dsRNA molecule of the disclosure are capable of inhibiting the expression of the CYP51 gene(s) of the fungal or oomycete phytopathogen, like the CYP51A, CYP51B and CYP51C genes of a phytopathogenic fungus of the genus *Fusarium.* The mRNA levels of said CYP51 gene(s) in a transgenic plant of the invention inoculated with the phytopathogen can be at least 50%, at least 60%, at least 70% or, preferably, at least 75% lower compared to those in a control plant not generating antisense-ssRNA or dsRNA molecules of the disclosure.

Such change in mRNA levels of the CYP51 gene(s) can be determined, e.g., in plant leaves inoculated three days earlier with the phytopathogen (e.g. *Fusarium* macroconidia). The control plant is a plant that is sensitive to the phytopathogen, and is grown and inoculated with the phytopathogen like the transgenic plant it is compared with. The control plant can be a plant that differs from the transgenic plant of the invention in lacking a transcriptional template of the antisense-ssRNA or the dsRNA molecule of the disclosure.

For example, a transgenic plant comprising an empty vector or marker gene but not DNA sequence(s) of the disclosure or a non-transgenic (i.e. wild-type) plant can be used as control plant. Expediently, the expression profiles of the transgenic plant of the invention and the corresponding control plant differ only in the generation of the antisense-ssRNA or the dsRNA molecules of the disclosure, or lack thereof.

The present invention also provides methods for controlling the phytopathogenic fungus or oomycete, wherein the methods comprise the cultivation of a transgenic plant of the invention to allow the generation of the antisense-ssRNA or the dsRNA molecule of the disclosure by the plant (i.e. the generation of RNA comprising at least the antisense sequence(s) of the dsRNA molecules of the disclosure).

The generation of antisense-ssRNA or dsRNA molecules of the disclosure by the transgenic plant can confer tolerance to the phytopathogenic fungus or oomycete. In a phytopathogen-tolerant transgenic plant of the present invention, the severity and/or frequency of the infestation with the phytopathogen can be reduced by at least 5%, at least 20%, at least 50%, at least 60%, preferably at least 75%, at least 80%, at least 90%, at least 95% or 100% compared to a corresponding control plant. Alternatively, the pathogen-tolerance of a transgenic plant of the present invention can be described by reference to a relative susceptibility index which compares the susceptibility of the transgenic plant to said pathogen with the susceptibility of a control plant to said pathogen, the latter being set to 100%. The relative susceptibility index of transgenic plants of the present invention can be less than 95%, less than 80, less than 50%, less than 40%, preferably less than 35%, less than 20% or less than 10%.

Tolerance of a plant to fungal or oomycete infestations can be tested by experimental inoculation with a phytopathogenic dose of the fungus or oomycetes (e.g. by contacting the plant or part thereof with a suspension of fungal conidia). The fungal or oomycete infestation can be evaluated, for example by comparing the degree of fungal or oomycete infestation in the transgenic plant with the degree of fungal or oomycete infestation in a corresponding control plant. Quantifiable parameters include, but are not limited to, the relative number of infested plants, the total number of infested plants within a given plot size; the average number of diseases symptoms; the relative size of leaf area showing fungus-induced discolorations, necrosis or fungal growth; the average kernel number and/or weight per plant; or the yield per cultivated area. For example, after a given time (such as 3 or 7 days after) inoculation with the phytopathogen, the infested leave area of a transgenic plant of the invention can be at least 50%, at least 60%, at least 70%, preferably at least 75%, at least 80 or at least 90% smaller than the infested leave area of a corresponding control plant not generating antisense-ssRNA or dsRNA molecules of the disclosure.

The methods of controlling a phytopathogen described herein using a composition and/or a transgenic plant of the invention are useful for increasing the yield and/or the quality of plants. Particular advantages of these methods of the invention include:
- The antisense-ssRNA or the dsRNA molecules of the disclosure are highly selective for the phytopathogenic fungus to be controlled. Thus, benevolent organisms such as mycorrhiza or bees are not affected.
- Humans and animals consuming food produced from a transgenic plant of the invention, or from plant treated with dsRNA molecules of the disclosure are not affected by the antisense-ssRNA or the dsRNA molecules of the disclosure which are highly selective and, being biodegradable RNA, do not persist and accumulate in the environment.
- Antisense-ssRNA or dsRNA molecules of the disclosure can be expressed in or transported to portions of the plant which are difficult to reach with chemical treatment, thus providing a comprehensive protection of the plant.
- The anti-fungal activity of the transgenic plants of the present invention does not rely on the expression of new proteins within the plant which might pose an allergenic risk.
- The transgenic plants of the present invention can have a high proteomic homology or even total proteomic identity with the corresponding parent, e.g. wildtype, plants.

### EXAM PLES

**Table 2: List of primers useful for PCR and qPCR studies**

| **No.** | **Primer name** | **Primer sequence** | **Application** | **SEQ ID NO** |
|---|---|---|---|---|
| 1 | *CYP51A_F* | CGGTCCATTGACAATCCCCGT | Cloning CYP51A | 32 |
| | *CYP51A_R* | GCAGCAAACTCGGCAGTGAG | Cloning CYP51A | 33 |
| 2 | *CYP51B(AatII)_F* | | Cloning CYP51B, dsRNA synthesis | 34 |
| | *CYP51B(Ncol)_R* | | Cloning CYP51B | 35 |
| 3 | *CYP51C(Bcul)_F* | | Cloning CYP51C | 36 |
| | *CYP51C(Sacl)_R* | | Cloning CYP51C, dsRNA synthesis | 37 |
| 4 | *CYP51B (HindIII)_F* | | Plant transformation | 38 |
| | *CYP51C (XmaI)_R* | | Plant transformation | 39 |
| 5 | T7_F | | dsRNA synthesis | 40 |
| | T7_R | | dsRNA synthesis | 41 |
| 6 | *CYP51A4_F* | CCTTTGGTGCCGGTAGACAT | Real-time RT-PCR | 42 |
| | *CYP51A4_R* | CCCATCGAATAAACGCAGGC | Real-time RT-PCR | 43 |
| 7 | *QCYP51B_F* | TCTACACCGTTCTCACTACTCC | Real-time RT-PCR | 44 |
| | *QCYP51B_R* | GCTTCTCTTGAAGTAATCGC | Real-time RT-PCR | 45 |
| 8 | *CYP51C2_F* | CGAGTCCCTGGCACTGAATG | Real-time RT-PCR | 46 |
| | *CYP51C2_R* | GCTCATCACCCCAAAACCGT | Real-time RT-PCR | 47 |
| 9 | *qpcrBtubulin_F* | ATCTCGAGCCCGGTACCATGG | Real-time RT-PCR | 48 |
| | *qpcrBtubulin_R* | CTCGGTGTAATGACCCTTGGCC | Real-time RT-PCR | 49 |

### A) Methods used in the examples

### i) Construction of CYP51A, CYP51B and CYP51C templates and synthesis of dsRNA

Gene annotations for Fg sterol 14 alpha-demethylases *(CYP51)* were obtained from database of the Broad Institute (http://www.broadinstitute.org). Primers were designed to generate PCR amplicons of 200-300 bp length corresponding to exons of selected genes: 294 bp of CYP51A (SEQ ID NO:4), 220 bp of *CYP51B* (SEQ ID NO:5), and 238 bp of *GYP51C* (SEQ ID NO:6). Genomic template DNA was extracted from fusing DNeasy Plant Mini Kit (Qiagen). All three amplicons were stacked into pGEM-T Easy cloning vector (Promega) (Fig. 5). The stacked clone (*CYP51B-CYP51A-CYP51C)* was used as template for the synthesis of dsRNA *(CYP3RNA;* SEQ ID NO:7) using primers *cyp51B (Aatll)_F and cyp51C (Sacl)_R*(Table 2). Synthesis of dsRNA for *in vitro* studies was performed using BLOCK-iT RNAi TOPO Transcription Kit (Invitrogen) as well as MEGAscript^{®} Kit *High Yield Transcription* Kit (Ambion). Following MEGAscript^{®} protocols, primer pairs T7_F and T7_R with T7 promoter sequence at 5' end of both forward and reverse primers were designed for amplification of dsRNA (Table 2).

### ii) Preparation of fungal macroconidia and in vitro silencing RNA treatment

Both *Fg* wildtype (wt) strain and GFP-expressing *Fg* strain WT-GFP (Jansen et al., Proc Natl Acad Sci USA. 46:16892-16897. 2005) were routinely cultured on synthetic nutrient SNA-medium (Nirenberg, Can. J. Bot. 59:1599-1609. 1996). Plates were incubated at room temperature under constant illumination from one near-UV tube (Phillips TLD 36 W/08) and one white light tube (Phillips TLD 36 W/830HF). Conidia were harvested from 1-week-old cultures with a sterile glass rod and sterile water. Macroconidia were filtered through 4 layers of sterile mira-cloth, collected and washed three times with sterile distilled water, and finally diluted to 100 macroconidia in a volume of 100 µl liquid SNA medium, and plated in a 96-well micro-titer plate. 1.5 µg of dsRNA (600 ng/µl) suspended in 2.5 µl of 50x annealing buffer was added to fungal samples and equal volume of 50x annealing buffer was added to the control sample. Plates were incubated at room temperature. After incubation for 72 h, microscopic studies were performed with an inverted microscope (Leica DM IL). Each experiment was done in triplicate. In parallel, experiments were conducted with tebuconazole (Sigma Aldrich). Five mg of tebuconazole was suspended in 1 ml absolute ethanol to obtain a stock solution of 5mg/ml. Control sample included addition of equal volume of solvent (sterile water and alcohol) without tebuconazole.

### iii) Generation of transgenic Arabidopsis plants

The dsRNA construct *CYP3RNA* was amplified by gene specific primers CYP51B-HindIII_F and CYP51C-Xmal_R (Table 2), and cloned into p7U10-RNAi binary vector (DNA cloning service) containing selectable marker gene bar under control of the Arabidopsis Ubiquitin-10 promoter (UBQ10) and inverted constitutive CaMV35s promoter (Fig. 5b). The resulting plasmid construct p7U10*-CYP3RNA* (Fig. 7) was introduced into the *Agrobacterium tumefaciens* strain AGL1 (Lazo et al., Biotechnology (NY). 9:963-7. 1991) by electroporation. Plant transformation and regeneration was performed as described (Bechtold et al., C. R. Acad. Sci. Paris, Life Sciences 316:1194-1199. 1993), and transformants were selected by Basta (7mg/L, Duchefa Biochemies).

### iv) Molecular screening of Arabidopsis transformants

Genomic DNA from young leaves of 5-week-old transgenic (T1) and non-transgenic A. *thaliana* lines was extracted using the CTAB method (Chen et al., Plant Mol Biol Rep 17:53-57. 1999). Integration of *CYP3RNA* into the Arabidopsis genome was confirmed by PCR using gene specific primers CYP51B-HindIII_F and CYP51C-Xmal_R (Table 2).

For detecting siRNA, total RNA was extracted from the Arabidopsis lines using TRIzol reagent (Invitrogen) following the manufacturer's protocol. A total of 15 µg total RNA was separated in a 16% polyacrylamide-7 M urea gel, and transferred onto Hybond Nylon membrane (Amersham, UK) using a semidry blotter (BioRad) at constant current (250 mA) for 3h. The RNA was UV-cross-linked to the membrane at 1200 x 100 µjoules (UV Stratalinker) energy. The membrane was pre-hybridized for 45 min at 42°C in 20 ml PerfectHyb-Puffer (Sigma). The complete 791 bp *CYP3RNA* PCR amplicon was used as a template for random labeling (Random Primer DNA Labeling with Kit, NEB), added to the hybridization buffer, and hybridized overnight at 42°C. The membrane was washed twice at room temperature with 5x SSC + 0.01% SDS. The membrane was exposed on phosphorimaging screens (Bio-Rad) and signals were detected using a molecular imager and the Quantity One software (BioRad). A parallel loading of low range RNA marker (Fermentas) was used as size marker.

### v) Arabidopsis plant infection assay

For the leaf infection assay, Fg conidia concentration in the inoculum was adjusted to 5 ×10⁴ conidia per ml. For each transgenic line (T2) and control plants (empty vector and Col-0 wild type) twenty rosette leaves of 15 different 5-week-old plants were detached and transferred to square petri plates containing 2% agar/water. Inoculation was carried out as described by Koch et al., J Phytopathology 160(10):606-610. 2012. For assessing the progression of disease symptoms, the lesion size (in cm) was measured by 3 dpi from the digital images using the free software ImageJ program (http://rsb.info.nih.gov/ij/index.html). The percentage of leaf area showing infestation symptoms relative to the non-inoculated leaf was calculated. Infested leaves were collected at 3 dpi, transferred into 2 ml tubes containing 1 ml of trypan blue solution (10 g of phenol, 10 ml of glycerol, 10 ml of lactic acid, 10 mg trypan blue, dissolved in 10 ml of distilled water), followed by boiling for 3 min. The leaves were then destained in 1 ml of chloral hydrate solution (2.5 g of chloral hydrate dissolved in 1 ml distilled water) overnight, and analyzed under a light microscope (Zeiss Axioplan 2 Imaging). GFP-tagged Fgwas analyzed using a confocal microscope (Leica TCS SP2).

### vi) Fungal transcript analysis

To assess silencing of the *CYP51* genes in *Fg,* mRNA expression analysis was performed using quantitative real-time RT-PCR (qRT-PCR). For the *in vitro* assay 1.2× 10⁵ Fg conidia were grown in 2 ml liquid SNA medium. 162 µg of dsRNA suspended in 200 µl of nuclease free water (810 ng/µl) was added. Samples were grown at room temperature while shaking (72 h). For the *in vivo* assay, detached Arabidopsis leaves were inoculated with *Fg* macroconidia and collected 3 days after inoculation (dai). RNA extraction from the fungal *in vitro* sample as well as diseased leaves was performed with TRIzol^{®} (Invitrogen, Germany) following manufacturer's instructions. Freshly extracted mRNA was used for cDNA synthesis using QuantiTect^{®} Reverse Transcription kit (QIAGEN, Germany). cDNA was stored at -20°C. For qRT-PCR, 50 ng of cDNA was used as template in the Applied Biosystems 7500 FAST real-time PCR system. Amplifications were performed in 7.5 µl of SYBER green JumpStart Taq ReadyMix (Sigma-Aldrich, Germany) with 0.5 pmol oligonucleotides. Each sample had three repetitions. Primers were used for studying expression of target *CYP51* genes with reference to beta-tubulin gene (Table 2) After an initial activation step at 95°C for 5 min, 40 cycles (95°C for 30 sec, 53°C for 30 sec, 72°C for 30 sec) were performed. Ct values were determined with the 7500 Fast software supplied with the instrument. Transcript levels of *CYP51* genes were determined via the 2^{-ΔΔCt} method (Livak et al., Methods 25:402-408. 2001) by normalizing the amount of target transcript to the amount of beta-tubulin.

### vii) Spraying of dsRNA and leaf infection assay

Leaves of 2-3 week-old barley wild type plants cultivar Golden Promise were detached and transferred into square Petri plates containing 1% agar. The Leaves were then sprayed with dsRNA. The dsRNA was generated using MEGAscript^{®} RNAi Kit (Invitrogen). Following MEGAscript^{®} protocols, primer pairs T7_F and T7_R with T7 promoter sequence at 5' end of both forward and reverse primers were designed for amplification of dsRNA (cf. Table 2). For the application by spraying, the dsRNA was diluted in 500 µl 0.02% (v/v) Tween 20 to a final concentration of 20 ng/µl. Spraying of the leaves was carried out using a spray flask (10 ml capacity). Each plate containing six detached leaves was evenly sprayed with *CYP3RNA* dsRNA, TE-buffer only or GFP-dsRNA, respectively, by giving 3-4 puffs. Thereafter, the plates were left standing open until the surface of each leaf was dried (which took approximately 2h) and were then covered with lids. 48h after spraying, the leaves were treated with suspension of *Fg* conidia in 0.02% (v/v) Tween 20 at a concentration of 2×10⁴ conidia per ml by applying a 20 µl-droplet of suspension to each of the upper, the middle and the lower part of the leaf. Again, the plates were covered with lids and incubated for 6 days at room temperature. To evaluate infection severity, fungal growth was evaluated by measuring the relative amount of fungal gene expression using qPCR. For this purpose, the droplets on each leaf were removed using a filter paper, the leaves were collected in 15 ml tubes and stored at -80°C until DNA was extracted.

viii) DNA extraction and PCR analysis for fungal quantification To assess inhibition of fungal growth the relative amount of fungal genomic DNA was measured using quantitative PCR (qPCR). DNA was extracted using the CTAB method (Chen et al., Plant Mol Biol Rep 17:53-57. 1999). For qPCR, 50 ng of DNA was used as template in the Applied Biosystems 7500 FAST realtime PCR system. Amplifications were performed in 7.5 µL of SYBER green JumpStart Taq ReadyMix (Sigma-Aldrich) with 0.5 pmol oligonucleotides. Each sample had three repetitions. Primers were used for determining the amount of fungal genomic beta-tubulin DNA with reference to barley genomic ubiquitin DNA. After an initial activation step at 95°C for 5 min, 40 cycles (95°C for 30 sec, 57°C for 30 sec, 72°C for 30 sec) were performed. Ct values were determined with the 7500 Fast software supplied with the instrument. Transcript levels of beta-tubulin gene were determined via the 2^{-Δ Δ Ct} method (Livak et al., Methods 25:402-408. 2001) by normalizing the amount of fungal beta-tubulin DNA to the amount of plant ubiquitin DNA.

### B) Examples (for illustrating the claimed invention)

### Example 1: Inhibition of fungal growth and silencing of Fg CYP51 genes by direct application of dsRNA

A 791 bp dsRNA *(CYP3RNA)* complementary to all three fungal *CYP51* genes (Fig. 5) was generated. Microscopic analysis of *Fg* macroconidia treated with *CYP3RNA* revealed swelling of hyphal tips by 48 h post treatment (hpt, data not shown). By 72 hpt, the morphological changes had become more pronounced, particularly at hyphal tips (Fig. 1a,ii). In comparison, no such swelling was detected in the mock-treated control (Fig. 1a,i). Since CYP51 proteins are the target of azole fungicides, also the phenotype of *Fg* macroconidia treated with tebuconazole was monitored. *Fg* macroconidia treated with tebuconazole (5 mg/l) exhibited a phenotype comparable to that elicited by *CYP3RNA* treatment (Fig. 1a,iii).

The similar phenotype of *CYP3RNA-* and tebuconazole-treated *Fg* indicated that the dsRNA silenced the expression of one or more *CYP51* genes. This was confirmed by quantifying the transcript levels for *CYP51A, CYP51B,* and *CYP51C* in control and dsRNA-treated *Fg* macroconidia at 72 hpt using qPCR with beta-tubulin as the reference gene. Transcripts for all three genes were significantly reduced in *Fg* macro-conida treated with *CYP3RNA* as compared to the mock controls (Fig. 1b).

Fungal growth (determined based on optical densities of *Fg* cultures at 600 nm at 0 hpt and 72hpt) was strongly inhibited by *CYP3RNA* in a dose dependent manner (Fig. 2).

The ability of *CYP3RNA* to reduce the growth of *Fg* and to silence the *CYP51* genes in *Fg* macroconidia indicates that the fungus can take up a 791 bp dsRNA without further delivery support, such as polyethylene glycol, calcium chloride, lipofectamine or electroporation.

### Example 2: Expression of CYP3RNA in planta confers resistance to Fg

*Arabidopsis thaliana* Col-0 plants were transformed with either p7U10-*CYP3RNA,* which contains two inverted 35S promoters that drive the constitutive production of sense and antisense copies of *CYP3RNA,* or an empty vector (ev) control (Figs. 7 and 8). Resistance to *Fg* was assessed by inoculating detached leaves of 5-week-old Arabidopsis T2 plants (expressing *CYP3RNA*) and the respective Arabidopsis Col-0 ev control plants with 5 × 10⁴ *Fg* macroconidia per ml (cf. method v) above).

At 3 days post inoculation (dpi), both wild-type (wt) Col-0 and the ev control line showed water-soaked lesions with chlorotic or necrotic lesions; these are typical symptoms of a *Fg* infestation (Fig. 3a,i-ii). In marked contrast, four independent Arabidopsis T2 lines containing the *CYP3RNA* silencing construct (L1, L8, L10, L11) showed no disease symptoms, and their leaves were indistinguishable from those of the mock-inoculated controls (Fig. 3a,iii,iv). The percentage of infested leaf area on the four transgenic lines was 0.9%, whereas that of the ev controls was 77% (Fig. 3b).The CYP3RNA-expressing lines remained free of disease symptoms at 5 dpi, unlike the Col-0 ev plants which exhibited substantial symptoms at this time (Fig. 3c). The fungal hyphae are due to germination and growth of fungi in the inoculated agar, not on the leaf (Fig 3b,ii). Furthermore fungal growth was slower and the colony pigment was yellow or brownish on plates with *CYP3RNA-*expressing lines rather than the typical pink on control plates (data not shown).

Fungal growth was characterized by microscopic analysis of detached leaves stained with trypan blue to visualize fungal hyphae as described above. On leaves from the Col-0 ev control, the fungal macroconidia germinated and developed mycelia within 3 dpi (data not shown). Profuse hyphal growth was seen outside and inside the leaf, and was not restricted to the inoculation site. Successful fungal development was further indicated by the formation of loose sporodochia formed by branched conidio-phores. In marked contrast, fungal mycelium formation on CYP3RNA-expressing leaves was strongly (to nearly 100%) reduced and was exclusively confined to the wounded area surrounding the infection sites; no fungal colonization was detected in the surrounding leaf tissue. The fungus growing at the wound sites rapidly formed a vast number of sporodochia, which is indicative of the fungus being impaired by stress.

The results from the detached leaf assay were confirmed by confocal microscopy of intact leaves from Col-0 ev control and CYP3RNA-expressing plants inoculated with macroconidia of a green fluorescent protein (GFP)-expressing Fg strain. As evidenced by GFP fluorescence, *Fg* effectively colonized Col-0 ev control leaves, developing mycelia within 3 dpi. In comparison, fungal growth on *CYP3RNA*-expressing leaves was restricted to the inoculation site, where stress-induced sporulation was observed.

Strikingly, the level of resistance achieved by targeting all three paralogous *Fg* CYP51 genes for inducing HIGS was substantially greater than that observed in previously published HIGS studies.

### Example 3: Fg resistance in the CYP3RNA-expressing lines is due to HIGS of the CYP51 genes

To assess whether HIGS of the *CYP51* genes was responsible for Fg resistance in the *CYP3RNA*-expressing lines, quantitative RT-PCR analysis of total RNA from the *Fg* inoculated leaves of CYP3RNA-expressing and ev control plants was performed 3 dpi (cf. method (vi) above). The relative transcript levels of *Fg CYP51A, Fg CYP51B* and *Fg CYP51C* were reduced on average by 92%, 89%, and 77%, respectively, in samples from CYP3RNA-expressing lines (L1, L8, L10, and L11), as compared with the ev control line (Figs. 5-7).

It was further assessed whether this reduction in *Fg CYP51* transcripts was associated with the production of corresponding siRNAs. In Northern analyses using *CYP3RNA* as the probe, siRNAs corresponding to the targeted *CYP51* sequences in both *Fg*-infested and uninfested leaves from CYP3RNA-expressing plants were detected (Fig. 8). By contrast, these siRNAs were not observed in the leaves of wt or ev control plants. Taken together, these results suggest that expression of *CYP3RNA in planta* effectively silences the three *Fg CYP51* genes, possibly by directing the host plant's silencing machinery to produce the corresponding siRNAs.

### Example 4: Fg resistance in CYP3RNA-expressing barley leaves

Barley was transformed with *p*7*i-*Ubi*-CYP3RNAi* (Fig*.* 9). *p*7*i-*Ubi*-CYP3RNA* was generated analogous to *p7U10-CYP3RNAi* by replacing the GUS-fragment in *p7i-Ubi-RNAi2 (**Fig.* 8) by a *CYP51ABC* fragment (CYP51A-CYP51B-CYP51C) using Hindlll/Xmal restriction enzymes. The binary vector p7i-Ubi-*CYP3RNAi* contains two inverted 35S promoters that drive the constitutive production of sense and antisense copies of *CYP51ABC.* For preparing control plants, barley was transformed with the empty vector (ev) (Fig. 8). Transgenic barley plants expressing *CYP51ABC* were identified by RT-PCR using gene specific primers. For each transgenic line (L2 to L9, L15 and L42) and control plants (empty vector and wildtype cv. Golden Promise) two leaves of two-week-old barley plants were detached and transferred to square petri plates containing 2% agar/water. Each leaf was inoculated at three different spots (top, middle, bottom) with 20µl drops of a suspension comprising 5× 10⁴ Fg macroconidia per ml. After 7 days, images of the leaves were taken (cf. Fig. 10) and the relative levels of CYP51A, CYP51B and CYP51C transcripts was determined analogous to method (vi) above (cf. Fig. 11). The leaves from transgenic lines (L2-L9, L14, L42) differed significantly from the leaves of the controls (ev, GP) with regard to symptoms of fungal infestation. Four of the 10 transgenic lines (L2, L7, L9, L42) exhibited no symptoms of fungal infestation (100% resistance). The remaining six lines (L3-L6, L8 and L14) showed only weak, locally restricted symptoms. In contrast, the control lines showed significant discoloration and bleaching of the Fg inoculated leaves (cf. Fig. 10).

### Example 5: Reduction of Fg growth after application of dsRNA on leaves

A 791 bp dsRNA *(CYP3RNA)* complementary to all three fungal CYP51 genes (Fig. 5) was generated and applied to detached barley leaves mounted on agar plates. As negative controls, only buffer or an unrelated dsRNA molecule (GFP-dsRNA, which has no significant homology to fungal genes) were applied. 48 h after spraying the barley leaves with *CYP3RNA* dsRNA or the negative controls, the leaves were infected with Fg macroconidia. 6 days after inoculation (i.e. 8 days after spraying), a significant inhibition of fungal growth was observed with CYP3RNA-treated leaves ("dsRNA") compared to the control leaves (which were sprayed with either buffer only ("control") or GFP-dsRNA ("GFP"), Fig. 12A). These findings were consistent with the results of qPCR, where the relative amount of fungal DNA in infected CYP3RNA-treated leaves was found to be significantly lower than in infected control leaves (Fig. 12C). Neither the TE-buffer nor the unrelated GFP-dsRNA had an inhibitory effect on fungal growth.

### Example 6: Reduction of Fg growth after application of application of RNA on distal leave areas

The upper part of the leaves were sprayed with dsRNA *(CYP3RNA)* or a control sample (buffer only ("control") or GFP-dsRNA ("GFP")), while the middle and lower areas were covered. After 2 days, the lower part of the leaves was subjected to fungal inoculation. The infection severity was determined 6 days after inoculation by measuring relative amount of fungal DNA. Fungal growth inhibition compared to the control-treated leaves was observed not only at the *CYP3RNA*-treated upper leaf area but also at the lower leaf area of the same leaf (Figs. 12B and 12D). These findings indicate that *CYP3RNA* dsRNA is taken up by and transported within the leaf so as to provide a systemic protection from fungal infestation.

## Claims

1. A transgenic plant, preferably a cereal crop, comprising a DNA sequence providing a promoter operably linked to a transcriptional template of at least the antisense sequence(s) of the dsRNA molecule, said dsRNA molecule being capable of inhibiting the expression of at least one CYP51 gene from the phytopathogen Phakopsora pachyrhizi, said CYP 51 gene having a sequence according to SEQ ID NO. 18, and the dsRNA comprising
- a sense sequence being at least 95% identical to at least 20 contiguous nucleotides of the coding sequence of said CYP51 gene, and
- an antisense sequence being complementary to said sense sequence,
wherein the arrangement of the sequences within the dsRNA molecule allows hybridization of said sense sequence and said corresponding antisense sequence; and wherein
the plant is capable of generating at least the antisense sequence(s) of the dsRNA molecule.

2. The transgenic plant of claim 1, wherein the plant is capable of generating said dsRNA molecules.

3. A method for controlling a *Phakopsora pachyrhizi* phytopathogen, wherein a transgenic plant according to any one of claims 1 and 2 is cultivated to allow the generation of RNA comprising the antisense sequence(s) of the dsRNA molecule, and said RNA inhibits the growth and/or propagation of said phytopathogen.

4. A method for controlling a *Phakopsora pachyrhizi* phytopathogen, wherein a plant infested by or at risk of being infested by said phytopathogen and/or the vicinity of said plant is contacted with an effective amount of the composition for controlling a fungal or oomycete phytopathogen comprising the dsRNA molecule defined in claim 1, and a plant-compatible carrier.

## Patentansprüche

1. Transgene Pflanze, vorzugsweise eine Getreidepflanze, die eine DNA-Sequenz umfasst, welche einen Promotor bereitstellt, der mit einer Transkriptionsmatrize zumindest der Antisense-Sequenz(en) des dsRNA-Moleküls funktionell verbunden ist, wobei das dsRNA-Molekül dazu fähig ist, die Expression mindestens eines CYP51-Gens aus dem Phytopathogen *Phakopsora pachyrhizi* zu hemmen, wobei das CYP51-Gen eine Sequenz gemäß SEQ ID Nr.: 18 aufweist und die dsRNA Folgendes umfasst:
- eine Sense-Sequenz mit einer Identität von mindestens 95 % zu mindestens 20 zusammenhängenden Nucleotiden der codierenden Sequenz des CYP51-Gens und
- eine Antisense-Sequenz, die komplementär zur Sense-Sequenz ist,
wobei die Anordnung der Sequenzen innerhalb des dsRNA-Moleküls eine Hybridisierung der Sense-Sequenz und der entsprechenden Antisense-Sequenz ermöglicht und wobei
die Pflanze dazu fähig ist, zumindest die Antisense-Sequenz(en) des dsRNA-Moleküls zu erzeugen.

2. Transgene Pflanze nach Anspruch 1, wobei die Pflanze dazu fähig ist, die dsRNA-Moleküle zu erzeugen.

3. Verfahren zur Bekämpfung eines *Phakopsora*pachyrhizi-Phytopathogens, wobei eine transgene Pflanze nach einem der Ansprüche 1 und 2 so kultiviert wird, dass die Erzeugung einer RNA ermöglicht wird, welche die Antisense-Sequenz(en) des dsRNA-Moleküls umfasst, und die RNA das Wachstum und/oder die Vermehrung des Phytopathogens hemmt.

4. Verfahren zur Bekämpfung eines *Phakopsora*pachyrhizi-Phytopathogens, wobei eine Pflanze, die vom Phytopathogen befallen ist oder für die ein Risiko für einen Befall mit dem Phytopathogen besteht und/oder die Umgebung der Pflanze mit einer wirksamen Menge der Zusammensetzung zur Bekämpfung eines Pilz- oder Eipilz-Phytopathogens in Kontakt gebracht wird, die das in Anspruch 1 definierte dsRNA-Molekül und einen pflanzenverträglichen Träger umfasst.

## Revendications

1. Végétal transgénique, préférablement culture céréalière, comprenant une séquence d'ADN fournissant un promoteur lié de manière fonctionnelle à une matrice transcriptionnelle d'au moins la ou les séquences antisens de la molécule d'ARNds, ladite molécule d'ARNds étant capable d'inhiber l'expression d'au moins un gène CYP51 du phytopathogène Phakopsora pachyrhizi, ledit gène CYP 51 possédant une séquence selon la SEQ ID NO: 18, et l'ARNds comprenant
- une séquence sens qui est au moins 95 % identique à au moins 20 nucléotides contigus de la séquence codante dudit gène CYP51, et
- une séquence antisens qui est complémentaire à ladite séquence sens,
l'agencement des séquences dans la molécule d'ARNds permettant l'hybridation de ladite séquence sens et de ladite séquence antisens correspondante ; et
le végétal étant capable de générer au moins la ou les séquences antisens de la molécule d'ARNds.

2. Végétal transgénique selon la revendication 1, le végétal étant capable de générer lesdites molécules d'ARNds.

3. Procédé pour la lutte contre un phytopathogène de type *Phakopsora pachyrhizi,* un végétal transgénique selon l'une quelconque des revendications 1 et 2 étant cultivé pour permettre la génération d'un ARN comprenant la ou les séquences antisens de la molécule d'ARNds, et ledit ARN inhibant la croissance et/ou la propagation dudit phytopathogène.

4. Procédé pour la lutte contre un phytopathogène de type *Phakopsora pachyrhizi,* un végétal infesté par ou à risque d'être infesté par ledit phytopathogène et/ou la proximité dudit végétal étant mis(e) en contact avec une quantité efficace de la composition pour la lutte contre un phytopathogène fongique ou oomycète comprenant la molécule d'ARNds définie dans la revendication 1, et un support compatible avec le végétal.


### SEQUENCE LISTING

<110> BASF SE
<120> RNAi for the control of phytopathogenic fungi and oomycetes by inhibiting the expression of CYP51 genes
<130> 75207/JAB
<160> 49
<170> PatentIn version 3.5
<210> 1
   <211> 1524
   <212> DNA
   <213> Fusarium graminearum
<400> 1
   atgttccatc tactcatcta tcccttatgg gtcttggtag cactctttgc tgtcataatt        60
   gccaacctat tgtatcagca gcttcctcga aggccggacg agcctccact agttttccac       120
   tggttccctt tctttggaaa cgcagtcgct tacggcctgg acccttgtgg cttctttgag       180
   aagtgccgag agaagcacgg agacgtcttc acctttatct tattcggtcg aaaaatcgtc       240
   gcttgccttg gcgtcgacgg caatgatttt gttctcaaca gtcgcctcca ggatgccaat       300
   gcagaagaag tttacggtcc attgacaatc cccgtctttg gtagcgatgt cgtatacgat       360
   tgtcccaact cgaagctcat ggaacaaaag aagtttgtca agtttggcct tacgcaaaaa       420
   gcactcgagt cacacgtcca gttaatcgag cgagaggttc ttgactacgt cgaaactgat       480
   ccatcctttt ctggcagaac tagcaccatc gatgtcccca aggcaatggc tgagataaca       540
   atctttactg cctcacgttc tttgcagggt gaggaagttc ggagaaaact cactgccgag       600
   tttgctgctc tgtatcacga tcttgacctt ggatttagac ctgtcaactt tctgtttcca       660
   tggttgccac tgcctcacaa ccgaaaacga gatgctgctc atataaagat gcgggaagtc       720
   tatatggata tcatcaacga tcgaagaaaa gggggcattc gaacagaaga tggtactgat       780
   atgatagcca acttgatggg ctgcacttac aagaacggac agcctgttcc tgacaaggaa       840
   atcgcccaca tgatgattac tctactcatg gcaggccagc actcttcatc ttctgccagt       900
   tcttggatcg tgctgcacct tgcttcttct cccgacatta ccgaagaact ctaccaggag       960
   cagctcgtaa atttgagcgt taacggtgct ctacctcccc ttcagtactc tgaccttgac      1020
   aagctccctc ttctccagaa cgtcgtcaag gagactctcc gcgtccactc ttccatccac      1080
   tcaatcctgc ggaaagtcaa gagacccatg caagttccca attcccctta cacgatcact      1140
   acagacaaag tcatcatggc ttcacctaca gttaccgcga tgagtgaaga gtacttcgag      1200
   aacgccaaaa catggaaccc tcatcgatgg gacaacagag caaaggagga ggttgacact      1260
   gaagatgtca tcgactatgg atatggggct gtgtctaaag gaacgaagag cccgtacttg      1320
   ccctttggtg ccggtagaca tcgctgcatt ggagagaagt ttgcctatgt caacttgggt      1380
   gtcattgttg ctactttggt gcgcaacttc agactttcga ccattgatgg aagacctgga      1440
   gtcccagaga cagactatac ttctttgttc tcacgacccg cccagcctgc gtttattcga      1500
   tgggagcgta ggaagaagat atag                                             1524
<210> 2
   <211> 1581
   <212> DNA
   <213> Fusarium graminearum
<400> 2
   atgggtctcc ttcaagaact ggcgggccat ccgctcgccc aacaattcca ggaacttccc      60
   ctgggccagc aagttggaat tggctttgca gtcttcctcg tgctctccgt cgtcctcaat     120
   gtcctcaacc agctcctctt ccgaaacccc aatgagcccc ctatggtctt ccactggttc     180
   ccctttgttg gaagcaccat cacatacgga atggaccccc ccacgttctt cagagagaac     240
   cgtgccaagc acggcgatgt cttcaccttt atcctcctcg gaaagaaaac cactgtcgct     300
   gttggccctg ctggaaacga cttcatcctc aacggcaagc tcaaggatgt gtgtgctgag     360
   gagatctaca ccgttctcac tactcctgtc tttggcaagg atgtcgtcta tgactgcccc     420
   aacgccaagc tcatggaaca gaagaagttc atgaagattg ccctgaccac cgaagccttc     480
   cgatcctacg ttcccatcat ctcctccgag gttcgcgatt acttcaagag aagccccgac     540
   ttcaagggca agtctggcat tgccgatatc cccaagaaga tggccgagat cactatcttt     600
   actgcttccc atgctctcca gggcagcgcc atccgcagca agtttgacga gtccctggcc     660
   gctctctacc acgacctcga tatgggcttc acccccatca acttcatgct tcactgggcc     720
   cctctcccct ggaaccgtaa gcgcgaccac gcccagcgca ctgttgccaa gatctacatg     780
   gacactatca aggagcgccg cgccaagggc aacaacgaat ccgagcatga catgatgaag     840
   caccttatga actctacata caagaacggt atccgtgtcc ctgaccacga ggttgcccac     900
   atgatgattg ccctccttat ggctggccag cactcttctt cttccaccag ctcatggatc     960
   atgctccgtc tcgctcagta ccctcacatc atggaagagc tctaccagga gcaggtcaag    1020
   aacctcggtg ctgatctgcc tcctctgact tacgaagacc ttgccaagct gccccttaac    1080
   caggctatcg tcaaggagac ccttcgtctc catgctccta tccactccat catgcgcgcc    1140
   gtcaagtctc ccatgcccgt ccctggcact aagtatgtca ttcccacttc gcacactctt    1200
   ctcgctgctc ctggtgtcag tgctaccgac tctgccttct tccccaaccc tgatgagtgg    1260
   gaccctcacc gatgggaggc agactctccc aacttccccc gcatggcttc caagggcgag    1320
   gacgaggaga agatcgacta cggttacggc cttgtcagca agggttcagc ttctccctac    1380
   ctgcctttcg gcgctggccg tcaccgatgc attggtgagc actttgccaa cgcccagctt    1440
   caaaccattg ttgccgaggt tgttcgcgag ttcaagttcc gcaacgttga tggcggtcac    1500
   accctgatcg ataccgacta cgcctcgctc ttctcccgac ctctggagcc cgccaacatt    1560
   cactgggagc gacgccagta a                                              1581
<210> 3
   <211> 1554
   <212> DNA
   <213> Fusarium graminearum
<400> 3
   atggaatcgc tctacgagac tctgcggact ctaccgctct cagtctcaat ccctctaaca      60
   accagcatca tcatcatctt gtccatcgtc accaacgtgg tcaaacaatt atggtttccc     120
   aacccacatc gtccacccgt tgtattccat atcttcccct tcattggaag caccgtacaa     180
   tatggcatcg acccgtacgc ttttttcttc gactgcagag ataaatacgg cgactgcttt     240
   acctttattc tccttggcaa atcaacgact gtctttcttg gtcccaaggg caatgacttt     300
   atcctcaacg gcaaacacgc cgatctcaac gccgaggacg tttatgggaa acttaccacg     360
   cccgtgtttg gtgaggaggt tgtttatgac tgctccaatg ctcgtttcat ggaccaaaag     420
   aggcttctca aacttggtct caccacagat tccttacggt gctacatccc aaagttcgtc     480
   aaagaagtcg aagactatgt taaaaactcg ccatacttca agggtgacac aggaatcgtc     540
   aacattacag aagtcatggc cgaaatcaca atctacacag catccggatc cctcctagga     600
   aacgaagtcc gatctatgtt tgacagcaca ttcgccactc tctaccgcca tctagatgat     660
   ggcttccaac ccattaattt cgtcatgcca ggtcttcccc tcccgcaaaa cttccgtcga     720
   aaccatgctc gaaaggtaat ggagaagctt ttcagcgata ttatttccaa gcgtcgcgag     780
   actggcaatc aaggcgacga gacggatatg atttggatgc ttatgaatgc acagtataag     840
   gatggggaac ctcttccgga tcaccatgct gcgcgtatgt tgattgctat cctgatgggt     900
   ggccagcata atactgctgt tagtggtgct tggcttcttc tcaatctggc ccataagcct     960
   catcttgttc aggaactgta cgaggaacag acccaggtcc ttggctcacc acaagagcct    1020
   ctgacatggg agaacttaca gaaattaact ctcaacggcc aagtcatcaa ggaaactctc    1080
   cgtcttcaca gtccaatcca ctccatcctc cgacaagtca aatcacccat gcgagtccct    1140
   ggcactgaat gggtagtgcc accatcccac acactgcttt cgtctcccgg cacaatggcc    1200
   cgctcagaag aattcttccc tcgaccatca gaatgggatc ctcatcgttg ggacaagatt    1260
   gaacctctcg tgaagaccgc cgaagatggt caaacagtgg attacggttt tggggtgatg    1320
   agcaaatccg tcagcagtcc ttatttgccc tttggagctg gacgacatcg atgtgttggc    1380
   gagaattacg cttatgcaca gctgggagct attgttgcca cgtttatcag attggttcac    1440
   attgaacagc ctgacccgaa ggctcctctt ccggcaccag attattcttc catgttttct    1500
   cggcccatga accctgccga gatccgatgg cgtcgacgcg agacagtaga atga          1554
<210> 4
   <211> 294
   <212> DNA
   <213> Fusarium graminearum
<400> 4
   cggtccattg acaatccccg tctttggtag cgatgtcgta tacgattgtc ccaactcgaa 60
   gctcatggaa caaaagaagt ttgtcaagtt tggccttacg caaaaagcac tcgagtcaca 120
   cgtccagtta atcgagcgag aggttcttga ctacgtcgaa actgatccat ccttttctgg 180
   cagaactagc accatcgatg tccccaaggc aatggctgag ataacaatct ttactgcctc 240
   acgttctttg cagggtgagg aagttcggag aaaactcact gccgagtttg ctgc 294
<210> 5
   <211> 220
   <212> DNA
   <213> Fusarium graminearum
<400> 5
   cagcaagttt gacgagtccc tggccgctct ctaccacgac ctcgatatgg gcttcacccc 60
   catcaacttc atgcttcact gggcccctct cccctggaac cgtaagcgcg accacgccca 120
   gcgcactgtt gccaagatct acatggacac tatcaaggag cgccgcgcca agggcaacaa 180
   cgaatccgag catgacatga tgaagcacct tatgaactct 220
<210> 6
   <211> 238
   <212> DNA
   <213> Fusarium graminearum
<400> 6
   attggaagca ccgtacaata tggcatcgac ccgtacgctt ttttcttcga ctgcagagat 60
   aaatacggcg actgctttac ctttattctc cttggcaaat caacgactgt ctttcttggt 120
   cccaagggca atgactttat cctcaacggc aaacacgccg atctcaacgc cgaggacgtt 180
   tatgggaaac ttaccacgcc cgtgtttggt gaggaggttg tttatgactg ctccaatg 238
<210> 7
   <211> 785
   <212> DNA
   <213> artificial sequence
<220>
   <223> combines partial sequences of Fusarium graminearum CYP51B-CYP51A-CYP51C
<400> 7
   cagcaagttt gacgagtccc tggccgctct ctaccacgac ctcgatatgg gcttcacccc 60
   catcaacttc atgcttcact gggcccctct cccctggaac cgtaagcgcg accacgccca 120
   gcgcactgtt gccaagatct acatggacac tatcaaggag cgccgcgcca agggcaacaa      180
   cgaatccgag catgacatga tgaagcacct tatgaactct ccatggcggc cgcgggaatt      240
   cgatatcggt ccattgacaa tccccgtctt tggtagcgat gtcgtatacg attgtcccaa      300
   ctcgaagctc atggaacaaa agaagtttgt caagtttggc cttacgcaaa aagcactcga      360
   gtcacacgtc cagttaatcg agcgagaggt tcttgactac gtcgaaactg atccatcctt      420
   ttctggcaga actagcacca tcgatgtccc caaggcaatg gctgagataa caatctttac      480
   tgcctcacgt tctttgcagg gtgaggaagt tcggagaaaa ctcactgccg agtttgctgc      540
   cactagtatt ggaagcaccg tacaatatgg catcgacccg tacgcttttt tcttcgactg      600
   cagagataaa tacggcgact gctttacctt tattctcctt ggcaaatcaa cgactgtctt      660
   tcttggtccc aagggcaatg actttatcct caacggcaaa cacgccgatc tcaacgccga      720
   ggacgtttat gggaaactta ccacgcccgt gtttggtgag gaggttgttt atgactgctc      780
   caatg                                                                  785
<210> 8
   <211> 1521
   <212> **DNA**
   <213> Fusarium oxysporum
<400> 8
   atgttctcac tcctatacta ccctctatgg gcctttgctt cctgcctagt tatcatcact 60
   ctcaacgtct tataccagaa gctccctcga aatgccaacg aacctccgtt agtgttccac 120
   tggcttccat tcgttgggaa tgctgttgct tatggactcg acccttatgg tttctttgtg 180
   aagtgtcgag aaaagcacgg cgatgtcttc acctttatcc tcttcggtcg aaaaatcgtt 240
   gcctgtcttg gtgttgacgg caatgacttt gttctcaaca gtcgaattca ggacgccaac 300
   gccgaagaaa tctacagtcc attgacaacg cctgtctttg gtagtgatgt cgtatacgat 360
   tgtcccaact cgaagctcat ggagcaaaag aagtttgtca agtttggcct tacacaaaag 420
   gctctcgagt cccatgtcca gttgatcgag cgagaggttc tggagtacat ccaagctgta 480
   ccttcattct ctggaaagtc tggcacagtt gatgtatcca aggcaatggc tgagataacc 540
   atcttcactg ctgctcgctc tctgcagggc gaagaagttc gacggaagct tacagctgag 600
   tttgcagctc tgtatcatga ccttgaccta ggcttcactc ctgtaaactt cctgttcccc 660
   tggctacctt tgcctcataa ccgacgtcga gatgctgctc atgcaaagat gagagagatc 720
   tacatggaca tcattaacgg acgaagaaga ggcgtagggg acttggagaa aggaactgac 780
   atgatcgcca acctgatgaa ttgcgagtac aaaaacgggc agccgattcc ggacaaagag 840
   atcgcgcaca tgatgatcac ccttctcatg gctggacaac actcttcgtc atctgctagt 900
   tcatggatca tactacatct ggcttcatcc actgacattg ctgaggaact ctaccaagag 960
   caactcatta acttgagtgc tgatggtgtt ctccctcccc ttcagtacac cgacctcgac 1020
   aagcttcccc ttcttcagaa tgtcgtcaaa gaaacactcc gtgttcattc ttccattcac 1080
   tccattctgc gaaaggttaa gagacctatg caagcacctg gatcacctta caccatcacc 1140
   acagacaagg ttctcctcgc ttcaccaact gttacagcgt tgagtgaaga acacttcacg 1200
   gacgcccaga gatggaatcc tcatcggtgg gataacaaac cccaggagga ggccgtgacg 1260
   gacgatgtca ttgactacgg ctacggcgct gtttctaaag gaacgaagag cccatactta 1320
   ccctttggcg ctggtcggca tcgctgcatc ggggagaagt ttgcttatgt caacttgggc 1380
   gttatcgtcg cgactttggt gcgcaacttc agactgtcga ctcttgatgg caagcctggt 1440
   attccagcaa ctgactacac ttctctcttc tcaaggccag cccaacctgc atacataaac 1500
   tgggagcgca ggagggctta a 1521
<210> 9
   <211> 1584
   <212> DNA
   <213> Fusarium oxysporum
<400> 9
   atgggtctcc tccaagaact tgcgagccac ccgctcgcac aacaatatca ggagctcccc 60
   ctgggccagc agattggaat tggcttcgga gcattcataa ttctctctgt cgttctgaat 120
   gtgctcaacc agctactctt caagaaccgc aatgaacctc ccctagtctt ccactggttc 180
   cctttcgttg gaagcactat cacatacgga atggaccccc ctaagttctt caaggagaac 240
   cgcgccaagc atggtgatgt tttcaccttt gtcctccttg gaaagaaaac cactgtcgct 300
   gttggcccca ctggaaacga cttcatcctc aacggaaagc tcaaggatgt ctctgccgag 360
   gagatttaca ctgttctcac tactcctgtc tttggcaagg atgtcgtgta cgattgccct 420
   aatgccaaac tcatggagca gaagaagttc atgaaaatcg ccctcacgac cgaagccttc 480
   cgatcatacg tgcctattat ctccgccgag gttcgcgatt acttcaagaa gagccctgat 540
   ttcaagggca agtccggtat tgtcgatatt cccaagaaaa tggccgagat cactatcttc 600
   actgcttcgc atgcccttca gggcagcgtc attcgtaaca agtttgacga gtctctggcc 660
   gctctctacc acgatctcga catgggtttc actcccatca acttcatgct tcactgggct 720
   cctctcccct ggaaccgcaa gcgtgaccac gcccagcgca ctgttgccaa gatctatatg 780
   gacaccatta aggagcgacg cgctaaggac aacgatgaca ccgagcacga tatgatgaag 840
   catctcatga actctactta caagaacggc acccctgtcc ctgatcatga ggtcgcccac 900
   atgatgattg ctctcctcat ggctggccag cactcttctt cttctaccag ctcttggatc 960
   atgctccgtc tcgctcagta ccctcacatc atggaggagc tgtaccaaga gcaggtcaga 1020
   gaactcggtg ctgatttgcc tcccctgact tacgacaacc ttgccaagct gcccctcaac 1080
   caggccatta tcaaggagac tctccgcctt cacgccccta tccactctat catgcgcgcc 1140
   gtcaagtctc ccatgcctgt ccctggaacc aagtacacca tcccgacctc gcacactctt 1200
   ctcgccgccc ccggtgtcag cgctactgac tcggcctact tccccaaccc cgatgagtgg 1260
   gatcctcacc gatgggaggt cgactctccc aacttcccca gaatggctac ccgtggcgat 1320
   gacgaagaga agatcgacta tggctacggc cttgtcagca agggttccgc ctctccttat 1380
   ctgccctttg gtgctggtcg tcaccgatgc attggcgagc actttgccaa cgcccagctt 1440
   cagacaattg ttgctgaggt tgtgcgtgag ttcaagttcc gcaatgtgga tggcggcaac 1500
   actctaatcg acaccgacta cgcctcgctt ttctcgcgac ccttggagcc cgccaacatt 1560
   cactgggaga gacgccagca gtag 1584
<210> 10
   <211> 1149
   <212> **DNA**
   <213> Fusarium oxysporum
<400> 10
   atggatcaga agaggcttct caaacttggt ctcacaactg actcattgcg atgctacatc      60
   cccaaattcg tcaaagaagt cgaagaatac atcgctactt caccctactt caaaggatca     120
   actggcatcg tcaacatcac cgaagtaatg gccgagatca caatctacac cgcagcaggc     180
   tccctcctcg gtaacgaagt ccgctccatg tttgacagca cattcgcaac cctgtaccgc     240
   catctcgacg atggctttca acctatcaac ttcgtcatgc ctggtcttcc cctaccacaa     300
   aacttccgtc gcgatcacgc acgaaaggtc atggaggaat tgttcagcga catcatccgc     360
   aagcgtcgtg agattggaaa tcaaggcggt gagactgata tggtttggac gcttatgaat     420
   gctaaataca aggatggtga ggacttgccg gatcatcatg cggcgaggat gttgattgct     480
   attctgatgg gtgggcagca caacactgct gctagtggcg cctggctact tctcaatctt     540
   gcgcataaac cgcatctcgt tcaagagttg tatgatgagc agcttgaggt tttgggatca     600
   ccgcaagagc cgttgacgtg ggagaatttg cagaagttga cgctgaatgg acaggttatc     660
   aaggagacct tgcgtcttca cagtccaatc cactctattc tccgacaggt caagtcacct     720
   atgcgagttc ccggcacaga ctgggttgtt ccgccgtctc atacactcct cgcttctccc     780
   ggtacacaag cacgatctga ggaattcttt cctagaccta tggaatggga tcctcatcgg     840
   tgggataaaa ttgagtctct tgaggactcg aagggtaatg gggagacagt tgattatggc     900
   ttcggcgtga tgaacaagtc tgtgagcagc ccatatctac cctttggcgc gggacggcat     960
   cgctgtgttg gagagaacta cgcgtatgcc cagttgggcg ccatcattgc aacgtttgtg    1020
   agactgcttc atattgaaca gcctgatccc aatgcacctc tacctgcgcc tgactattcg    1080
   tcaatgtttt ctcggcctat gaacccagcc gtcatccgat ggactcgtcg taacacggag    1140
   gccaattag                                                           1149
<210> 11
   <211> 1338
   <212> DNA
   <213> Fusarium verticillioides
<400> 11
   atggagctgg tgcacggcga tgtcttcacc tttgtcctct tcggtcgaaa gatcgttgcc 60
   tgtcttggtg ttgatggcaa tgactttgtt ctcaacagtc gaattcaaga cgccaacgcc 120
   gaagaaatct acgggccatt gacaacgcct gtctttggca gcgatgtcgt atacgattgt 180
   ccaaactcaa agctcatgga gcaaaaaaag tttgtcaagt ttggtcttac ccaaaaggct 240
   cttgagtccc atgttcagtt gatcgaacga gaggttttag agtacatcca agcagtacct 300
   tcattctctg agaagtctgg cacagttgat gtatccaaag cgatggctga gatcaccatc 360
   tttactgctg cccgttctct gcaaggtgaa gaagttcgac ggaagcttac cgctgagttt 420
   gcagctctgt atcatgacct cgatctaggc tttactccgg tcaacttcct gttcccctgg 480
   ctacctctgc ctcacaaccg acgtcgagac gctgctcatt caaagatgag agagatctat 540
   atgggcatta tcaatgaacg aagaagaggc ggaggagact tggaaaaaag aaccgatatg 600
   atcgccaact tgatgagttg tgcctacaag aacaggcagc ccattcctga caaggagatc 660
   gcacacatga tgatcactct tctcatggcc ggacaacact cttcatcatc tgctagttca 720
   tggatcgtac tgcatctggc ttcatcccct gatatcactg aggaactcta ccaagagcaa 780
   gtcatcaact tgagtgctag cggcgctctc ccacccctgc agtactccga cctcgacaag 840
   ctcccgcttc tccagaatgt tgtcaaagaa acactccgag ttcattcttc tatccactct 900
   attctgcgaa aggtcaagag acccatgcaa gcacctggcc caccttacac catcaccacc 960
   gacaaggtta tcctcgcttc accaactgtt acagcgttga gtgaagaaca cttcccagac 1020
   gcccaaagat ggaatcctca tcggtgggat aataagcccc aggaggaggc cgtgacggac 1080
   gaagtcattg actacggcta cggtgctgtc tctaaaggaa caaaaagccc atatttaccc 1140
   tttggtgcgg gccggcatcg atgtatcgga gagaagtatg cttatgtcaa cttaggagtt 1200
   atcgtcgcga cgttggtgcg taacttcaga ctgtcgactc ttgatggcaa gcctggtgtt 1260
   ccagcaaccg acttcacttc tctcttctcg agaccagccc aacctgccta catcaaatgg 1320
   gaacgcagga aggcttag 1338
<210> 12
   <211> 1584
   <212> DNA
   <213> Fusarium verticillioides
<400> 12
   atgggtctcc tccaagaact tgcgagccac ccgctcgcac aacaatatca ggagcttccc 60
   ctggcccagc aaattggaat tggctttgga gctttcgtag ttctctctgt cgttctcaat 120
   gtactcaacc agctactctt caagaaccgc aatgaaccac ccctggtctt ccactggttc 180
   cccttcgttg gaagcactat cacatacgga atggaccccc ctaaattctt cagggagaac 240
   cgtgccaagc atggtgatgt cttcaccttt gtcctcctcg gaaagaaaac cactgtcgct 300
   gttggcccca ctggaaacga cttcatcctc aacggaaagc tcaaggatgt atctgccgag 360
   gagatttaca ctgttctcac tactcctgtc ttcggcaagg atgtcgtgta tgattgcccc 420
   aatgccaaac tcatggagca gaagaagttc atgaaaatcg ccctcacgac cgaagccttc 480
   cgatcatatg tgcctattat ctcctccgag gttcgcgatt acttcaagaa gagccccgat 540
   ttcaagggca agtccggtat tgtcgacatt cccaagaaaa tggccgagat cactatcttc 600
   actgcctcgc atgccctcca aggcagcgtt atccgtaaca agtttgacga gtctctggcc 660
   gctctctacc acgatctcga catgggtttc actcccatca actttatgct tcactgggct 720
   cctctgccct ggaaccgcaa gcgcgatcac gcccagcgca ctgttgccaa gatctatatg 780
   gacaccatta aggagcgacg cgctaaggac aacgatgaca ccgagcacga tatgatgaag 840
   catctcatga actctactta caagaacggc acccctgtcc ctgatcacga ggtcgcccac 900
   atgatgattg ctcttctcat ggctggccag cactcttctt cttctaccag ctcttggatt 960
   atgctccgtc tcgctcagta ccctcacatc atggaagagc tgtaccaaga gcaggttaga 1020
   gagctcggtg ctgatttgcc tcccctgact tacgacgacc ttgccaagct gcccctcaac 1080
   caggccatta tcaaggagac tctccgcctt cacgctccta ttcactccat catgcgcgcc 1140
   gtcaagtctc ccatgcctgt tcctggaacc aagtacacca tcccgacctc gcacactctt 1200
   ctcgccgctc ctggtgtcag cgctaccgac tcggcctact tccccaaccc cgatgagtgg 1260
   gatcctcacc gatgggaggc cgactctccc aacttcccca gaatggctaa ccgtggcgat 1320
   gacgaagaga agatcgacta cggctacggt cttgtcagca agggttctgc ctctccttat 1380
   ctgccctttg gtgctggtcg tcaccgatgc attggcgagc actttgccaa cgcccagctt 1440
   cagacaattg ttgctgaggt tgtgcgtgag ttcaagttcc gcaatgtgga tggcggcaac 1500
   actctgatcg acaccgacta cgcctcgctt ttctcacgac ccttggagcc cgccaacatc 1560
   cactgggaaa gacgacagca gtag 1584
<210> 13
   <211> 1146
   <212> DNA
   <213> Fusarium verticillioides
<400> 13
   atggatcaga agaggcttct taagctcggt ctcacgactg agtcgttgcg atgctacatc        60
   cccaaattcg tcaaagaagt agaagactac atcgccacgt caccatactt caaaggtaac       120
   accggaatcg tcaacatcac cgaagtaatg gccgagatca caatctacac tgcagcaggc       180
   tccctcctcg gcaacgaagt ccgctccatg ttcgatagca cattcgcaac gctttaccga       240
   catctcgacg atggtttcca gcccataaac ttcgtcatgc ctggtcttcc cttaccacag       300
   aacttccgac gcgatcatgc gcgaaaggtc atggaagagc ttttcagcga tatcattcgc       360
   aagcgtcgtg agatgggcaa tcaaggtgat gagactgata tggtttggac gcttatgaat       420
   gctaaataca aggatggtga ggatctgccg aatcatcatg ccgcgaggat gttgattgct       480
   attcttatgg gtggacagca taacactgct gcgagtggtg cttggctact tctcaacctt       540
   gcgcataaac cgcatctggt caaggaattg tatgacgaac aagttgaggt tttgggatca       600
   ccgcaggagc ccttgacgtg ggagaattta cagaaattga ccctcaatgg acaggtcatc       660
   aaggaaactc tacgtcttca cagtccaatt cattctattc tccggcaggt caaatcacct       720
   atgcgagttc ccggcacaga ctgggtagtt cctccatctc atacacttct cgcttcacct       780
   ggtacacaag ctcgatctga ggaattcttc cctcggccta tggaatggga tcctcatcga       840
   tgggataaga tcgagtctct tgatgatgcc aagaatgggg agacagtcga ttatgggttc       900
   ggtatgatga gcaagtccgt cagcagcccg tatctgcctt ttggtgcagg gcgacatcgt       960
   tgtgttgggg agaactacgc atatgcacag cttggtgcga ttattgcatc gtttgtgaga      1020
   ttgcttcata ttgagcagcc tgatcctaag gcacctcttc ctgcgccaga ttattcttca      1080
   atgttttctc ggcctatgaa cccagccgtc atccgatgga ctcgccgtaa cgcggagact      1140
   ggttag                                                                1146
<210> 14
   <211> 1593
   <212> DNA
   <213> Blumeria graminis
<400> 14
   atgggaatat cagaaagctt tatgtctcca tacttgcagc cgttgctcca attcggcttt 60
   agcattgcgt tggctagtgg aattataagt ttattattac tattaacctt cttgaatgta 120
   ttgaagcagc tactattcaa aaatccaaat gagccaccga tcgtgtttca ttggattcct 180
   atcattggta gtacaatttc atatggaatg aatccctata aattctttca tgaatcccaa 240
   gccaagtacg gaaatatttt cactttcata ttactgggta aaaagacgac agtatatcta 300
   ggtcgacagg gaaacaattt tattcttaat ggaaaactga gggatgttaa tgctgaagaa 360
   gtttattcag tcttaacgac tcctgtcttc gggactgatg tagtgtatga ctgtcctaat 420
   tcaaaattaa tggaacaaaa gaagttcatg aaagcagccc ttacaactga ggccttccgc 480
   tcttatgtac ctatcatcca aaatgaagtg gagagcttta taaataaatg cgacgatttt 540
   cgaaaatcag aaggtatcat caatatcgct gccgtaatgg ctgaaattac gatatatacc 600
   gcttcacaca ccctacaagg aaaagaggtt cgcgatagat ttgattcttc tttggcagtt 660
   ttgtatcatg acctagatat gggctttacc ccaatcaatt tcatgcttca ctgggcacca 720
   cttccgcaca atcgagctcg tgatcatgcc caacggacag tcgcaaaaat atacatggag 780
   attatcaaca gccgtcggac gcagaaagaa actgataatt ccaatttaga tatcatgtgg 840
   caattaatgc gctcttccta caaagatggc actcccgtac cggataaaga aattgcacat 900
   atgatgatcg cgctcctgat ggctgggcaa cattcttcgt cctcgtccag cacatggatc 960
   atgctgtggc ttgctgctcg accagatatc actgaagaac tctaccaaga acagctagaa 1020
   atattgggat cagaattacc tcctgctaaa tatgaagatc tctcgaaact tactctgcat 1080
   caaaatgtag tgaaagaggt cctccgtctg catgctccca tacattcgat cttacgaaaa 1140
   gtaaagaatc caatgcccgt tccaggaact agttatgtaa tacctaagac caattctctc 1200
   ttggcggccc ctgggtggac aagtcgagac gcctcatact tccctaatcc gcttacgtgg 1260
   gacccacatc gttgggacac tggatctagt ggggtgatag gcacggatat ggaggatgaa 1320
   aaattcgact atgggtatgg attaattagc acaggggcag caagccctta cttaccgttt 1380
   ggggccggac ggcatcgctg cataggcgag cagtttgcaa cggtgcaatt agttacaatc 1440
   atggccacca tggttcgcag tttcaagttt cacaaccttg acggaaggga gggcgttgcc 1500
   gaaactgatt actcaagtat gttttctcgg ccaatggcac ctgccataat tgcatgggag 1560
   aagagggaca aaaaggacaa aacggagtgt taa 1593
<210> 15
   <211> 1581
   <212> DNA
   <213> Magnaporthe grisea
<400> 15
   atgggccttc tacaggacac caccggcccg ctggtagatg ccttctacca gctgggcact      60
   ggggcgcagg ttggtgttgc cttcgtcagc ttcatcttcc tctcggtctt tttccacgta     120
   gcgcagcaga tcttcttcaa gaacccccat gagccgcccg tcgtcttcag ctggttcccc     180
   gtcgtcggtt cgacggtcac gtatggaaag gacccgccac agttcttccg ggacatggcc     240
   aagaagtacg gcaacatctt taccttcatt ctcctcggaa agaagacaac cgtctacatc     300
   ggcaccgaag gcaacgagtt catcctcaac ggcaagctgc gcgatgtcaa tgcagaggag     360
   atctacggac ccatgaccac tcccgtgttc ggcaaggacg tcgtctatga ctgccccaac     420
   gccaagctga tggagcaaaa gaagtttatg aagatcgccc tcaccaccga ggccttccgt     480
   tcttacgttc ctatcattgc cgacgaggtt tcgagctacc tgaagcggac ccccgccttc     540
   aagggcccgt cgggcgtcgt caacatcccg cccaagatgg ccgagattac catcttcacc     600
   gcctcgcacg ccctccaggg taaggagatc cgcgaccagt tcgacgagac cctggccgat     660
   ttgtaccacg acctcgacat gggcttccac ccggtcaact tcaagcttca ctggctaccc     720
   ctcccccgca acatccgccg cgacaaggca caaaagacca tcgccaagat ctacatggac     780
   accatccagc gccgccgtgc gaagggtaag gactccgagg ccaaggatat gatgtaccac     840
   cttatgaact cgacctacaa aaatggcacc cccgtccctg accacgagat cgcccacatg     900
   atgatcgctc tcctgatggc cggacagcac tcgtcgtcgt ccaccagctc ctggatcatg     960
   ctccgcctcg ccagccgccc ggatattatg gaggaactgt accaggagca ggtccgcgcg    1020
   ctgggtgccg acttgccccc tctccgctac gaggacctcg ccaatctgcc tctccacctc    1080
   gccgtcatca aggagactct ccgcctccac gctccgatca actccattct ccgcgccgtg    1140
   aagcaggacc tccccgttcc gggtaccaac tacgtcatcg ccaaggacac caccgtcctc    1200
   gccgcccccg gatactcggc cggtgacccc aaccacttcc ctgagccgga actttgggag    1260
   ccgcaccgtt gggaggccga ctcgcgcctg gctccacgca tctcgatgag caacgacaac    1320
   gatgaggagg agaagattga ttacggatac ggcctcgtca gcaagggtac tacctctcct    1380
   tacttgccct ttggcgccgg tcgccaccgc tgcatcggtg aacacttcgc caacgtccag    1440
   cttcagacca ttgtcgccat gattgtgcgc gagttcaagt tccgcaacgt cgacggcagc    1500
   ggcaaggtcg tcggcaccaa ctacgcctcg ctcttctcca ggcctgagga gcccgccaaa    1560
   atttactggg agaggcgcta a                                               1581
<210> 16
   <211> 1548
   <212> DNA
   <213> Magnaporthe grisea
<400> 16
   atggctttct tcttcccatc tgccccagtc tgggtttact cagccggcgc agccctactc      60
   ttcatcattg gctccataat actcaacttc atctggcagc aactgccccg tcccaaatcg     120
   gaaccaccgc tggttttcca ctggttgccc ttcatcggca acgccgtctc ctacggcatg     180
   gacccctacc gcttctactc tcaatgccgg gaaaagcacg gcgatgtctt tacgtttgtc     240
   ttgttcggca ggcgcatgac cgtcttctta ggcgtccagg gcaacgactt catcctgaac     300
   ggcaagctgc aggacctcaa tgctgaggag atatacagcc ctctcaccac accagttttc     360
   ggaagtgata ttatctacga ctgccccaac tccaaactca tggagcaaaa gaagtttgtc     420
   aagtttggcc tgacgcaaaa ggctctggat tcctacgtcc ccctgatcga gagggaggtc     480
   ctcgactaca tagagtcctc acccgtcttc caagccggca accacggcat cgtagacatt     540
   cccagcatga tggccgagat aaccattttc acagccagtc gtaccttgca aggccccgag     600
   gtcaggaaga agctgactgg agaattcgca cgactctatc acgacttgga cctgggtttc     660
   cgccccatca acttcctagc cccgtgggcg ccccttcccc agaaccgccg acgcgacgtt     720
   gctcatgctc gaatgcgcga tgtttacatg gacctcatca acaaacgccg ccgacagaag     780
   gacgatcaag aagaagaaga agaagcagag ccagacatga tccgccacct gatgggcagc     840
   tgcgtgtaca aaaacggcca agccctcccc gacaaggaaa tcgcccacat gatgatcacg     900
   cttctcatgg caggccagca ttcttcctcc tcttcgagcg cgtggatcat gctccgcctc     960
   gcgtcgcggc ccgacatcgc cgaggaggtg taccaagaag tgcagcggct cgggcacgca    1020
   tccctgcagc actcggacct cgacaagctg ccgctgctcg caaacgtggt caaggagacg    1080
   ctgcgggtgc actcgtccat ccactccatc atgcgcaagg tgaagcggcc gatgcggatt    1140
   ccgggcagcg actacgtcgt caccccgggc aaggtgctcg tgtccgcgcc catcatgacg    1200
   cacctggacg aggagcactt ccgcgacgcg cgggcttggg agccgcatcg gtgggatgac    1260
   gccgtcgacg cccaggacga cgagattgtc gattacggct acggggccac gtccaagggc    1320
   accaagagtc cttacctgcc ctttggcgcc ggtcggcatc gctgcatcgg tgaaaagttt    1380
   gcctatctca acctggcggc catcgtcagc accttggtcc ggaacttcaa gttctccacg    1440
   ctggatggca aggccaccgt gccgcccact gattacactt ctatgttctc tcggcctatg    1500
   cagcctgcga cggtgaggtg ggagcgacgc agcccgaaga ctgcgtag                 1548
<210> 17
   <211> 1569
   <212> DNA
   <213> Sclerotinia sclerotinium
<400> 17
   atgggtattc tcgaaacaat tgccgggcca ttggctcaag agatttcgca aaggtcaacc 60
   tttgctgttg ttgctgctgg cgtggcagca ttcgtcgttc tatctgtcat tctcaatgtc 120
   ctgaatcaag tgttatttgc gaaccccaat gaaccaccag tggtcttcca ctggtttcca 180
   atcattggta gcaccgtcac ttatggtatg gacccttata aattcttctt cgagtgtcgc 240
   gcaaagtacg gtgatatttt cacatttgtc ttgctcggaa agaagaatac agtatatctt 300
   ggacgaaatg gcaatgactt tattctcaat ggcaagctta aggatctcaa tgcggaggaa 360
   atatatactg ttttgacaac tcccgtgttt ggaaaggatg tagtctacga ttgccccaat 420
   gcgaaattga tggagcaaaa aaagttcatg aaaattggct tgtctactga agctttccga 480
   tcctacgtcc caattataca aatggaagtg gaaaacttca tgaaacgttc ttcggtattc 540
   aagggacaaa agggaactgc cgatattggt cccgctatgg ctgaaatcac catctatacc 600
   gcttcgcata ctctacaagg aaaggaagtc cgtgatcgat ttgatactac tttcgcctct 660
   ctctaccacg accttgatat gggctttagt cccatcaact ttatgcttca ctgggctcct 720
   cttcctcaca accgtgcccg cgaccatgcg cagagaactg tcgcagcaac atatatggat 780
   attattaaaa aacgacgtgc tcaggctacg gaagccgact tcaaatccga cattatgtgg 840
   caattgatgc gctcgtccta caaagatgga acccccgttc cagaccgaga gattgctcac 900
   atgatgatcg ctcttctcat ggccggacag cactcttcct catcttctat ctcttggatt 960
   ctgcttcgtc ttgcctcacg cccagatatc atggaagaac tctatcaaga acaaatccaa 1020
   gttctgggcg ccgatctccc tgctctcaag tacgaggacc tggccaaact tcctcttcat 1080
   caaaacatct tgaaggaaac tctccgcatc cacactccca tccattctat tatgcgcaaa 1140
   gtcacaacac caatgccaat tagcggaaca aaatatgtca ttccaacctc gcatactctt 1200
   atggcatctc ctggttgtac aagtcgagac gcggattact tcccagagcc acttgagtgg 1260
   gaccctcata gatgggacat tggctcgggc cgtgtaattg gcaatgatca ggacgaagaa 1320
   ttccaagatt atggctatgg aatgatcagc aaaggtgctt ctagtcctta ccttccattc 1380
   ggtgctggca gacacaggtg tatcggtgaa caattcgcca atgtacagct catcactatc 1440
   atggccactg tggttagaat gttcaaattc aagaacgttg atggcagcaa ggatgtcatt 1500
   ggtactgatt acaccagttt attcaccagg ccattggcgc cagcagttat agcatgggag 1560
   cgacgataa 1569
<210> 18
   <211> 1587
   <212> DNA
   <213> Phakopsora pachyrhizi
<400> 18
   atgtcttcca gcgttataat cgatcagctc tactcattct caactaccag tctcatcatc      60
   acatcgctaa cttccatcct cacaatcatc gtcatcctca atgtcactaa tcagttgttg     120
   ttcaaggaca ggaatacccc accgctagtc ttccacatat ttccggtgct cggctctgtg      180
   ataagctatg gcatggatcc gtaccaattc tttgaggact gtcggaggaa gcatggaaat     240
   gtgtttacgt ttgtgctgtt gaacaagaag gtcaccgtag ctctcggtcc ggagggaaac     300
   acgttggtct taaatggaaa gctctcagag gtcaatgccg aagaagctta cacccatttc     360
   actacacctg tctttggtaa ggatgtagtc taygatgtac ccaactcgat cctaatgcag     420
   cagaaaaagt tcatcaaggc tggccttact actgagaaat ttaagaaata tgtggggata     480
   attgttagag aarcaacaag ttacctggaa gatcatttat tctgctctcc aaatgtaaaa     540
   tctgtcacta aggacgtcca tgatattact tctgagataa cgatttgtac tgcagcagca     600
   actctacagg gaaaagaagt cagagaaggg ctagacaaat cctttgctaa actctatcac     660
   gacctagatg gtggatttac accgttgaac tttgtttttc cgaacctgcc cttaccctcc     720
   tacagacgta gggacaaggc acaggtctcg atgacaaact tttatctgaa cattctaaag     780
   aaaaggagag ccgaaaacag gcaggatgag ttcaatgaca tgttggatgt cttgcaggrg     840
   cagcactaca aggacggcag agctttatcg gacagggaaa ttgctcacat aatgattgcc     900
   gtccttatgg ctggtcaaca cactagtgct gcgaccggcg cctggttatt gacccatctg     960
   gctcattgcc ctgacttggt tgatagactg aggcgtgaac aggycgaggt ctttggtaag    1020
   ggggatggta gtggagagtt ggaagatcta gattacgatc gactccaaac acctctatta    1080
   aattcttgta tcaaagaggt cttacgtcta caccctccca ttcattcaat cttgcgcaaa    1140
   gttaagtcac cgatccttgt tcccaaaaca ctctcctcaa ttgacaagaa caaccaatac    1200
   atcataccct cttcccacta cgtcctagcc gcacctggcg tctctcagat tgatccatcg    1260
   gtttgggacc atccaaaaga gttcagacca gagagatggc tatcgaactt taagaaagat    1320
   aagcaggaac aggaggagga gatggttgac tacggctttg gagcgatcag cagcggagcc    1380
   aattcgccct atctaccctt tggggctggt cgacatcggt gtatcggaga gcagtttgcg    1440
   tacatccagt tggccgccgt tgctgttgct gtcattagga actgcgacct cgagctcgtc    1500
   agaaaggagt tccctctacc tgattatacc acgatgttag ttggacccag gaaacctaca    1560
   actgtgaagt ttactagaag aaattaa                                        1587
<210> 19
   <211> 1569
   <212> DNA
   <213> Botrytis cinerea
<400> 19
   atggggattc ttgaagctgt aaccgggcca ttggctcagg aaatttcgca gcggtcaact 60
   ggcgttgttg tagctgctgg cgtggcagca ttcatcgttt tatctgtcgt tctcaatgtt 120
   ttgaaccaag tgttatttgc aaaccccaat gagcctccca tggtcttcca ctggctccca 180
   gtaattggca gcactatcac ttatggtatg gacccttaca aattcttctt cgattgtcgc 240
   gcaaagtatg gtgacatttt cacatttgtc ttgcttggaa agaagaccac cgtatatctc 300
   ggacgcaagg gcaacgactt tattctcaat ggcaagctca aggatctcaa cgcggaggag 360
   atatatactg ttttgacaac ccccgtattt ggcaaagatg tagtttacga ctgcccaaat 420
   gcgaagttga tggagcaaaa gaagttcatg aaaattggct tgtctacaga agctttccga 480
   tcctacgtcc caatcataca aatggaggtg gaaaacttta tgaagcgttc ttcggcgttc 540
   aaaggtccaa agggaactgc tgacattggt cccgctatgg ctgaaatcac catctacact 600
   gcttcgcaca ctctgcaagg aaaggaagtc cgcgatcgat tcgatacctc ctttgcctct 660
   ctctaccacg acctcgacat gggcttcagt cctatcaact ttatgcttca ctgggcccct 720
   ctccctcaca accgtgcccg cgatcatgcc cagcgaactg tagccaaaac ctatatggat 780
   atcattcaaa accgacgtgc tcaagctacg gaagcagagt tcaaatctga tattatgtgg 840
   cagttgatgc gctcgtctta caaggatgga actccagttc cagataagga aatcgctaac 900
   atgatgattg ctcttctcat ggccggacaa cattcttcct catcttctat ctcgtggatt 960
   atgcttcgcc tcgcctcacg cccagacatt atggaagaac tctaccaaga acaaatccaa 1020
   gtcttaggtg ctgatctccc tgctctcaag tatgaggatt tgtccaaact ccctctccat 1080
   cagaacgttc tcaaggaaac tctacgcctc cacaccccaa tccattctat catgcgcaaa 1140
   gttaccacac caatgccaat cagcggaacc aaatatgtca ttccaacatc acatacactt 1200
   atggcatctc ctggttgcac aagtcgagat gacgaattct tccccgaagc acttgagtgg 1260
   gatcctcaca gatgggatct tggttccggc cgtgttgttg gaaatgatca ggatgaggaa 1320
   ttccaagatt atggttacgg aatgattagc aaaggcgctt ccagtcctta ccttccattt 1380
   ggtgctggca gacatagatg cattggtgaa caattcgcga ctgtacagct tgtcacgatc 1440
   atggcaactg tcgttagatt gttcaaattt aagaacattg atggcagcaa ggatgtgatt 1500
   ggtaccgatt acgcaagttt attcaccagg ccattggcgc cagccgttgt agcttgggag 1560
   cgacgataa 1569
<210> 20
   <211> 1569
   <212> DNA
   <213> Puccinia triticina
<400> 20
   atgtcttctg tgatcggctc gctgctcgag cccatcggat ccttctcaac tttcaaccaa      60
   gtgctcatct acttggtgct agccgttgtc tcgatcatct cgatcaacat cttcgaccaa     120
   ttggctattc ccaaagatcc gacggctcca ccagtggtgt ttcatttgtt tccgttcatt     180
   ggctcggccg tgtcctacgg aatcgacccc tacgctttct tggaatcatg caggaagaag     240
   tatgggaatg tgttcacctt cgtcctcttg aacaagaaag tcaccgtcgc tctcggtctc     300
   gaaggaaacg cactggtcct caacggaaaa ctatctcaag ttaatgctga agaagcttac     360
   acagcactca caatcttgat gcaacaaaag aaattcgtca agtctgggtt gactaacgaa     420
   aactttcgga aatacgtatc actgattgcg gaggagacga taagctatct tgaagatcat     480
   gtatttgaga accccaaaac gcaacagact gtgaaagata ccttcaaagt ggcttctgag     540
   attactatct gcacggcctc agccaccctc caagggcccg aagtccgaga agctctcaac     600
   aaatcattcg cccagttata ccatgactta gatggcggtt ttaccccttt gcatttcgct     660
   ttccccaact tgcccttacc atcatatcgg cgtcgagacc gggcgcaact ggcgatgcga     720
   aacttctata tgaacatcat caagaagaga cgagaagatg acagagaggg ccagcttgga     780
   gacatgatcg atagcttaca gggccaaacg tacaaggatg gccgcccttt gaacgataaa     840
   gagatcgccc acattatgat tgcccttctg atggccggcc aacacaccag tgctgctact     900
   ggctcctggc tactcctgca tctcgcttct cgtcccgaca tcgtcgctga attgagacag     960
   gagcagatcg acttgtttgg caaaccgggc caaactgacg atcaagaact cgaccctcta    1020
   gaccttgaac gtgtccagag tcccttaatg attgcatgca tcaaggaagt cctcaggctt    1080
   catcccccaa tccattctat catgcgaaag gttaaatcgc ctatcaccgt gccgaggact    1140
   ttggcgtcac gcaacgagga tacaccatac ataatccctt caagcaactt tgtcttggca    1200
   gcgccgggaa cagcccagct cgacggatcg atctggagct cgccccacga gttcgacccg    1260
   agccgatggc tgaagctcca gtctcctttc aaggccggag agacgcagga agagatggtc    1320
   gattacggct tcgggatgat cagcagtggg gctaattcgc ccttcctgcc cttcggcgcc    1380
   ggccgtcatc gctgtatcgg tgaacagttc gcttacatcc agctgtctac tttcgccgct    1440
   accgtcatca ggaactgcga tctcgaatta actgcccctg agttccctaa acctgattat    1500
   accgttcgtt tatctctctg tctctcacac atttcgatta tcagccgtca agttgaagct    1560
   catctctga                                                            1569
<210> 21
   <211> 1524
   <212> DNA
   <213> Puccinia graminis
<400> 21
   atgtcttccc tcatcgaccc actgatcgag ttcattggct cattctcaac cttcaatcaa 60
   atcctcatct acttcctact ctccatcacc tcgatcatct ccatcaacat cttcaaccaa 120
   ttggctattc ccaaagatcc gaccacccca cctgtggtct tccatctatt cccattcatc 180
   ggctcagctg tgtcctacgg aatcgaccct tacgctttcc tcgaatcttg tcggaagaaa 240
   tacgggaatg tcttcacctt cgtcctcttg aataaaaaag tcaccgtcgc actcggtctc 300
   gaaggaaacg cattgatcct caacggaaaa ctgtctcaag ttaatgctga agaagcttac 360
   acagcactca ctacacctgt gttcggaact aaaatggtct atgatacgat aagctacctc 420
   gaagaccatg tatttgagaa accgaaaacg cagcaagctg tcaaagactg cttcaaagtg 480
   gcatctgaga tcactatctg cacagcctca gccacccttc aaggtcccga agtccgcgaa 540
   ggactcaaca aatcatttgc caatctatac cacgatttag acggcgggtt taccccacta 600
   catttcgcat tccccaacct acccttacca tcatatcgac gacgagatcg ggcgcaagtg 660
   gcgatgcgca acttctacat gaacataatc cagaagagac gagaggacaa ccgagaaggc 720
   cagctcggag acatgatcga cagcttgcag ggccaaacct acaaggatgg gcggccgttg 780
   accgacaagg agatcgctca tatcatgatt gcccttctga tggccggcca acataccagt 840
   gctgccactg gatcatggct cctcctccac ctcgcctctc gcccagatat tgttgccgaa 900
   ttgaggcagg aacagatcga agtgttcggc aaacctggac aaactgatga taaagaactc 960
   gaccctctag acctcgaacg tgtgcagagt cccttgatgc tcgcttgcat caaggaggtc 1020
   ctcagacttc atccgcccat ccattcaatc atgcgaaagg tcaaatcacc gatcactgtc 1080
   ccgcgaacat tagcatccca taacgaagat acgccataca tcattccgtc gagtaacttt 1140
   gtcttagcag cgccgggggc atcgcagatc gacccggcaa tctggagctc acctcacgag 1200
   ttcgagccca gtcgatggct gaagctcacc tcgcccttca aggccggcgg gggagagaca 1260
   caagaagaga tggtcgacta cggcttcgga atgatcagca gcggcgccaa ctcgcccttc 1320
   ctccccttcg gcgcaggccg tcatcgctgt atcggcgaac agttcgctta ccttcagctc 1380
   tctactctcg gcgctaccgt cattaggaac tgcgaactcg aactagtctc caatcagttc 1440
   cctaaacctg attatactac tatgttggtc tgtcctatca aaccaagaga cgtcaagttt 1500
   actaggagga acacccactc gtaa 1524
<210> 22
   <211> 1611
   <212> DNA
   <213> Puccinia recondita
<400> 22
   atgtcttctg tgatcggctc gctgctcgag cccatcggat ccttctcaac tttcaaccaa      60
   gtgctcatct acttggtgct agccgttgtc tcgatcatct cgatcaacat cttcgaccaa     120
   ttggctattc ccaaagatcc gacggctcca ccagtggtgt ttcatttgtt tccgttcatt     180
   ggctcggccg tgtcctacgg aatcgacccc tacgctttct tggaatcatg caggaagaag     240
   tatgggaatg tgttcacctt cgtcctcttg aacaagaaag tcaccgtcgc tctcggtctc     300
   gaaggaaacg cactggtcct caacggaaaa ctatctcaag ttaatgctga agaagcttac     360
   acagcactca cgacccctgt attcggaact gatgttgtct atgatgtccc caacgcaatc     420
   ttgatgcaac aaaagaaatt cgtcaagtct gggttgacta acgaaaactt tcggaaatac     480
   gtatcactga ttgcggagga gacgataagc tatcttgaag atcatgtatt tgagaacccc     540
   aaaacgcaac agactgtgaa agataccttc aaagtggctt ctgagattac tatctgcacg     600
   gcctcagcca ccctccaagg gcccgaagtc cgagaagctc tcaacaaatc attcgcccag     660
   ttataccatg acttagatgg cggttttacc cctttgcatt tcgctttccc caacttaccc     720
   ttaccatcat atcggcgtcg agaccgggcg caactggcga tgcgaaactt ctatatgaac     780
   atcatcaaga agagacgaga agatgacaga gagggccagc ttggagacat gatcgatagc     840
   ttacagggcc aaacgtacaa ggatggccgc cctttgaacg ataaagagat cgcccacatt     900
   atgattgccc ttctgatggc cggccaacac accagtgctg ctactggctc ctggctactc     960
   ctgcatctcg cttctcgtcc cgacatcgtc gctgaattga gacaggagca gatcgacttg    1020
   tttggcaaac cgggccaaac tgacgatcaa gaactcgacc ctctagacct cgaacgtgtc    1080
   cagagtccct taatgattgc atgcatcaag gaagtcctca ggcttcatcc cccaatccat    1140
   tctatcatgc gaaaggttaa atcgcctatc accgtgccga ggactttggc gtcacgcaac    1200
   gaggatacac catacataat cccttcaagc aactttgtct tggcagcgcc gggaacagcc    1260
   cagctcgacg gatcgatctg gagctcgccc cacgagttcg acccgagccg atggctgaag    1320
   ctccagtctc ctttcaaggc cggagagacg caggaagaga tggtcgatta cggcttcggg    1380
   atgatcagca gtggggctaa ttcgcccttc ctgcccttcg gcgccggccg tcatcgctgt    1440
   atcggtgaac agttcgctta catccagctg tctactttcg ccgctaccgt catcaggaac    1500
   tgcgatctcg aattaactgc ccctgagttc cctaaacctg attataccac catgttggtt    1560
   tgtcctctga aaccaaggga catcaaattt actcgaagaa accatctctg a             1611
<210> 23
   <211> 1584
   <212> DNA
   <213> Pyrenophora teres
<400> 23
   atgggtctct tcgctgatgt cgccggccct ctggctaact ttacatccaa gtcgtccacg      60
   cctgtcgtcg ctgccgccgc cttcgcctcc tttatcctgc tctccgtcgt cctcaatgta     120
   ctcaagcagt tgctgttcaa gaaggcgaat gagccgccca tggtcttcca ttggctgcct     180
   ataattggca gtacagtcac atatggcatg gatccgtatg ccttcttctt tgcgaatcac     240
   aagaagtatg gcaatgtctt cacatttatt ctccttggcc gcaagatgac cgtgtgcctt     300
   gacacggcgg gcaacaactt catcctaaac ggaaagatca aggacgtcaa cgccgaggag     360
   atttactctc cgctgaccac ccccgtcttc ggcaaggacg tcgtgtacga ctgtccaaac     420
   tcgaaactca tggagcagaa aaagtttgtc aagtatggac tcacgcagga ggccctccgt     480
   tcctacgtca ccctcatcac acaagaatgc gaagacttca tgaagcgcca caaagctttc     540
   aagggacaac ggggcacttt tgacgtgacc aaggtcatgg ctgagctcac catctacact     600
   gcctctcgtt cgctccaggg cgaggaaatc cgcaagtcgt tcgattcaaa gtttgccgaa     660
   ctgtaccacg acctcgacat gggcttttca cctgtcaact ttatgctctc atgggccccg     720
   cttccccaca accgcgcacg cgacaatgca cgcgagacta tgataaagct atactctgat     780
   ctggttcgca agcgcaggtc gggcgccgtg aagaaggact cacacgacat gatttggcac     840
   ctgatggaat gcaagtacaa ggacggcacc caggtgccgg aacacgagat tgctggcatc     900
   atgattgccc tgctcatggc tggacagcat tcgtcttcct cgaccatcgc atggatcctc     960
   ctccgcctcg cgcagaaccc gcacattatt gacgaactgc ttgcggagca aacgtccatc    1020
   ctaggcaaaa acctccccgc ccttacctat gacgacctcc agaagcttcc ccttcacgcc    1080
   caagtcgtca aggaaaccct ccgtatccac gcccccatcc actccatcat gcgcaaggtc    1140
   aagcaacctc tggttgtcga tggaacaaat tacgttgttc ccacttccca cacactcatg    1200
   tcctcgcccg gtttctctgc acagctcgat acccactttg tcaaccccgc tgtctgggac    1260
   ccccaccgtt gggatccaga ccaaaacaac tacgacgaag agcgcgacga cgccgatcag    1320
   gagaagattg actatggctg gggtgtcgtg tccaagggaa ccaactcgcc atatcttccc    1380
   tttggcgctg gacggcatcg ttgcattggt gagcaatttg cctacttgca attacagact    1440
   atcctagtag cgtttgtgag agagttcaag ctcaggaatg ttggtggtag taaagacatt    1500
   gttggcaccg actactccag tctcttctcc cgcccactag cacctggcat agttgagtgg    1560
   gagaggcggc agaaggaatg ttag                                           1584
<210> 24
   <211> 1584
   <212> DNA
   <213> Pyrenophora tritici
<400> 24
   atgggtctct tcgctgatgt cgccggccct ctggctaact ttacatccaa gtcgtccacg 60
   cctgtcgtcg ctgccgccgc cttcgcctcc tttatcctgc tctccgtcgt cctcaatgta 120
   ctcaagcagt tgctgttcaa gaaggcgaat gagccgccca tggtcttcca ttggctgcct 180
   ataattggca gtacagtcac atatggcatg gatccgtatg ccttcttctt tgcgaatcac 240
   aagaagtatg gcaatgtctt cacatttatt ctccttggcc gcaagatgac cgtgtgcctt 300
   gacacggcgg gcaacaactt catcctaaac ggaaagatca aggacgtcaa cgccgaggag 360
   atttactctc cgctgaccac ccccgtcttc ggcaaggacg tcgtgtacga ctgtccaaac 420
   tcgaaactca tggagcagaa aaagtttgtc aagtatggac tcacgcagga ggccctccgt 480
   tcctacgtca ccctcatcac acaagaatgc gaagacttca tgaagcgcca caaagctttc 540
   aagggacaac ggggcacttt tgacgtgacc aaggtcatgg ctgagctcac catctacact 600
   gcctctcgtt cgctccaggg cgaggaaatc cgcaagtcgt tcgattcaaa gtttgccgaa 660
   ctgtaccacg acctcgacat gggcttttca cctgtcaact ttatgctctc atgggccccg 720
   cttccccaca accgcgcacg cgacaatgca cgcgagacta tgataaagct atactctgat 780
   ctggttcgca agcgcaggtc gggcgccgtg aagaaggact cacacgacat gatttggcac 840
   ctgatggaat gcaagtacaa ggacggcacc caggtgccgg aacacgagat tgctggcatc 900
   atgattgccc tgctcatggc tggacagcat tcgtcttcct cgaccatcgc atggatcctc 960
   ctccgcctcg cgcagaaccc gcacattatt gacgaactgc ttgcggagca aacgtccatc 1020
   ctaggcaaaa acctccccgc ccttacctat gacgacctcc agaagcttcc ccttcacgcc 1080
   caagtcgtca aggaaaccct ccgtatccac gcccccatcc actccatcat gcgcaaggtc 1140
   aagcaacctc tggttgtcga tggaacaaat tacgttgttc ccacttccca cacactcatg 1200
   tcctcgcccg gtttctctgc acagctcgat acccactttg tcaaccccgc tgtctgggac 1260
   ccccaccgtt gggatccaga ccaaaacaac tacgacgaag agcgcgacga cgccgatcag 1320
   gagaagattg actatggctg gggtgtcgtg tccaagggaa ccaactcgcc atatcttccc 1380
   tttggcgctg gacggcatcg ttgcattggt gagcaatttg cctacttgca attacagact 1440
   atcctagtag cgtttgtgag agagttcaag ctcaggaatg ttggtggtag taaagacatt 1500
   gttggcaccg actactccag tctcttctcc cgcccactag cacctggcat agttgagtgg 1560
   gagaggcggc agaaggaatg ttag 1584
<210> 25
   <211> 1512
   <212> DNA
   <213> Pyrenophora tritici
<400> 25
   atgccctctc cccttctcct cgcatccggt ttcctcgccc tctgcatcgc ctacatcttc      60
   gccaacatca tccgccagct gcttcttcca aacaccaaag aaccacccgt cgtcttccac     120
   tggcttccct ggcttgggag cgccatcacc tacggcaaag acccctataa gtttctgttc     180
   gccgccagag agaagcatgg agatgtcttc acctttgtcc tgctaggccg caacgtcacc     240
   gttcacttgg gcgttgccgg aaacgatttt gtcttcaacg gcaaagagac acatgtcaat     300
   gccgaggaaa tctacggtcc cctatgtaac cccgtcttcg gcgagggcgt cgtgtacgac     360
   tgtcccaatt ccaagctcat ggaacagaaa aaattcgtca agtttggtct gacaaccgac     420
   gctctcaagg cacatgtgca actgattgag caagaggtcg tagactacat caaagcctcc     480
   cgagagttca agggacaatc aggcaccatc aatgtgcccc ccgtcatggc tcaaatcacc     540
   atcttcaccg ccgccatcgc cttacaaggg cctgaagtgc gcagcaagct cacaaacgag     600
   tttgcaagcc tataccacga tctcgacggc ggttttagtc ccatcaattt tgttctccct     660
   cgcgcgccct tcccccacaa catcaagaga gatcgggccc agctaaagat gcgcaagatt     720
   tacgagacca tcatcgcaga acgccgcgct ggcaagatcc ctcccaccac cgacatgatc     780
   agtcatctca tgcagtgctc gtataaagac ggccgtcctg tgccagactc ggaaatcgca     840
   aacatgatga ttaccattct aatggcgggc cagcacaact cctccaacat tgcgtcttgg     900
   atcatgttgc accttgccaa caagccgcaa ctctgcgaag agctatacca ggaacagctt     960
   gatcaactgg ctgatgagca tggcaacttg cccaagctcg acctgcagac cttggagaag    1020
   ctcaagctgc attctaatgt cgtcaaagag acgcttcgca tgcacaactc cattcactct    1080
   atcatgcgcc ttgtcaaaca gccactcccc gtccccaata cgtcgtggac cataccgcct    1140
   ggccatgccc ttctcgcttc acctggtatc tcagcaaaca gcgaagaata cttctataac    1200
   ccggataagt ggaatccaca tcgctgggac gaccgcgtca tcgaagaaga cgatgagagc    1260
   gagatggtgg actacggcta tggacgcatg tccaggggta caaagagcgc ctacctcccg    1320
   ttcggtggag gtcgccatcg ctgcattggc gaaaagtttg cttacctcaa cttggaagtc    1380
   atcacggcga ttatggtgcg aaacttttgc tttaagaatc tcgatggtag ggagggtgtt    1440
   ccaggcaccg attacagcac catgttttcg cgtccgctag agcctgccga gattgtttgg    1500
   cagaggcgat ga                                                        1512
<210> 26
   <211> 1599
   <212> DNA
   <213> Phaeosphaeria nodorum
<400> 26
   atgggaattc tcgctgatgt cgccggaccc gtggggaact tcacctctca atcgtccacc      60
   gccactctca ttgccgcggg cttcgcctcc tttatcgtcc tctccgtcgt tctcaacgtt     120
   ttgaagcagc tgctgtggaa ggatcccact gcgcccccgg tcgtcttcca cctgttcccg     180
   atcatcggaa gcaccgtcac gtacggcatc gacccgtaca agttcttctt cgcgcagcag     240
   aagaagaagg cgcaagctaa caagagacag tatggcgatg tcttcacctt catccttctc     300
   ggccgcaaga tgaccgtgtg tctcgacacc aagggcaaca acctcatcct gaacggaaag     360
   ctcaaggagg tcaatgccga ggagatctac tcgccgctca ccacacccgt cttcggcaag     420
   gatgtcgtct acgactgccc gaactcgaag ctcatggagc agaagaagtt cgtcaagttt     480
   ggtctgaccc aggaagccct ccgctcctac gttggcatca tcacccaaga atgcgaggat     540
   tttttcaagc gtcacaaggc tttcaaggga caaaagggca cttttgatgt gaccaaggtc     600
   atggccgagc tcaccatcta caccgcctcc cactctcttc agggtaagga gatccgcaag     660
   tcgttcgact ccaagtttgc cgacctttac cacgatctcg atatgggctt ctctcccgtc     720
   aacttcatgc tgtcatgggc ccccctcccc cacaaccgcg cccgcgacgt cgctcgcgag     780
   acaatgatca agctatactc cgagattgtg cgcaagcgaa gggctggcaa cgtcaagaag     840
   gactcgcatg acatgatctg gcacttgatg gactgcaagt acaaggatgg cacacaggtt     900
   cctgaacacg agatcgctgg tatcatgatt gcgctgctga tggctggaca acactcctct     960
   tcctccacaa tcgcctggat tattctccgt ctggcacagt accctcacct tctcgaagag    1020
   cttcttgcag agcagaaggc ggtcatgggc gaggaccttc cagcagtcac ctacgaagac    1080
   ctcaacaagc tgccgctaca cgcccaggtt gtcaaggaga ctcttcgcat ccacgctcct    1140
   atccactcaa tcatgcgcac cgtcaaatcg cctatcgttg ttgagggaac aaactacgtt    1200
   atccccactt cgcacaacct catgtcctcc cccggctact ccgcactcct cgacagctac    1260
   tttgtcaacg ctgcaacctg ggacccacat cgatgggacg ccggccagca caactacgac    1320
   gaggctgaga acgacgacga cgagaagatc gactatggct ggggtgttgt ctccaaaggc    1380
   acaaactcgc cttacctacc atttggtgct ggaaggcatc gatgcattgg cgagcagttc    1440
   gcctacctgc agctccagac gatcctcgtc gcatttgtca gggagttcaa gttcaggaac    1500
   gttaacggta gcaaggacat tgttggtaca gattacacaa gtctgttctc gagaccgctt    1560
   gcacctggta cggtcgagtg ggagcgcagg gaatcatag                            1599
<210> 27
   <211> 1635
   <212> DNA
   <213> Septoria tritici
<400> 27
   atgggtctcc tccaggaagt cctcgcgcag ttcgacgcgc aattcggcca gaccagcctc      60
   tggaaacttg tcggacttgg attcctcgcc ttcagcacgc tcgccatcct cctcaatgtc     120
   ctctcacaac ttctcttccg tggcaagttg tccgatccgc cactcgtatt ccactgggtg     180
   cccttcatcg gaagcaccat cacatacggc atcgacccat acaagttctt cttctcctgt     240
   cgggaaaagt atggagatgt ctttacattc atcctgctgg gaaagaagac gacggtgtgc     300
   ttgggcacca agggcaatga ttttattttg aatggaaaac tgaaggacgt caacgcggag     360
   gagatataca gcccgctgac cactcctgtc tttggcaagg atgtggttta tgattgtccc     420
   aattcgaagc tcatggagca gaagaagttc gtcaagtacg gcctcacaac ctctgccctc     480
   cagtcctacg tgaccttgat cgccgccgag acccgccagt tcttcgaccg caacaaccct    540
   cataagaagt tcgcatcgac cagcggcacg atcgatctcc caccagccct cgccgaactt     600
   acgatctata ctgccagccg atcattgcaa ggaaaggaag tccgcgaggg cttcgactcg     660
   tctttcgcgg acctctacca ctacctcgat atgggattca caccgatcaa ctttatgctt     720
   ccgtgggcgc cccttcccca gaaccgacgc cgcgattatg cgcagaagaa gatgtccgag     780
   acatacatgt cgatcattca gaagagacga gagtccaaaa cgggcgaaca tgaggaagac     840
   atgatccaca acttgatgca gtgcaaatac aaggacggca atgccattcc cgacaaggag     900
   attgctcata tgatgattgc gctgctcatg gccggccagc actcttcatc tgcgaccgag     960
   tcctggatca ctctccgcct cgcatcccgc cccgacatcc aagacgaact cctccaagaa    1020
   caaaaggata tgctcggtgt gaacgccgac ggcagtatca aggagctcac atacgccaac    1080
   ctctcgaaac tcaccctcct caatcaagtc gtcaaagaaa cccttcgtat tcacgctcca    1140
   atccactcca ttctgcgcaa ggtcaagtct cccatgccca tcgaaggtac ggcatacgtc    1200
   attccaacca cccacactct tctggccgct ccgggcacaa cgagccgcat ggacgagcac    1260
   tttcccgact gcctccattg ggagccgcat cgatgggacg agagcccgtc cgagaaatac    1320
   aagcacctgt ccccgacgac tgccctagga agcatcgccg aggagaaaga agactatggc    1380
   tacggcctgg taagcaaggg cgcggcgtcg ccatacttac cctttggtgc gggacgacac    1440
   agatgtatcg gcgagcaatt cgcgtacgtg caattgcaga ccattacagc gacgatggtt    1500
   cgcgatttca agttttacaa tgtggatggc agcgacaacg tggtgggtac ggattacagc    1560
   agtttgttca gccggccgct gtcgccggca gtagtaaagt gggagaggag ggaggagaag    1620
   gaggagaaga actga                                                     1635
<210> 28
   <211> 1581
   <212> DNA
   <213> Cochliobolus sativus
<400> 28
   atgggtctcc tggctgatgt caccggccct ctggccaact ttacatccca gtcgtccacg      60
   cctgtcgtcg cttctgccgc cttcgcctcc tttatcctgc tctccgtcgt cctcaatgta     120
   ctgaagcagt tgctgttcaa gaaggcgaat gagccgccca tggtcttcca ttggctgcct     180
   ataattggta gtacagtcac atatggcatg gatccgtatg ccttcttctt cgcgaatcac     240
   aagaagtatg gcaatgtctt tacatttatt cttctcggcc gcaagatgac tgtgtgtctg     300
   gacacggcgg gcaacaactt catcctaaat ggaaagatca aggacgtcaa cgccgaggag     360
   atttactctc ccctcaccac acccgtcttc ggcaaggacg tcgtgtatga ttgtccaaac     420
   tcgaaactca tggagcagaa aaagtttgtc aagtacggtc tcacgcagga ggccctccgt     480
   tcctacgtca ccctcatcac acaagaatgc gaagacttta tgaagcgcca caaggctttc     540
   aagggagaaa agggcacttt tgacgtgacc aaggtcatgg ctgagctcac catctacacc     600
   gcctctcgtt cgctccaggg cgaggaaatc cgcaagtctt tcgattcaaa gtttgccgaa     660
   ctgtaccacg acctcgatat gggcttttcg cctataaact ttatgctctc atgggccccg     720
   cttcctcaca accgcgcacg cgacaatgca cgcgagacca tgataaagct atactcggat     780
   ctggttcgca agcgcaggtc gggcgccgtg aagaaggact cgcacgacat gatttggcac     840
   ctgatggact gcaagtacaa ggacggcacc caggtgcccg aacacgagat tgctggcatc     900
   atgattgccc tgctcatggc cggacagcat tcgtcttcct caaccatcgc atggatcctt     960
   ctccgcctcg cgcagaaccc gcacgttatt gaggaattac ttgccgagca aaagtccatc    1020
   ctaggcaaaa acctccccgc cctaacctac gacgacctcc agaagcttcc cctccatgcc    1080
   caggttgtca aggaaaccct ccgtatccac gcccccattc actccatcat gcgcaaggtc    1140
   aagcaacctc ttgttgtcga tggaacaaac tacgttgttc ccacttccca cacactcatg    1200
   tcctcgcccg gtttctccgc acagctcgat acccactttg tcaaccccgc tgtctgggac    1260
   ccccaccgtt gggatccaga ccaaaacaac tacgacgaag agcgcgacga cgccgaccag    1320
   gagaagattg actatggctg gggtgtcgtg tccaagggaa ccaactcacc atatcttccc    1380
   tttggcgctg gacggcatcg ttgtattggg gagcaattcg cctacctgca actacagact    1440
   attctagtag catttgtgag agagttcaag ttcaggaacg ttggtggtag caaagacatt    1500
   gttagcaccg actacaccag tctcttctcc cgcccactag cacctggcat agttgagtgg    1560
   gagaggcggc agaacctata a                                              1581
<210> 29
   <211> 1686
   <212> DNA
   <213> Ustilago maydis
<400> 29
   atggtggcct cctcgtcttc ggcgacagcg tcgttgctcg accagctgtt tgcgcttacg 60
   cctctcgccg attcttcggc ctggatcaaa accatcacgg tgctcgtttt gcttcccttg 120
   ctcgcggtcg ttctcaacgt agcatcgcag cttctgcttg cgactcccaa gaatcatcct 180
   ccggtggtgt ttcactttgt gcctgtgatt ggatcggcga tctactatgg tatcgaccct 240
   tacaagttct ttttcgaatg ccgcgaaaag tatggcgatg tgttcacgtt tgttctgctc 300
   ggacgcaaga tcacggtggc gttgggaccc aagggaagca atctcgtctt taacgccaag 360
   catcagcagg tgacggcaga ggacgcgtat acgcacctca ccacgcccgt gtttggcaaa 420
   gaggtggtat acgatgtgcc taatgcggtg tttatggagc agaaaaagtt tgtcaaggtg 480
   ggtctgtcga tcgaaaactt tcgcgtctac gtgccgcaga tcgtggatga agtgcgagag 540
   tacatcaaga gcgatgcgcg tttcagcgca ctcaagacgc gcaagacgat cacagtcgat 600
   atttttcaag ccatgtcgga actcatcatc ctgaccgcat ccagaacgct gcagggcaag 660
   gaagtcagac agggtctcga caagtcattt gcgcaactgt atcacgatct cgactcgggc 720
   ttcactccga tcaactttgt gattcccaac ctgccgctgc cgagcaactt taaacgcgac 780
   agggcgcaga agaaaatgtc gcagttctac caggacattg tggcgaaacg acgagctgcg 840
   ggtgcatcca cgtctgccga cgacgccagt ggcgaaaacg atatgatcgc agcactgatc 900
   gagcaaaagt acaagaacgg acgtgcactc agcggcgtcg agattgcaca tatgatgatt 960
   gcactcttga tggcgggtca gcacacgagc agcgccacgt cgtcgtgggc gtttctgcgt 1020
   ctggctagtc gacccgaaat catcgaggag ctatacgagg agcagctcaa cgtgtactct 1080
   gacggacacg gtggtttgag ggaactcgac tacgagacgc aaaagacgtc ggtgcctctg 1140
   ctggatgctg tggtcaaaga gacgttgcga ctgcacccgc ctctgcacag catcatgcga 1200
   tatgtgaaat ccgaccttgc cgttccacct acgctctcgt cgccgacgtc gactaaatcc 1260
   gagccggatg cccactatgt gattcccaag ggccactaca ttatggctgc acctggcgtg 1320
   tcgcaagtgg atcctcagat ttggaagtca tcggaccagt ttgatccaca ccggtggttg 1380
   gacgctacga ccgctgcggc gatgcaggac tcgggcgagg acaagcaaga ctttggcttc 1440
   ggaatgatct ccaccggtgc caatagccca taccttccct ttggcgccgg aagacatcgg 1500
   tgcatcggag agcagttcgc ctatctccag atcggcgtca tcctagccac cttcgtccgc 1560
   atcttcaaat ggcacctcga ctccaaattc cccgatcccg actaccaaag catggtcgtc 1620
   cttcccagca aaaacggttg cgccatcgtc ctcacccccc gagccgaatc cctccacctc 1680
   gactag 1686
<210> 30
   <211> 1588
   <212> DNA
   <213> Rhynchosporium secalis
<400> 30
   atgggaattt ttgaaggctg ttacagtccc gctggctcag caggtctcgc agcgcggatt      60
   gggcgttgtg atcgccgctg gtttcgcagc cttcttggtc gtttctgtta ttctcaatgt     120
   cctgagccag atactgttca agaaccccaa tgagcctccg attgttttcc acttcttccc     180
   tatcattggt agcacagtta catacggtat tgacccttac aaattcttct tcgacaacaa     240
   ggcaaagtat ggcgaagtct ttacctttat cctgctcggc aagaagacga ctgtgtacct     300
   tggtacccac ggaaacgaat tcattctcaa tggaaagatt aaggatgtta atgcggagga     360
   ggtctacacg gtcctgacca ccccagtctt tggaaaggat gtggtttacg attgtccgaa     420
   ctataaggga agggagcaga aaaaagttta tgaaaaatcg ggactttcca cagaagcctt     480
   cagacttacg ttccaatcat tcaggacgaa gtcgaaacat ttatcaaggg ctcggcagca     540
   ttcaaggggc ataaaggcac tgtcaacatt cctgctcaaa tggctgaaat cacaatctat     600
   accgcttctc acgccctcca aggaaaggat tgccgagaca aattcgacca ctcatttgct     660
   gagctwtayc acgcmctsga catgggcttt agcccgataa attttatgct tcactgggct     720
   cctcttcccc ataatcgcgc acgggaccac gcacagcgaa ctgtcgccaa agcctacatg     780
   gaaatcatgg agacgcgccg aaaagacaag aagagcctcg acaatatgga catcatgtcc     840
   cagctcatgc gctccactta caagaatgga gttccggtcc cggatatgga aatcgcgcac     900
   atgatgattg ccctgctcat ggctggacaa cattcgtcct cttcctcaag tacctggatc     960
   atactcagac ttgccgctag accagatatc ttggaggagc tataccagga gcaactcgaa    1020
   gtgctcggat ctgatctacc agccctacaa tacgaagatc tcgccaaact caccaagcac    1080
   caaaacgttc tgaaggaggt cctgagactt cacactccta tccactccat catgcgaaaa    1140
   gtcaagagcc ctatgccaat tgcaggtact aagtttgtca tcccaacctc acacgtcctc    1200
   ctcgcatcac ctggtttttc cagccgagag gctacctatt ttccctgacc cgctaaagtg    1260
   gracccccay cgatgggaac ccgaatctgg tggagtcctg ggtactgagg tcgaggaaga    1320
   gagctacgat tacggatacg gtcttattag caagggtgcc aagagtccat accttccatt    1380
   cggagctggt agacataggt gtatcggaga gcaatttgcc aatgtgcagc tgatcactat    1440
   cacagctgtc atggtacgat atttcaaatt caggaacctc gatggaagct cgaaggttgt    1500
   tgagactgat tatacgagtc tattctcgag acctctagca ccggcggtgg ttgagtggga    1560
   gaagagagaa aaagttaagg tttgaagg                                       1588
<210> 31
   <211> 1575
   <212> DNA
   <213> Venturia inaequalis
<400> 31
   atgggactcc tctctccttt gctcgcctcg ttaccgggca gcgaccgcag ttggttattt      60
   tacactcttg cctccttcgg cttcaccgtt gcaatcgtcg ccgccaacct tgtcaagcaa     120
   ctcttattct caaacccaaa cgaacctcca gtagtcttcc actggtttcc cttcttcggc     180
   aacacggtcg tctacggcat cgatcctatc aagtttttcg ccgagtgcaa ggaaaagcat     240
   ggcgatatct ttaccttcat tcttcttggc aggaaaacaa cagtctacat tggtacaaag     300
   ggaaacgaat tcattctcaa tggcaaacag agccatgtca acgcagagga aatctatagc     360
   cccctgacga cgcccgtctt cggctccgat gttgtctatg attgcccaaa ctcgaaattg     420
   atggagcaaa agaagttcgt caagtacggt ctcaccaccg aagctctcaa atcctatgtc     480
   accctcatcc aacaagaagt cgaagactat accaaacgct accctcaatt caaaggcgaa     540
   aagggcagct tcgatgtttg cgcttccatg gccgaaatca caatcttcac tgcttcccgc     600
   tcactacaag gcaaggaggt tcgcgacaag tttgacgcca gctttgcaga cctcttccac     660
   gatttggata tgggcttctc tcctatcaac ttcatgcttc cctgggcccc tcttccacac     720
   aatcgtcgcc gagatgccgc gaacaaaaag atgacggaga catatttgga aattatccaa     780
   tcgagaaaag cagagggcgt caaaaaggat tcagaggaca tgatttggaa tttgatgcag     840
   tgtgtataca agaatggcac tcccatcccg gacaaagaaa tcgcccacat gatgatcgcg     900
   ctgctcatgg ccggccagca ctcgtcctct agcacctcgt cctggattct acttcgacta     960
   gctaccagac ctgatatcca ggaagaacta taccaagaac aaattcgggt ttgcggcgct    1020
   gatcttccac cgttgcagta cgaagatctt gctcgcatgc ctctccacaa ccagattatc    1080
   aaagaaactc ttcgcatgca ttcgccaatt cacagcatct tgcgtgccgt caaacagcct    1140
   atgcctgtcg aaggaactcc ttacaccatc cccacctcgc atgttctcct tgctgctccc    1200
   atcgcatctg gaggctcgcc aatgtacttt ccagctcctg agaagtggga gcctcaccgt    1260
   tgggacgaag gatcaggagg aaccaacatc tcgggcggcg aaaacggtgg cgaagagaaa    1320
   gaggattacg gctatggact catcacaaag ggcgccagct cgccgtacct tccgttcggc    1380
   gctggaagac ataggtgtat cggcgaacaa tttgcatata tgcagttgaa cacggttctc    1440
   gcgacgcaag ttcgcgaatt caagttcagt ttgagggaag gagagtcgtt ccccaagacc    1500
   gacttctctt ctctattttc tggacctcta cgccccgcgt ggttgaactg ggaacgtaga    1560
   gagaagtcct catga                                                     1575
<210> 32
   <211> 21
   <212> DNA
   <213> artificial sequence
<220>
   <223> primer
<400> 32
   cggtccattg acaatccccg t 21
<210> 33
   <211> 20
   <212> DNA
   <213> artificial sequence
<220>
   <223> primer
<400> 33
   gcagcaaact cggcagtgag 20
<210> 34
   <211> 24
   <212> DNA
   <213> artificial sequence
<220>
   <223> primer
<400> 34
   gacgtccagc aagtttgacg agtc 24
<210> 35
   <211> 26
   <212> DNA
   <213> artificial sequence
<220>
   <223> primer
<400> 35
   ccatggagag ttcataaggt gcttca 26
<210> 36
   <211> 25
   <212> DNA
   <213> artificial sequence
<220>
   <223> primer
<400> 36
   actagtattg gaagcaccgt acaat 25
<210> 37
   <211> 27
   <212> DNA
   <213> artificial sequence
<220>
   <223> primer
<400> 37
   gagctccatt ggagcagtca taaacaa 27
<210> 38
   <211> 24
   <212> DNA
   <213> artificial sequence
<220>
   <223> primer
<400> 38
   aagcttcagc aagtttgacg agtc 24
<210> 39
   <211> 27
   <212> DNA
   <213> artificial sequence
<220>
   <223> primer
<400> 39
   cccgggcatt ggagcagtca taaacaa 27
<210> 40
   <211> 38
   <212> DNA
   <213> artificial sequence
<220>
   <223> primer
<400> 40
   taatacgact cactataggg cagcaagttt gacgagtc 38
<210> 41
   <211> 40
   <212> DNA
   <213> artificial sequence
<220>
   <223> primer
<400> 41
   taatacgact cactataggc attggagcag tcataaacaa 40
<210> 42
   <211> 20
   <212> DNA
   <213> artificial sequence
<220>
   <223> primer
<400> 42
   cctttggtgc cggtagacat 20
<210> 43
   <211> 20
   <212> DNA
   <213> artificial sequence
<220>
   <223> primer
<400> 43
   cccatcgaat aaacgcaggc 20
<210> 44
   <211> 22
   <212> DNA
   <213> artificial sequence
<220>
   <223> primer
<400> 44
   tctacaccgt tctcactact cc 22
<210> 45
   <211> 20
   <212> DNA
   <213> artificial sequence
<220>
   <223> primer
<400> 45
   gcttctcttg aagtaatcgc 20
<210> 46
   <211> 20
   <212> DNA
   <213> artificial sequence
<220>
   <223> primer
<400> 46
   cgagtccctg gcactgaatg 20
<210> 47
   <211> 20
   <212> DNA
   <213> artificial sequence
<220>
   <223> primer
<400> 47
   gctcatcacc ccaaaaccgt 20
<210> 48
   <211> 21
   <212> DNA
   <213> artificial sequence
<220>
   <223> primer
<400> 48
   atctcgagcc cggtaccatg g 21
<210> 49
   <211> 22
   <212> DNA
   <213> artificial sequence
<220>
   <223> primer
<400> 49
   ctcggtgtaa tgacccttgg cc 22